# EUROPEAN PATENT SPECIFICATION

(11) **EP 2 099 434 B1**
(45) Date of publication and mention of the grant of the patent: **05.06.2019**
(21) Application number: 07864490.3
(22) Date of filing: 16.11.2007
(51) Int. Cl.: A61M 5/20, A61M 5/19, A61M 5/24, A61M 5/32, A61M 5/50, A61M 5/31

(54) **DEVICES AND SYSTEMS FOR MEDICAMENT DELIVERY**
VORRICHTUNGEN UND SYSTEME ZUR MEDIKAMENTENABGABE
DISPOSITIFS ET SYSTÈMES POUR L'ADMINISTRATION DE MÉDICAMENTS

(30) Priority: 21.11.2006 US 562061; 04.12.2006 US 566422; 05.06.2007 US 758393
(43) Date of publication of application: 16.09.2009
(73) Proprietor: kaleo, Inc., Richmond, VA 23219 (US)
(72) Inventor: EDWARDS, Eric Shawn, Moseley, Virginia 23120 (US); EDWARDS, Evan Thomas, Charlottesville, VA 22903 (US); LICATA, Mark J., Doswell, Virginia 23047 (US); MEYERS, Paul F., Fishers, Indiana 46037 (US)
(74) Representative: Gill Jennings & Every LLP
(86) International application number: PCT/US2007/084891
(87) International publication number: WO 2008/064092

(56) References cited:
- WO-A2-2006/083876
- US-A- 3 055 362
- US-A1- 2005 267 403
- US-A1- 2007 239 114

## Description

### Background

The invention is defined in claim 1 and relates generally to a medical device, and more particularly to a medicament delivery device for automatically injecting a medicament into a body of a patient.

Exposure to certain substances, such as, for example, peanuts, shellfish, bee venom, certain drugs, toxins, and the like, can cause allergic reactions in some individuals. Such allergic reactions can, at times, lead to anaphylactic shock, which can cause a sharp drop in blood pressure, hives, and/or severe airway constriction. Accordingly, responding rapidly to mitigate the effects from such exposures can prevent injury and/or death. For example, in certain situations, an injection of epinephrine (i.e., adrenaline) can provide substantial and/or complete relief from the allergic reaction. In other situations, for example, an injection of an antidote to a toxin can greatly reduce and/or eliminate the harm potentially caused by the exposure.

Because emergency medical facilities may not be available when an individual is suffering from an allergic reaction, some individuals carry an auto-injector to rapidly self-administer a medicament in response to an allergic reaction. Some known auto-injectors are cylindrical in shape and include a spring loaded needle to automatically penetrate the user's skin and inject the medicament. Such known auto-injectors can be bulky and conspicuous, which can make carrying them inconvenient and undesirable. Moreover, some known auto-injectors do not have a retractable needle and, as such, cause a sharps hazard when injection is complete. Thus, a need exists for an auto-injector that can be more conveniently carried by a user and does not present a sharps hazard upon completion of the injection.

Some known auto-injectors include a locking cap at the proximal end of the auto-injector to prevent inadvertent actuation and a needle cover at the distal end of the auto-injector. Such a configuration can, at times, cause a user to become confused as to which end of the auto-injector is the "needle end" (i.e., the distal end) and which end of the auto-injector is the "actuation end" (i.e., the proximal end). As such, in some situations, a user may mistakenly actuate the known auto-injector away from the intended injection site. Such an error can result, for example, in the auto-injector being actuated into the user's thumb and/or finger. Furthermore, the locking cap can be removed prior to removal of the needle cover, thus allowing the auto-injector to be actuated before the needle cover has been removed. A need exists for an auto-injector that can be actuated from its distal end. A need also exists for an auto-injector that cannot be actuated until the needle cover has been removed.

Some known auto-injectors include a needle cover that collapses or buckles when the auto-injector is actuated and the needle breaks through the cover. In application, this leaves the needle cover bunched around a portion of the needle, which can cause the needle cover to interfere with penetration of the needle into the user. A need exists for an auto-injector with a needle cover that will not interfere with consistent penetration of the needle.

Some known auto-injectors use pressurized gas to insert a needle and/or inject a medicament into the patient. Such known auto-injectors often do not include a mechanism for completely releasing or venting the pressurized gas upon completion of the injection event. Thus, a need exists for a gas-powered auto-injector that has an improved gas release mechanism.

Manufacturing techniques of known auto-injectors require much of the manufacturing process of an auto-injector to occur in a sterile environment. In particular, a sterile environment is needed for filling the auto-injector with a medicament and for assembly of the auto-injector. Providing and maintaining a sterile environment during the entire manufacturing process, however, can be quite expensive. A further need exists for a more economical method of manufacturing auto-injectors.

WO 2006/083876 discloses an injection apparatus.

According to the present invention, there is provided an apparatus, comprising:
a housing having a distal end portion and a proximal end portion, the distal end portion including a distal end surface;
a medicament injector including a medicament container and a needle, the needle configured to move between a first needle position and a second needle position, in the first needle position the needle is contained within the housing, in the second needle position at least a portion of the needle extends from the distal end portion of the housing;
an energy storage member configured to produce a force to move the needle between the first needle position and the second needle position when the energy storage member is actuated; and
an actuator configured to actuate the energy storage member; and
a locking member configured to be removeably coupled to the distal end portion of the housing, a protrusion of the locking member disposed through an opening defined by the distal end surface of the housing such that the protrusion engages the actuator to limit movement of the actuator when the locking member is coupled to the distal end portion of the housing.

### Brief Description of the Drawings

FIG. 1 is a perspective view of a system according to an embodiment of the invention.
FIG. 2 is a front view of a system according to an embodiment of the invention.
FIG. 3 is a side view of a system according to an embodiment of the invention.
FIG. 4 is a cross-sectional view taken along line A-A of FIG. 3 of a system according to an embodiment of the invention in a first operative position.
FIG. 5 is a cross-sectional view taken along line A-A of FIG. 3 of a system according to an embodiment of the invention in a second operative position.
FIG. 6 is a cross-sectional view taken along line A-A of FIG. 3 of a system according to an embodiment of the invention in a third operative position.
FIG. 7 is a cross-sectional view taken along line A-A of FIG. 3 of a system according to an embodiment of the invention in a fourth operative position.
FIG. 8 is a cross-sectional view taken along line A-A of FIG. 3 of a system according to an embodiment of the invention in a fifth operative position.
FIG. 9 is a cross-sectional view taken along line A-A of FIG. 3 of a system according to an embodiment of the invention in a sixth operative position.
FIG. 10 is a flowchart illustrating a method according to an embodiment of the invention.
FIG. 11 is a perspective view of a system according to an embodiment of the invention.
FIG. 12 is a perspective cross-sectional view the system illustrated in FIG. 11 taken along line B-B of FIG. 11.
FIG. 13 is a perspective view of an apparatus according to an embodiment of the invention.
FIG. 14 is a cross-sectional view of a mechanism according to an embodiment of the invention taken along line A-A of FIG. 3.
FIGS. 15A and 15B are schematic illustrations of an auto-injector according to an embodiment of the invention in a first configuration and a second configuration, respectively.
FIGS. 16A and 16B are schematic illustrations of an auto-injector according to an embodiment of the invention in a first configuration and a second configuration, respectively.
FIG. 17 is a perspective view of an auto-injector according to an embodiment of the invention.
FIG. 18 is a perspective view of the auto-injector illustrated in FIG. 17 in a first configuration, with at least a portion of the auto-injector illustrated in phantom lines for ease of reference.
FIG. 19 is a front view of the auto-injector illustrated in FIGS. 17 and 18 in a first configuration.
FIG. 20 is a perspective view of the auto-injector illustrated in FIG. 17 showing an assembly according to an embodiment of the invention being removed.
FIG. 21 is a front view of the auto-injector illustrated in FIG. 17 showing a member according to an embodiment of the invention being removed.
FIG. 22 is an exploded perspective view of a portion of the auto-injector illustrated in FIG. 20.
FIG. 23 is a cross-sectional view of a component illustrated in FIG. 22.
FIG. 24 is a perspective view of a component illustrated in FIG. 22.
FIG. 25 is a perspective view of a member of the auto-injector illustrated in FIG. 21.
FIG. 26 is a perspective view of a portion of the auto-injector illustrated in FIGS. 17 and 21.
FIG. 27 is a perspective view of a portion of the auto-injector illustrated in FIGS. 17 and 26.
FIG. 28 is a partially exploded perspective view of a base of the auto-injector illustrated in FIG. 26.
FIG. 29 is an exploded perspective view of a portion of the auto-injector shown in FIG. 19.
FIG. 30 is a front view of a component of the auto-injector shown in FIG. 29.
FIG. 31 is a front view of the auto-injector illustrated in FIG. 19 in a second configuration.
FIG. 32 is a perspective view of a portion of the auto-injector shown in FIG. 31.
FIGS. 33 and 34 are perspective views of a portion of the auto-injector shown in FIG. 32.
FIG. 35 is a top view of the housing of the auto-injector shown in FIG. 31.
FIG. 36 is a cross-sectional view of the housing taken along line 36-36 in FIG. 35.
FIG. 37 is front view of the auto-injector illustrated in FIGS. 19 and 31 in a third configuration.
FIG. 38 is a front view of the portion of the auto-injector labeled as 38 in FIG. 37.
FIG. 39 is a perspective view of a portion of the auto-injector shown in FIG. 37.
FIG. 40 is a cross-sectional view of a portion of the auto-injector as shown in FIG. 37.
FIG. 41 is a perspective view of a portion of the auto-injector as shown in FIG. 37.
FIG. 42 is an exploded perspective view of a portion the auto-injector as shown in FIG. 37.
FIG. 43 is a front view of a portion of the auto-injector illustrated in FIGS. 19, 31 and 37 in a fourth configuration.
FIG. 44 is a front view of the auto-injector illustrated in FIGS. 19, 31, 37 and 43 in a fifth configuration.
FIG. 45 is a front view of the auto-injector illustrated in FIGS. 19, 31, 37, 43 and 44 in a sixth configuration.
FIG. 46 is a front view of an auto-injector according to an embodiment of the invention.
FIG. 47 is a schematic illustration of an auto-injector according to an embodiment of the invention.
FIG. 48 is a schematic illustration of an auto-injector according to an embodiment of the invention.
FIGS. 49 and 50 are schematic illustrations of an auto-injector according to an embodiment of the invention in a first configuration and a second configuration, respectively.
FIGS. 51 and 52 are schematic illustrations of an auto-injector according to an embodiment of the invention in a first configuration and a second configuration, respectively.
FIGS. 53-55 are schematic illustrations of an auto-injector according to an embodiment of the invention in a first configuration, a second configuration and a third configuration, respectively.
FIGS. 56 and 57 are schematic illustrations of an auto-injector according to an embodiment of the invention in a first configuration and a second configuration, respectively.
FIG. 58 is a front view of a portion of an auto-injector according to an embodiment of the invention.
FIGS. 59 - 61 are schematic illustrations of an auto-injector according to an embodiment of the invention in a first configuration, a second configuration and a third configuration, respectively.
FIGS. 62 and 63 are schematic illustrations of an auto-injector according to an embodiment of the invention in a first configuration and a second configuration, respectively.
FIGS. 64 - 66 are schematic illustrations of an auto-injector according to an embodiment of the invention in a first configuration, a second configuration and a third configuration, respectively.
FIGS. 67 and 68 are schematic illustrations of a portion of an auto-injector according to an embodiment of the invention in a first configuration and a second configuration, respectively.
FIG. 69 is a schematic illustration of an auto-injector according to an embodiment of the invention in a first configuration.
FIGS. 70 - 73 are schematic illustrations of a portion of the auto-injector in FIG. 69 in a second configuration, a third configuration, a fourth configuration and a fifth configuration, respectively.
FIG. 74 is a plot showing the pressure within the auto-injector shown in FIG. 69 as a function of the position of a portion of the auto-injector.
FIGS. 75 and 76 are perspective views of an auto-injector according to an embodiment of the invention in a first configuration and a second configuration, respectively.
FIGS. 77 - 79 are front views of an auto-injector according to an embodiment of the invention in a first configuration, a second configuration and third configuration, respectively.
FIG. 80 is a front view of a portion of the auto-injector illustrated in FIGS. 78 and 79.
FIG. 81 is an exploded perspective view of the portion of the auto-injector illustrated in FIG. 80.
FIGS. 82 and 83 are perspective views of an auto-injector according to an embodiment of the invention in a first configuration and a second configuration respectively.
FIG. 84 is a front view of a portion of the auto-injector illustrated in FIG. 83.
FIG. 85 is a perspective view of the portion of the auto-injector illustrated in FIG. 83.
FIG. 86 is a flowchart illustrating a method according to an embodiment of the invention.

### Detailed Description

Apparatuses and methods for automatic medicament injection and methods for manufacturing automatic medicament injectors (also referred to herein as "auto-injectors") are described herein.

In some embodiments, an apparatus includes a housing, an injection member at least partially disposed in the housing, and an actuator configured to be disposed within the housing. The actuator is configured to move the injection member between a first position and a second position. The actuator includes an energy storage member that has a first configuration and a second configuration. The energy storage member is configured to produce a force when moved from the first configuration to the second configuration to move the injection member between its first position and its second position.

In some embodiments, an apparatus includes a movable member configured to be disposed within a housing of a medical device and a gas release assembly coupled to the movable member. The movable member has a first end portion and a second end portion. A portion of the first end portion is configured to define a portion of a boundary of a gas chamber. The gas release assembly is configured to selectively allow fluid communication between the gas chamber and the area outside the gas chamber.

In some embodiments, an apparatus includes a housing, a needle at least partially disposed within the housing, and a guard removably coupled to the housing. The guard is configured to cover at least a portion of the needle.

In some embodiments, an apparatus includes a housing, a medicament container disposed within the housing and an actuator. The actuator is configured to be disposed within the housing and to move the medicament container within the housing. The actuator includes a release member and an energy storage member. The energy storage member, which can be, for example, a compressed gas container, has a first position and a second position. When in the first position, the energy storage member has a first potential energy. When in the second position the energy storage member has a second potential energy less than the first potential energy. The energy storage member is configured to convert a portion of the first potential energy into kinetic energy when moved from its first position to its second position to move the medicament container within the housing. The energy storage member has a longitudinal axis offset from a longitudinal axis of the medicament container. The release member is configured to selectively deploy the energy storage member from its first position to its second position.

In some embodiments, an apparatus includes a housing, a needle and an actuator. The needle has a first end and a second end and defines a longitudinal axis. The actuator is configured to be disposed within the housing and to move the needle between a first needle position and a second needle position. When in the first needle position, the second end of the needle is within the housing. When in the second needle position, the second end of the needle is outside the housing. The actuator includes a release member and an energy storage member. The energy storage member has a first position and a second position. When in the first position, the energy storage member has a first potential energy. When in the second position the energy storage member has a second potential energy less than the first potential energy. The energy storage member is configured to convert a portion of the first potential energy into kinetic energy when moved from its first position to its second position to move the needle between the first needle position and the second needle position. The energy storage member has a longitudinal axis offset from the longitudinal axis of the needle. The release member is configured to selectively deploy the energy storage member from its first position to its second position.

In some embodiments, an apparatus includes a housing, a needle, a medicament container and an actuator. The needle has a first end and a second end and defines a longitudinal axis. The actuator is configured to be disposed within the housing and to move the needle between a first needle position and a second needle position. When in the first needle position, the second end of the needle is within the housing. When in the second needle position, the second end of the needle is outside the housing. The actuator is further configured to move the medicament container between a first medicament container position and a second medicament container position. When in the first medicament container position, a lumen defined by the needle is fluidically isolated from the medicament container. When in the second medicament container position, the first end of the needle is disposed within the medicament container such that the lumen is in fluid communication with the medicament container. The actuator includes a release member and an energy storage member. The energy storage member has a first position and a second position. When in the first position, the energy storage member has a first potential energy. When in the second position the energy storage member has a second potential energy less than the first potential energy. The energy storage member is configured to convert a portion of the first potential energy into kinetic energy when moved from its first position to its second position to move the needle between the first needle position and the second needle position. The energy storage member has a longitudinal axis offset from the longitudinal axis of the needle. The release member is configured to selectively deploy the energy storage member from the first position to the second position.

In some embodiments, an apparatus includes an actuator disposable within a housing of an auto-injector. The actuator is configured to move a medicament container relative to the housing, and includes a gas container, a biasing member and a puncturer. The gas container, which is configured to store a compressed gas, is movable between a first position and a second position. The biasing member has a retracted configuration and an expanded configuration. The biasing member is configured to engage the gas container such that when the biasing member moves from the retracted configuration to the expanded configuration the gas container is moved from the first position to the second position. The puncturer is configured to penetrate a portion of the gas container when the gas container moves to the second position to allow a portion of the compressed gas to be released from the gas container into a gas chamber defined within the housing adjacent the medicament container.

In some embodiments, an apparatus includes a housing having a distal end portion and a proximal end portion, a medicament injector, an energy storage member and a retainer. The medicament injector is disposed within the housing and includes a medicament container and a needle. The energy storage member, which can be, for example, a gas container configured to contain a pressurized gas, is configured to produce a force when moved from a first configuration to a second configuration to move the medicament injector between a first position and a second position. The retainer has a first position and a second position. When the retainer is in its first position, the retainer is configured to retain the energy storage member in its first configuration. When the retainer is in its second position, the retainer is configured to allow the energy storage member to be moved from its first configuration to its second configuration. The retainer is configured to be selectively moved from its first position to its second position by manipulating an actuator adjacent the distal end portion of the housing.

In some embodiments, an apparatus includes a movable member and a valve coupled to the movable member. The movable member is configured to be disposed within a housing of a medical device and has a first end portion and second end portion. A portion of the first end portion is configured to define a portion of a boundary of a gas chamber. The first end portion defines an opening configured to be in fluid communication between the gas chamber and an area outside the gas chamber. The second end portion is configured to be coupled to a needle configured to deliver a medicament into a body. The valve is configured to selectively allow fluid communication between the gas chamber and the area outside the gas chamber through the opening defined by the first end portion of the movable member.

In some embodiments, an apparatus includes a movable member, a valve and an actuator. The valve and the actuator are each coupled to the movable member. The movable member is configured to be disposed within a housing of a medical device and has a first end portion and second end portion. A portion of the first end portion is configured to define a portion of a boundary of a gas chamber. The first end portion defines an opening configured to be in fluid communication between the gas chamber and an area outside the gas chamber. The second end portion is configured to be coupled to a needle configured to deliver a medicament into a body. The valve is configured to selectively allow fluid communication between the gas chamber and the area outside the gas chamber through the opening defined by the first end portion of the movable member. The actuator is configured to move the valve between a first position and a second position. When the valve is in the first position the gas chamber is fluidically isolated from the area outside the gas chamber. When the valve is in the second position the gas chamber is in fluid communication with the area outside the gas chamber.

In some embodiments, an apparatus includes a housing, a medicament container, a medicament injector, an injection member and a valve. The housing defines a gas chamber. The medicament container is configured to be movably disposed within the housing and defines a portion of a boundary of the gas chamber. The medicament injector includes a seal configured to engage a portion of the housing to fluidically isolate the gas chamber from an area outside the gas chamber. A portion of the medicament injector is engaged with a medicament container that is movably disposed within the housing. The injection member, which can be, for example, a needle, defines a lumen configured to be in fluid communication with the medicament container and is configured to convey a medicament from the medicament container into a body of a patient. The medicament injector has a first position and a second position. When in the first position, the injection member is contained within the housing. When in the second position, a portion of the injection member extends from the housing. The valve, which can be disposed on the medicament injector, has a first configuration and a second configuration. When the valve is in the first configuration, the gas chamber is fluidically isolated from the area outside the gas chamber. When the valve is in the second configuration, the gas chamber is in fluid communication with the area outside the gas chamber.

In some embodiments, an apparatus includes a housing defining a gas chamber, a movable member and a gas release assembly. The movable member has a first portion and a second portion. The first portion defines a portion of a boundary of the gas chamber. The second portion is configured to be coupled to a needle that can deliver a medicament into a body. The movable member is disposable within the housing in a first position and a second position. When the movable member is in the first position, the needle is disposed within the housing. When the movable member is in the second position, a portion of the needle extends outside the housing. The gas release assembly, which can include, for example, a valve, an actuator and a passageway between the gas chamber and an area outside of the gas chamber, has a first configuration and a second configuration. When the gas release system is in its first configuration, the gas chamber is fluidically isolated from the area outside the gas chamber. When the gas release system is in its second configuration, the gas chamber is in fluid communication with the area outside the gas chamber. The gas release assembly is configured to be moved from its first configuration to its second configuration when the movable member is in its second position. The gas release system is further configured to be maintained in its second configuration independent of the position of the movable member.

In some embodiments, an apparatus includes a housing defining a gas chamber, a movable member and a valve. The movable member is configured to move longitudinally within the housing. The movable member has a first portion and a second portion. The first portion defines a portion of a boundary of the gas chamber. The second portion is configured to move a plunger within a medicament container to expel a medicament contained within the medicament container. The valve defines a flow passageway between the gas chamber and an area outside the gas chamber. The flow passageway has a flow area that varies as a function of a longitudinal position of the movable member.

In some embodiments, an apparatus includes a housing, a needle, an energy storage member, an actuator, a locking member, and a needle guard. The needle is configured to move between a first position and a second position. In its first position, the needle is contained within the housing. In its second position, at least a portion of the needle extends from the housing. The energy storage member has a first configuration and a second configuration and is configured to produce a force when moving between its first configuration and its second configuration to move the needle from its first position to its second position. The actuator is configured to move the energy storage member from its first configuration to its second configuration. The locking member is movably coupled to the distal end portion of the housing such that the locking member can be moved between a first position and a second position. In its first position, the locking member is configured to engage the actuator to prevent the actuator from moving the energy storage member to the second configuration. The needle guard is removably coupled to at least one of the distal end portion of the housing or a base movably coupled to the distal end portion of the housing.

In some embodiments, an apparatus includes a housing and a safety guard. The safety guard includes a locking portion and a needle guard portion. The locking portion is configured to inhibit actuation of a medicament delivery device. The needle guard portion is configured to substantially cover a needle of the medicament delivery device. The safety guard has a first position and a second position. In its first position, the safety guard is configured to be selectively coupled to at least one of the housing or a base movably coupled to the housing. In its second position, the safety guard is removed from the housing.

In some embodiments, an apparatus includes a needle guard configured to cover at least a portion of a needle of a medical injector. The needle guard is configured to substantially prevent microbes from passing through the needle guard. The needle guard is configured to allow a sterilant gas to pass through the needle guard.

In some embodiments, an apparatus includes a housing, a medicament injector, and a porous needle guard. The medicament injector is disposable within the housing and includes a needle. The needle has a first position and a second position. In its first position, the needle is contained within the housing. In its second position, at least a portion of the needle extends from the housing. The porous needle guard is removably coupled to the distal end portion of the housing. The porous needle guard is constructed from a microbial resistant material.

A method of manufacturing an automatic medicament injector includes inserting at least a portion of a needle into a needle hub disposed in a housing. A needle cover is installed over at least a portion of the needle to substantially cover a portion of the needle extending from the needle hub. The needle is sterilized after the needle cover is installed over at least a portion of the needle.

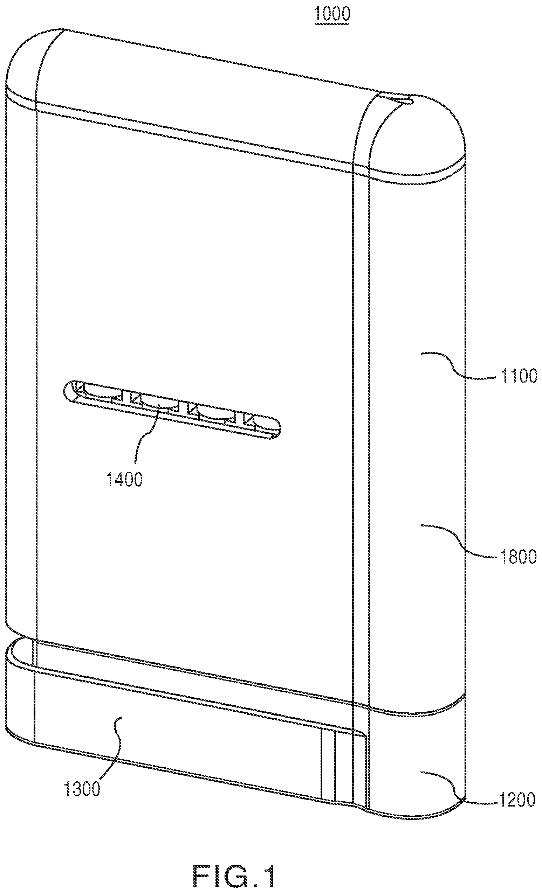

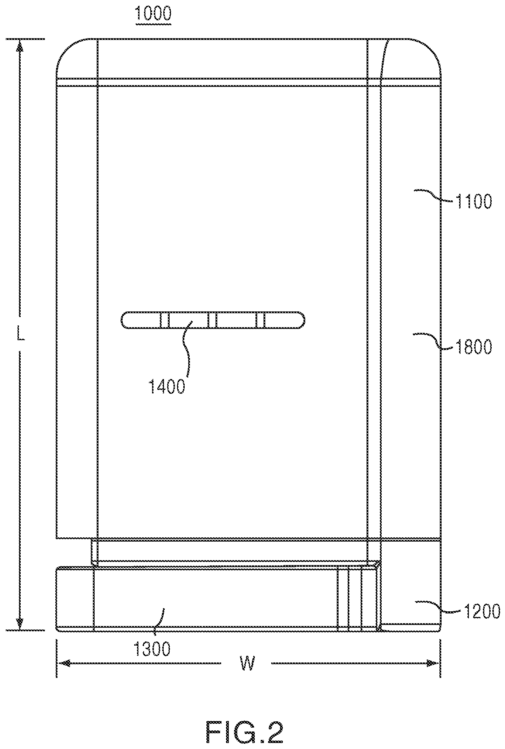

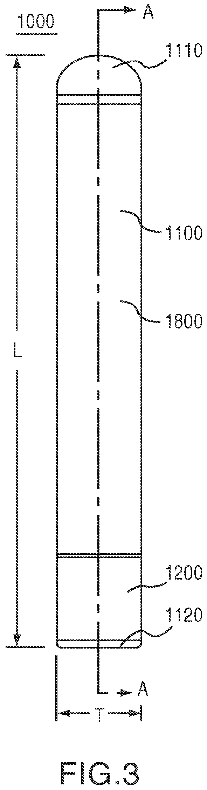

FIG. 1 is a perspective view, FIG. 2 is a front view, and FIG. 3 is a side view, of a system 1000 according to the invention, which can comprise a housing 1100, which, in some embodiments, can comprise a handheld portion 1800 separated via an actuation guard 1200 from an actuation bar 1300. Actuation guard 1200 can prevent accidental activation of system 1000. Housing 1100 can be constructed of a durable material, such as stainless steel, aluminum, polycarbonate, etc., to protect a compressed gas container, medicament, injection apparatus and/or user of system 1000. The injection apparatus can be actuated by a fluid pressure, such as pressure provided by the compressed gas, which upon completion of actuation can escape housing 1100 via gas escape opening, such as via status indicator 1400.

A status of a system 1000 can be determined via status indicator 1400, which can provide a view, such as via a UV blocking, photo-sensitive, and/or translucent window, into an interior of housing 1100. Viewable through the window can be a status of medicament carried by housing 1100, a location of a needle and/or injection apparatus for the medicament, and/or an activation status of system 1000. For example, if the medicament has aged to the point of discoloration, which aging might or might not render the medication useless, harmful, etc., status indicator 1400 can allow that situation to be determined. In some embodiments, gas can escape housing 1100 via status indicator 1400 and/or another opening in housing 1100.

Some embodiments of system 1000 can provide a compact medicament delivery mechanism that can efficiently and/or rapidly deliver a prescribed dose. The length (L) and width (W) of system 1000 can be similar to that of a credit card, and the thickness (T) can be less than 2.54 cm (one inch). Thus, some embodiments of system 1000 can provide a conveniently carried, easy-to-use, easy to activate drug delivery apparatus that can require little to no training to safely carry, use, and/or dispose of.

To assist a user in positioning system 1000 in a correct orientation for injection, system 1000 and/or housing 1100 can provide various tactile clues. For example, a top 1110 of housing 1100 can be rounded, and a bottom 1120 of actuation bar 1300 of housing 1100 can be flat. Other tactile clues are also possible, such as bulges, ribs, grooves, gaps, roughened surfaces, indentations, etc.

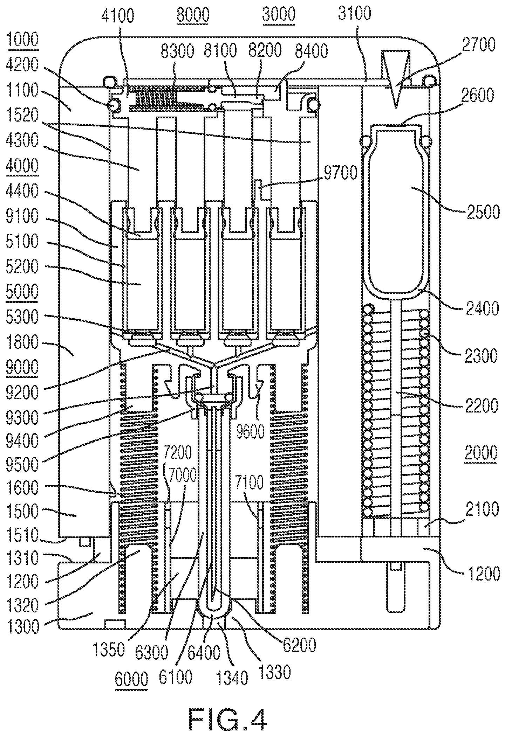

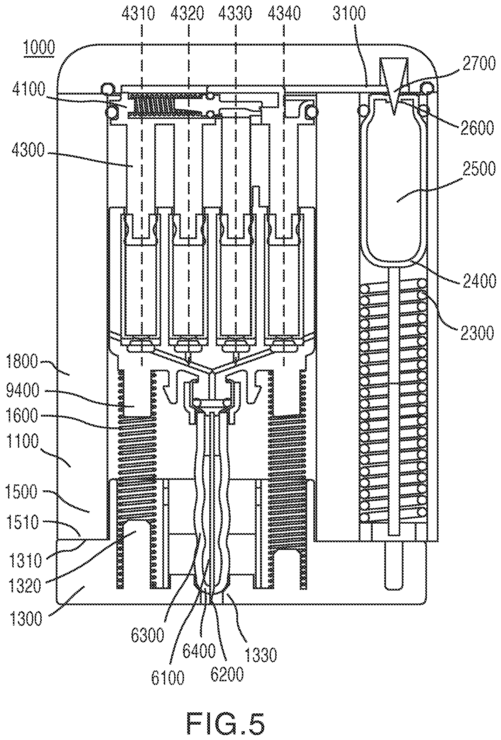

FIG. 4 is a cross-sectional view taken along line A-A of FIG. 3 of an embodiment of a system 1000 in a first operative position. FIGS. 5, 6, 7, 8, and 9 show system 1000 of FIG. 4 in second, third, fourth, fifth, and sixth operative positions, respectively.

System 1000 comprises a housing 1100, handheld portion 1800, actuation guard 1200, and/or actuation bar 1300. System 1000 can comprise system actuator 2000, gas reservoirs 3000, medicament actuator 4000, medicament storage assembly 5000, medicament carrier 9000, needle assembly 6000, use indicator 7000, and/or gas vent mechanism 8000, etc.

Upon removal, release, rotation, and/or relocation of actuation guard 1200, system actuator 2000 can be adapted to rapidly discharge an actuating portion of a contents of a compress gas container. For example, system actuator 2000 can comprise a compressed gas container 2400, which initially can contain a compressed gas 2500, an actuating portion of which can be released from container 2400 by penetration of a gas port 2600 via a point of a puncturer 2700. Upon removal and/or relocation of actuation guard 1200, actuation bar 1300 can be moved closer to and/or in contact with handheld portion 1800. Upon removal and/or relocation of actuation guard 1200, gas container 2400 can be brought into contact with puncturer 2700 via extension of a pre-compressed spring 2300 and/or movement of an actuation stick 2200. Thus, actuation guard 1200 can prevent accidental activation of system 1000 and/or unintended discharge of an actuating portion of the contents 2500 of gas container 2400.

Once gas port 2600 has been punctured, an actuating portion of compressed gas 2500 can escape from container 2400 and flow via gas reservoirs 3000, such as gas channel 3100. The flowing gas can meet and/or apply gas pressure to medicament actuator 4000, which can comprise a pusher 4100, which can travel within a sleeve 1500 defined by walls 1520. Sleeve 1500 can be constructed of metal, stainless steel, aluminum, plastic, polycarbonate, etc. Seals 4200, such as o-rings, can resist gas leakage, such as past pusher 4100 and/or out of housing 1100. Thus, pusher 4100 can function as a piston traveling within a cylinder, although it is not necessarily required that the cross-sectional shape of sleeve 1500 be round.

Medicament actuator 4000 can interface with medicament storage assembly 5000. For example, medicament actuator 4000 can comprise a plurality of plungers 4300, each of which can be capped with a piston 4400 which can sealingly slide and/or move within a corresponding vial 5100 containing a liquid medicament 5200. For example, in response to pressure applied by an actuating portion of the contents 2500 of compressed gas container 2400, pusher 4100 can cause plungers 4300 and/or pistons 4400 to simultaneously move. The number of corresponding sets of plungers 4300, pistons 4400, and/or vials 5100 can be 2, 3, 4, 5, 6, or more. Pistons 4400 can be constructed of a resilient, durable, and/or sealing material, such as a rubber. Each plunger 4300 from the plurality of plungers can define a longitudinal axis, the longitudinal axes (e.g., axes 4310, 4320, 4330, 4340) of the plurality of plungers can be parallel, non-coaxial, and/or co-planar.

Each vial 5100 from the plurality of vials can be substantially cylindrical with a substantially round and/or substantially elliptical cross-sectional shape. Thus, each vial 5100 can define a longitudinal axis, the longitudinal axes of the plurality of vials can be parallel, non-coaxial, and/or co-planar. The longitudinal axis of each vial can be co-axial with the longitudinal axis of its corresponding plunger.

Each vial can be capped at one end with a frangible seal 5300, which can be burst when piston 4400 generates sufficient pressure upon medicament 5200, thereby allowing at least a portion of medicament 5200 to flow out of vial 5100 and into medicament carrier 9000. Thus, the plurality of vials can be fluidly coupleable to the actuating portion of the contents 2500 of gas container 2400.

Medicament carrier 9000 can hold each of vials 5100 and can travel within sleeve 1500. Medicament carrier 9000 can comprise a plurality of channels 9200 adapted to receive medicament 5200 as it exits its respective vial 5100, and direct medicament 5200 to a common conduit 9300. Medicament carrier 9000 can interface with needle assembly 6000 and/or use indicator 7000.

From common conduit 9300, medicament 5200 can enter needle assembly 6000, such as into a single needle 6100 via which medicament can approach needle tip 6200. As medicament actuator 4000 and/or medicament carrier 9000 are driven toward actuator bar 1300, needle tip 6200 can penetrate an end 6400 of needle sheath 6300 and exit actuator bar 1300 at needle port 1340.

Referring to FIG. 5, upon movement of actuation bar 1300 closer to handheld portion 1800, sheath seat 1330 can come in contact with sheath tip 6400, thereby causing sheath 6300 to buckle and/or crumble. As actuator bar 1300 comes in contact with handheld portion 1800, bar stop 1320 can approach medicament carrier stop 9400, while carrier spring 1600 is compressed.

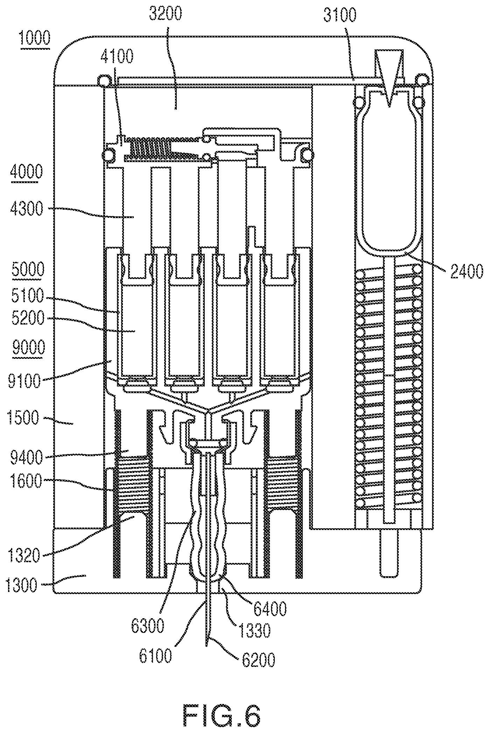

Referring to FIG. 6, as at least a portion of contents 2500 of gas container 2400 escapes, it can flow through channel 3100. The gas, which can still be relatively pressurized, can begin to accumulate behind pusher 4100 to form an expanding gas chamber 3200 and to cause medicament actuator 4000, medicament storage assembly 5000, and medicament carrier 9000 to slide together within sleeve 1500. As medicament actuator 4000, medicament storage assembly 5000, and medicament carrier 9000 slide closer to actuator bar 1300, spring 1600 becomes increasingly compressed between bar stop 1320 and medicament carrier stop 9400. As medicament actuator 4000, medicament storage assembly 5000, and medicament carrier 9000 slide closer to actuator bar 1300, needle tip 6200 can extend further from actuator bar 1300 and sheath 6300 can become further compressed and/or deformed. At its ultimate extension point, needle tip 6200 can extend from housing 1100 from approximately 0.25 millimeters to approximately 20 millimeters, including all values and subranges therebetween, such as up to approximately 2 millimeters, greater than approximately 5 millimeters, from approximately 5.13 millimeters to approximately 9.98 millimeters, etc.

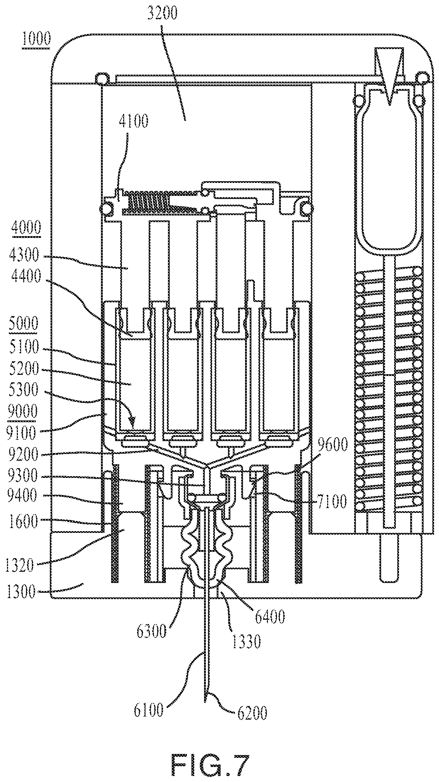

Referring to FIG. 7, as gas chamber 3200 continues to expand, medicament carrier 9000 can be driven until medicament carrier stop 9400 contacts actuator bar stop 1300 thereby resisting further travel of medicament carrier 9000. At that point, additional expansion of gas chamber 3200 can cause medicament actuator 4000, pusher 4100, plungers 4300, and/or pistons 4400 to initiate travel with respect to medicament storage assembly 5000, thereby generating an expulsion pressure in vials 5100, and/or thereby rupturing frangible seals 5300 and allowing medicament 5200 to enter medicament carrier 9000, and begin flowing through medicament channels 9200, medicament conduit 9300, needle 6100, and/or out needle tip 6200 and into a patient. Alternatively, frangible seals 5300 can be replaced and/or augmented by a frangible seal located at or near where medicament conduit 9300 couples to needle 6100. Frangible seals 5300 can be constructed of a thin, taught, resilient, durable, and/or sealing material potentially having a predetermined yield strength, such as a rubber, such as chromo butyl rubber, and/or of a relatively brittle material potentially having a predetermined yield strength, such as ceramic, certain plastics, such as polystyrene, etc.

As medicament carrier stop 9400 contacts actuator bar stop 1320, medicament carrier hooks 9600 can engage with engagement receivers 7100 in use indicator 7000.

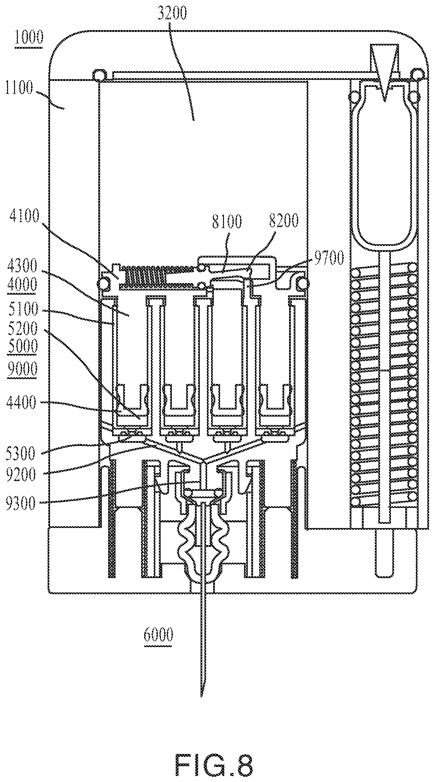

Referring to FIG. 8, as gas chamber 3200 continues to expand, medicament actuator 4000, pusher 4100, plungers 4300, and/or pistons 4400 can continue moving until they complete their travel within medicament storage assembly 5000, thereby expelling a predetermined dose of medicament 5200 from vials 5100, out of needle assembly 6000, external to housing 1100, and/or into the patient. As gas chamber 3200 reaches its maximum size, medicament actuator 4000, pusher 4100, plungers 4300, and/or pistons 4400 can continue moving until they complete their travel with respect to medicament carrier 9000, thereby causing gas release actuator 9700 to engage with gas relief valve 8200. Engagement of gas release actuator 9700 with gas relief valve 8200 can cause gas within gas chamber 3200 to exit gas chamber 3200, discharge away from pistons 4400, and/or exhaust from system 1000 and/or housing 1100, such as via status indicator 1400 and/or a gas escape port located on housing 1100).

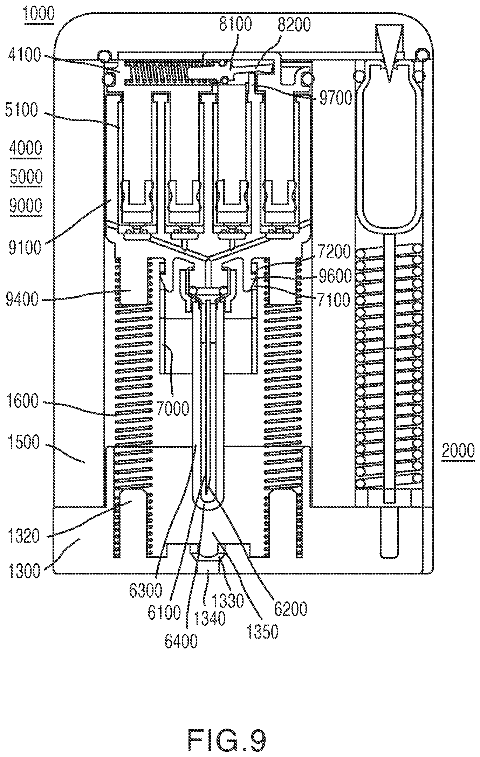

Referring to FIG. 8 and FIG. 9, as sufficient gas is vented from gas chamber 3200, the pressure applied by the gas in gas chamber 3200 can decrease until the force applied by the gas on medicament actuator 4000 is less than the force of compressed spring 1600. Thus, spring(s) 1600 can begin to expand, thereby moving medicament carrier 9000, vial assembly 5000, and medicament actuator 4000 away from actuator bar 1300 and helping to exhaust gas from gas chamber 3200. As medicament carrier 9000 moves, use indicator 7000 can travel with it, due to the engaged relationship of medicament carrier hooks 9600 and engagement receivers 7100 and/or engagement catches 7200 in use indicator 7000. As use indicator 7000 moves away from actuation bar 1300, sheath 6300 can travel with it, thereby creating a gap between sheath tip 6400 and needle port 1340, and thereby exposing a previously non-visible colored portion 1350 of actuation bar 1300 and/or providing an indication that system 1000 has been used (and likely substantially exhausted of its medicament), thereby discouraging any further attempts to use system 1000.

As medicament carrier 9000 moves away from actuator bar 1300, needle 6100 can retract into sheath 6300 which un-buckles and/or un-deforms towards its original shape. Eventually, needle 6100 can retract completely within the boundaries of housing 1100, thereby tending to prevent accidental needle sticks after the initial injection and/or potentially reducing and/or eliminating a sharps hazard.

In some embodiments, system actuator 2000 can comprise a finger triggered, twistable, pivotable, and/or lever-operated mechanism. For example, system actuator 2000 can comprise a twistable handle that can screw into gas port 2600. In some embodiments, system actuator 2000 can be a finger trigger located on a side of the housing.

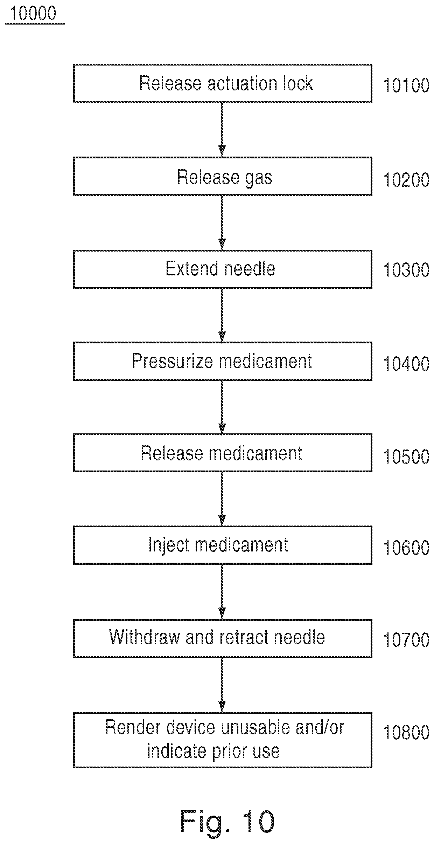

FIG. 10 is a flowchart of an embodiment of a method 10000 for operating a medicament delivery apparatus. At activity 10100, an actuation lock for the apparatus is released. At activity 10200, an actuating portion of the contents of a compressed gas container are released. At activity 10300, via pressure provided by the released gas, a needle is extended from the apparatus. At activity 10400, via pressure provided by the released gas, a piston applies pressure to a medicament stored in one of a plurality of vials. At activity 10500, a frangible seal containing the medicament in the vial is burst. At activity 10600, the medicament flows from the vial, through the needle, and into a patient. At activity 10700, once a predetermined dose is expelled and/or injected, the needle is withdrawn from the patient and/or retracted into the pre-use bounds of the apparatus. At activity 10800, the apparatus is rendered unusable for additional injections and/or indicated as previously utilized.

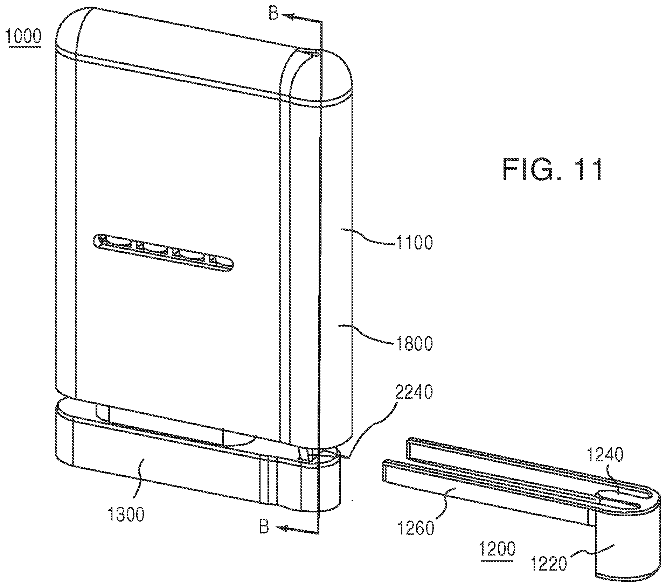

FIG. 11 is a perspective view of an embodiment of system 1000, showing actuation guard 1200 removed from housing 1100, so that actuation guard 1200 no longer separates actuator bar 1300 from handheld portion 1800. Actuation guard 1200 can comprise a grippable portion 1220 that can be gripped by a user to pull actuation guard 1200 away from housing 1100, thereby allowing system 1000 to be activated, such as via slapping actuator bar 1300 against a thigh of the user. Actuation guard 1200 can comprise an actuation stick separator portion 1240, that can keep separate actuation stick prongs 2240 when actuation guard 1200 is installed on housing 1100. Actuation guard 1200 can comprise a guard portion 1260 that can separate actuator bar 1300 from handheld portion 1800 when system 1000 is not in use and/or when system 1000 has not been used.

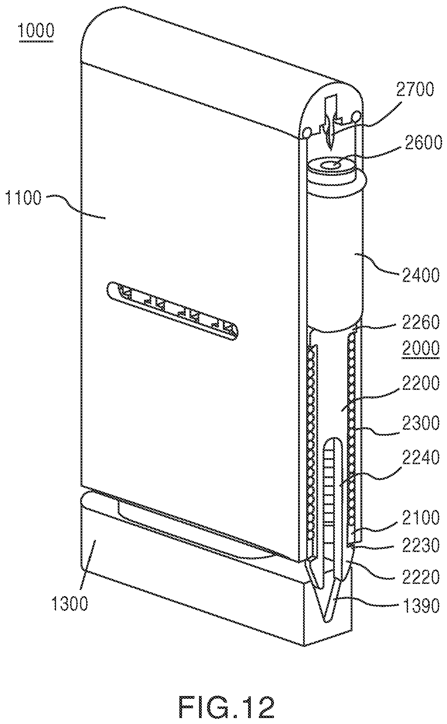

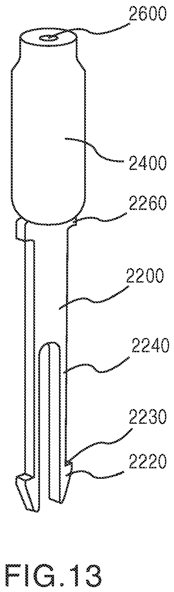

FIG. 12 is a perspective cross-sectional view taken along line B-B of FIG. 11, and FIG. 13 is a perspective view of an embodiment of actuation stick 2200. Referring to FIGS. 12 and 13, system 1000 can comprise housing 1100, actuation bar 1300, and system actuator 2000, which can comprise prong squeezer 1390, actuation stick 2200, prong retainer 2100, spring 2300, upper spring retainer 2260, gas container 2400, gas port 2600, and/or puncturer 2700. When actuation bar 1300 is pressed firmly against a user's body, such as via slapping housing actuation bar against the user's thigh, buttocks, and/or arm, prong squeezer 1390 can urge prong tips 2220 of prongs 2240 of actuation stick 2200 toward one another. Note that prong tips 2200 can have a triangular, wedge, angular, and/or frusto-conical shape. As prongs tips 2220 slide along the angled V-groove of prong squeezer 1390, prong catches 2230 can substantially lose contact with prong retainer 2100. This can allow compressed spring 2300 to rapidly urge actuation stick 2200 and gas container 2400 toward puncturer 2700, which can penetrate gas port 2600, thereby allowing gas to escape from gas container 2400. Although any of many different types of gas containers can be utilized, an example of a suitable gas container can be obtained from Leland Limited, Inc. of South Plainfield, NJ.

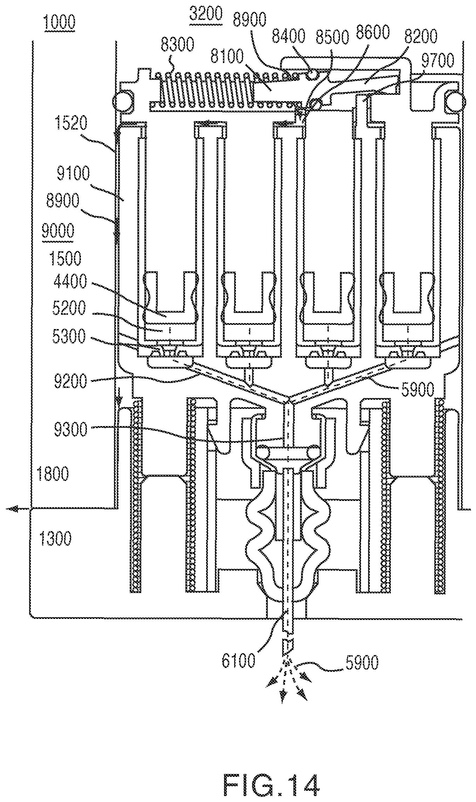

FIG. 14 is a cross-sectional view of an embodiment of gas venting mechanism 8000 of system 1000 taken along line A-A of FIG. 3. System 1000 can comprise handheld portion 1800, actuator bar 1300, sleeve 1500. As pistons 4440 near the limit of their travels, medicament 5200 can be expelled along medicament path 5900, which can extend past frangible seal 5300, through medicament channels 9200, medicament conduit 9300, and needle 6100, and into the body of a user, such as subcutaneously, intramuscularly, and/or at a depth of from approximately 0.25 millimeters to approximately 20 millimeters, including all values and subranges therebetween, such as up to 2 millimeters, greater than 5 millimeters, etc.

As pistons 4440 near the limit of their travels, engagement of gas release actuator 9700 with gas relief valve 8200 can cause compressed spring 8300 to move valve arm such that o-ring 8400 is urged away from its seat 8500. This movement can reveal a passage 8600, via which gas can exit gas chamber 3200 along gas exhaust path 8900, which can extend between sleeve inner walls 1520 and outer walls 9100 of medicament carrier 9000. Eventually, gas exhaust path 8900 can extend between handheld portion 1800 and actuator bar 1300. Likewise, an alternative embodiment of valve 8200, made of rubber or any other resilient material, can be placed across seat 8500 to provide a seal that, once gas release actuator 9700 interacts with valve 8200, allows valve 8200 to bend or flap upwards away from seat 8500, causing the gas to escape via passage 8600.

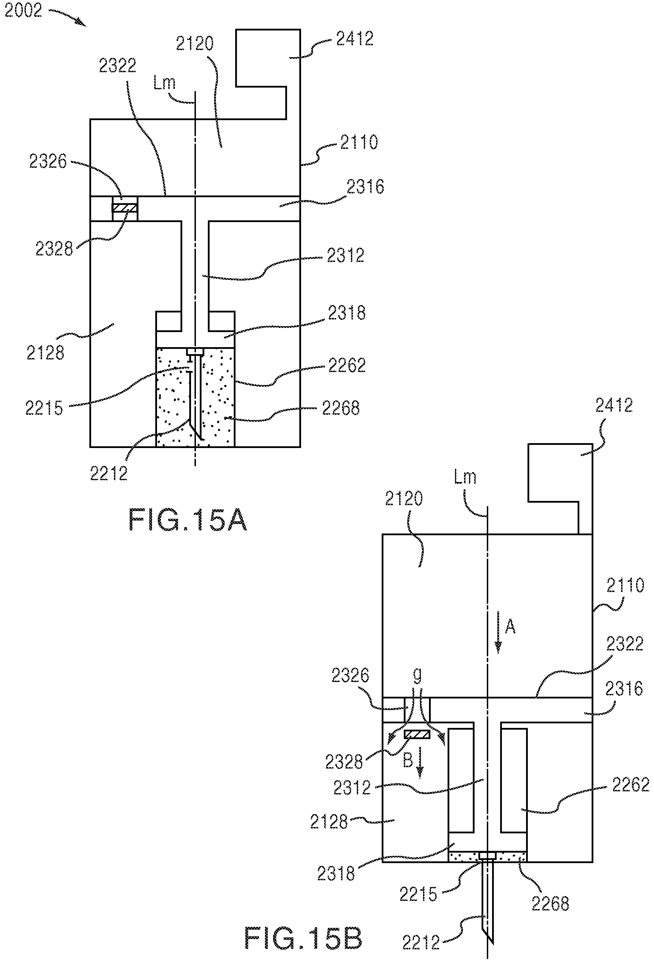

FIGS. 15A and 15B are schematic illustrations of an auto-injector 2002 according to an embodiment of the invention in a first configuration and a second configuration, respectively. The auto-injector 2002 includes a housing 2110, a medicament container 2262, a movable member 2312, a gas relief valve 2328 and a compressed gas source 2412. The medicament container 2262, which can be, for example, a pre-filled cartridge, a vial, an ampule or the like, is fixedly disposed within the housing 2110 and defines a longitudinal axis Lm. The medicament container 2262 contains a medicament 2268, such as, for example, epinephrine.

The movable member 2312 includes a proximal end portion 2316 and a distal end portion 2318. The proximal end portion 2316 includes a surface 2322 that, together with the housing 2110, defines a gas chamber 2120. Said another way, the surface 2322 defines a portion of a boundary of the gas chamber 2120. The proximal end portion 2316 defines an opening 2326 therethrough, which is in fluid communication between the gas chamber 2120 and an area outside of the gas chamber 2128. The distal end portion 2318 is movably disposed within the medicament container 2262 along the longitudinal axis Lm, as shown by the arrow A. A needle 2212 is coupled to the distal end 2318 of the movable member 2312. The needle 2212 defines a lumen (not shown) and a side opening 2215.

The gas relief valve 2328 is coupled to the movable member 2312 such that it can selectively allow fluid communication between the gas chamber 2120 and the area outside of the gas chamber 2128. The gas relief valve 2328 can include, for example, a movable membrane, a frangible seal, a spring-loaded gas relief valve body or the like.

In use, when the auto-injector 2002 is actuated, the gas chamber 2120 is placed in fluid communication with the compressed gas source 2412, thereby allowing a pressurized gas to flow into the gas chamber 2120. In response to a force produced by the pressurized gas on the surface 2322 of the movable member 2312, the movable member 2312 moves within the housing 2110 and the medicament container 2262, as indicated by arrow A. As a result, as shown in FIG. 15B, the needle 2212 is extended through the housing 2110. The movement of the movable member 2312 also forces the medicament 2268 through the side opening 2215 and into the lumen (not shown) defined by the needle 2212. In this manner, the medicament injection occurs while the needle 2212 is being extended from the housing 2110 (i.e., while the needle 2212 is being inserted into the body).

In use, the pressure of the pressurized gas within the gas chamber 2120 can be controlled by the gas relief valve 2328. As shown in FIG. 15B, the gas relief valve 2328 is actuated as indicated by the arrow B, thereby allowing pressurized gas to flow from the gas chamber 2120 to the area outside of the gas chamber 2128 through the opening 2326, as shown by the arrows g. Although the gas relief valve 2328 is shown as being actuated after substantially all of the medicament 2268 has been injected, in other embodiments, the gas relief valve 2328 can be actuated at any time during the injection event. For example, in some embodiments, the gas relief valve 2328 can be actuated as the injection event is beginning to control the rate of needle insertion and/or medicament injection. In other embodiments, the gas relief valve 2328 can be actuated at the end of the injection event to allow the needle 2212 to be retracted to a position within the housing 2110. In yet other embodiments, the gas relief valve 2328 can be actuated upon completion of the injection event to prevent residual gas from undesirably building up within the gas chamber 2120.

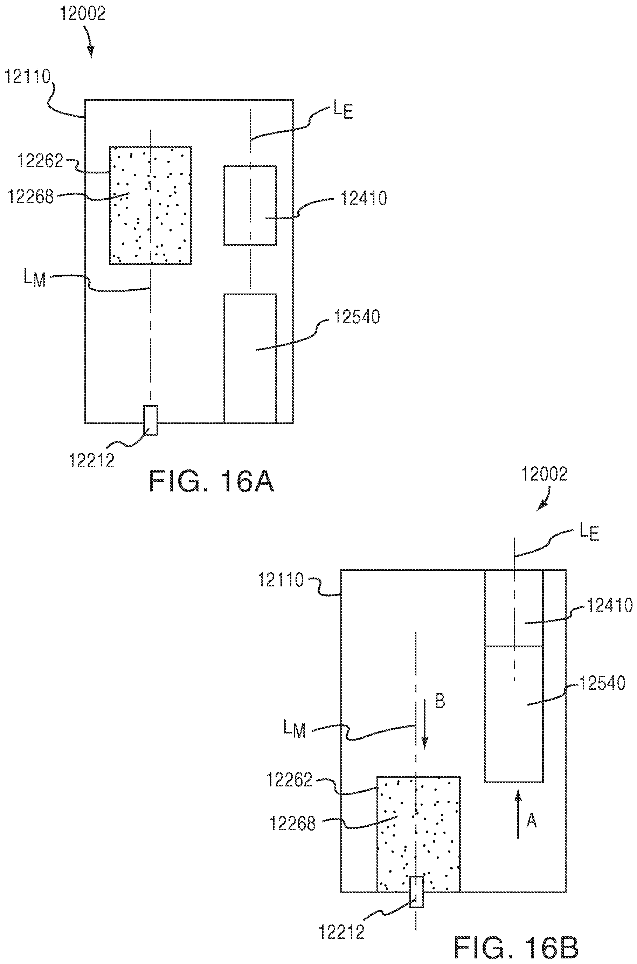

FIGS. 16A and 16B are schematic illustrations of an auto-injector 12002 according to an embodiment of the invention in a first configuration and a second configuration, respectively. The auto-injector 12002 includes a housing 12110 that contains a medicament container 12262, an energy storage member 12410, a release member 12540 and an injection member 12212. The medicament container 12262, which can be, for example, a pre-filled cartridge, a vial, an ampule or the like, is movably disposed within the housing 12110. The medicament container 12262 contains a medicament 12268, such as, for example, epinephrine. As illustrated, the medicament container 12262 can be moved, as indicated by arrow B in FIG. 16B, along its longitudinal axis Lm between a first position (FIG. 16A) and a second position (FIG. 16B). When the medicament container 12262 is in its first (or retracted) position, the medicament container 12262 is spaced apart from the injection member 12212. When the medicament container 12262 is in the second (or advanced) position, the medicament container 12262 is placed in fluid communication with the injection member 12212. In this manner, when the medicament container 12262 is in the second (or advanced) position, the medicament 12268 can be conveyed via the injection member 12212 from the medicament container 12262 into a body of a patient. The injection member 12212 can be, for example, a needle, a nozzle or the like.

The energy storage member 12410, which can be any suitable device for storing energy, such as, for example, a spring, a battery, a compressed gas cylinder or the like, is also movably disposed within the housing 12110. As shown, the energy storage member 12410 defines a longitudinal axis Le that is offset from the longitudinal axis Lm of the medicament container 12262. The energy storage member 12410 can be moved, as indicated by arrow A in FIG. 16B, within the housing 12110 along its longitudinal axis Le between a first position (FIG. 16A) and a second position (FIG. 16B). When the energy storage member 12410 is in its first position, the energy storage member 12410 has a first potential energy. When the energy storage member 12410 is in its second position, the energy storage member 12410 has a second potential energy that is less than the first potential energy. When the energy storage member 12410 moves from its first position to its second position, it converts at least a portion of its first potential energy into kinetic energy to move the medicament container 12262 between its first position and its second position.

Said another way, the movement of the energy storage member 12410 from its first position to its second position results in the production of a force that acts upon the medicament container 12262 to move the medicament container 12262 between its first position and its second position. The non-coaxial relationship between the longitudinal axis Lm of the medicament container 12262 and the longitudinal axis Le of the energy storage member 12410 allows the medicament container 12262 and the energy storage member 12410 to be arranged within the housing 12110 in any number of different configurations. In this manner, the auto-injector 12002 can have any number of different sizes and shapes, such as, for example, a substantially rectangular shape.

The release member 12540 is disposed within the housing 12110 and is configured to selectively deploy the energy storage member 12410 from its first position to its second position. The release member 12540 can be any suitable mechanism for moving the energy storage member 12410, such as, for example, a mechanical linkage, a spring-loaded rod or the like. In this manner, a user can actuate the auto-injector by manipulating a portion of the release member 12540.

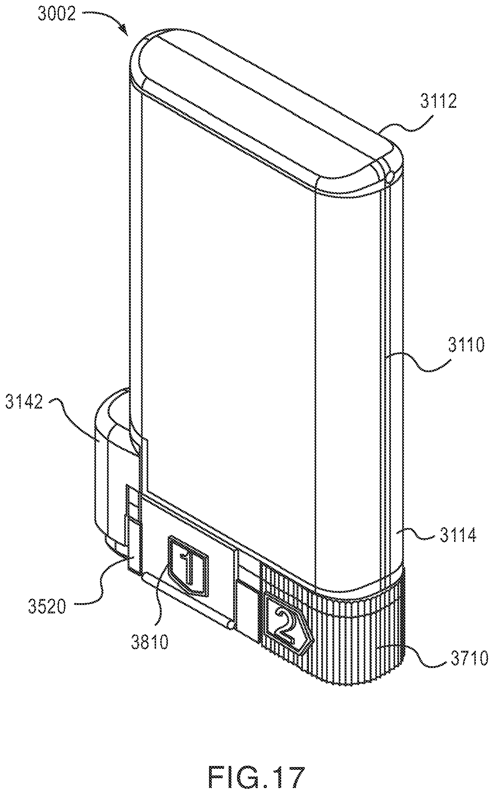

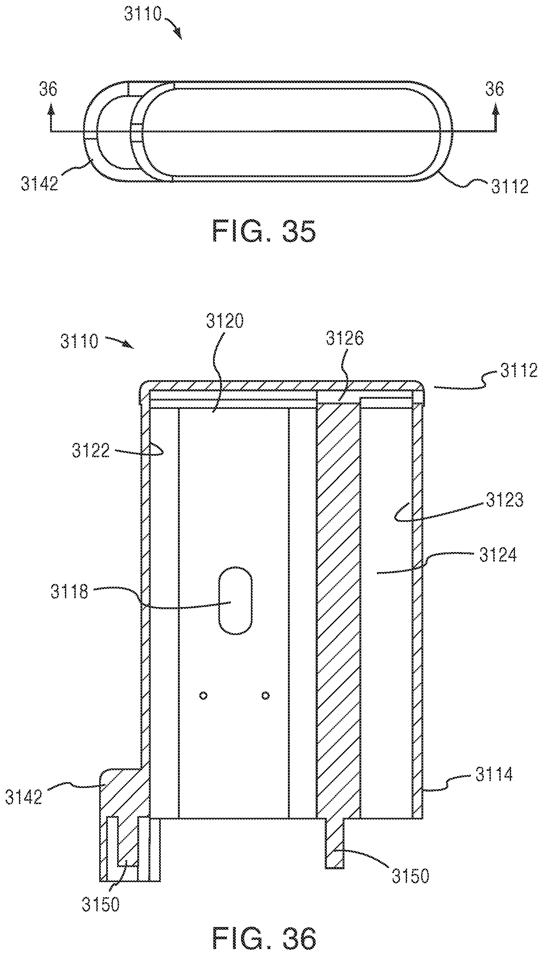

FIG. 17 is a perspective view of an auto-injector 3002 according to an embodiment of the invention in a first configuration. The auto-injector 3002 includes a housing 3110 having a proximal end portion 3112 and a distal end portion 3114. The distal end portion 3114 of the housing 3110 includes a protrusion 3142 to help a user grasp and retain the housing 3110 when using the auto-injector 3002. Said another way, the protrusion 3142 is configured to prevent the auto-injector 3002 from slipping from the user's grasp during use. A base 3520 is movably coupled to the distal end portion 3114 of the housing 3110. A needle guard assembly 3810 is removably coupled to the base 3520. Similarly, a safety lock 3710 is removably coupled to the base 3520. To inject a medicament into the body, the distal end portion 3114 of the housing is oriented towards the user such that the base 3520 is in contact with the portion of the body where the injection is to be made. The base 3520 is then moved towards the proximal end 3112 of the housing 3110 to actuate the auto-injector 3002. The housing 3110 also includes a transparent status window 3118 (see FIG. 36) to allow a user to determine the status of the auto-injector 3002 or the medicament contained therein.

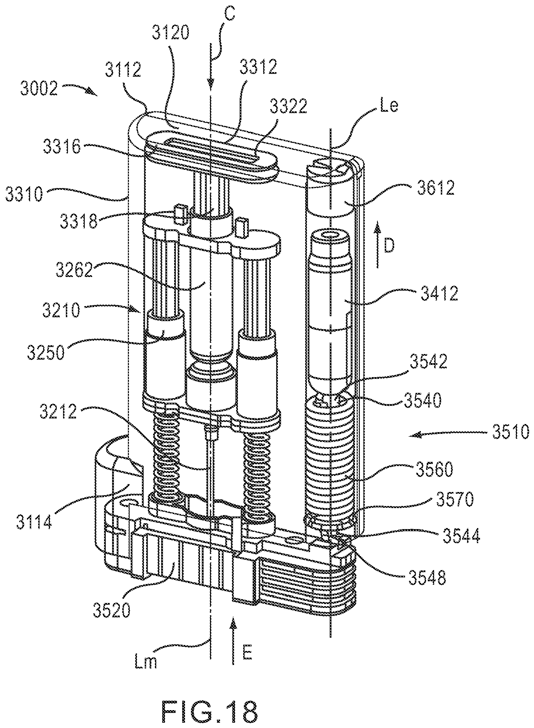

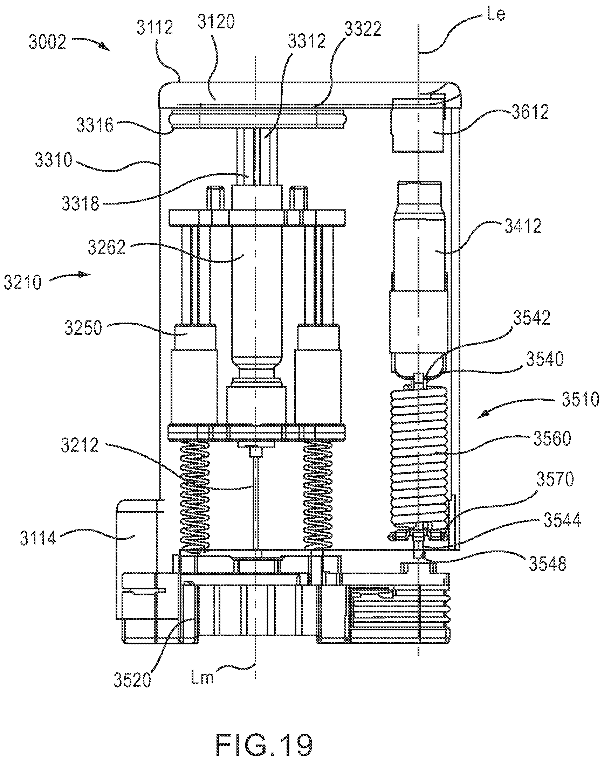

FIG. 18 is a perspective view of the auto-injector 3002 showing the housing 3110 in phantom lines so that the components contained within the housing 3110 can be more clearly seen. For clarity, FIG. 18 shows the auto-injector 3002 without the needle guard assembly 3810 and the safety lock 3710. Similarly, FIG. 19 is a front view of the auto-injector 3002 showing the housing 3110 in phantom lines. The auto-injector 3002 includes a medicament injector 3210 and a movable member 3312 engaged with the medicament injector 3210, each of which are disposed within the housing 3110. The auto-injector 3002 also includes a system actuator 3510, a compressed gas container 3412 and a gas release mechanism 3612.

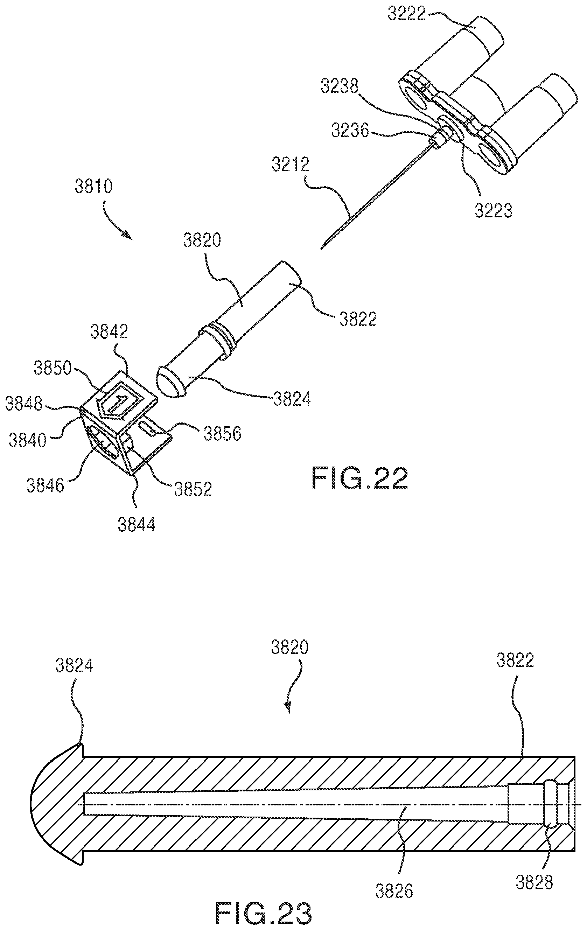

The medicament injector 3210 includes a carrier 3250 that is movable within the housing 3110, a medicament container 3262 and a needle 3212. The medicament container 3262 is coupled to the carrier 3250. The needle 3212 is disposed within a needle hub portion 3223 (see FIG. 22) of the carrier to allow the needle 3212 to be placed in fluid communication with the medicament container 3262 during an injection event.

The movable member 3312 includes a proximal end portion 3316 and a distal end portion 3318. The proximal end portion 3316 includes a surface 3322 that, together with the housing 3110, defines a gas chamber 3120. Said another way, the surface 3322 defines a portion of a boundary of the gas chamber 3120. The distal end portion 3318 is disposed within the medicament container 3262. In use, the movable member 3312 moves towards the distal end portion 3114 of the housing 3110, as indicated by arrow C, in response to a force produced by a pressurized gas on the surface 3322 of the movable member 3312. As a result, the movable member 3312 and the medicament injector 3250 are moved towards the distal end portion 3114 of the housing 3110, thereby exposing the needle 3212 from the housing 3110. The movable member 3312 then continues to move within the medicament container 3262 to expel a medicament from the medicament container 3262 through the needle 3212.

The auto-injector 3002 is actuated by the system actuator 3510, which is configured to move the compressed gas container 3412 into contact with the gas release mechanism 3612. The gas release mechanism 3612 punctures a portion of the compressed gas container 3412 to release the pressurized gas contained therein into the gas chamber 3120 defined by the housing 3110.

The system actuator 3510 includes a rod 3540, a spring 3560 and a spring retainer 3570. The rod 3540 has a proximal end portion 3542 and a distal end portion 3544. The proximal end portion 3542 of the rod 3540 is coupled to the compressed gas container 3412. The distal end portion 3544 of the rod 3540 is coupled to the spring retainer 3570 by two projections 3548, which can be moved inwardly towards each other to decouple the rod 3540 from the spring retainer 3570, as discussed below.

The spring 3560 is disposed about the rod 3540 in a compressed state such that the spring 3560 is retained by the proximal end portion 3542 of the rod 3540 and the spring retainer 3570. In this manner, the rod 3540 is spring-loaded such that when the distal end portion 3544 of the rod 3540 is decoupled from the spring retainer 3570, the force of the spring 3560 causes the rod 3540, and therefore the compressed gas container 3412, to move proximally as indicated by arrow D and into contact with the gas release mechanism 3612.

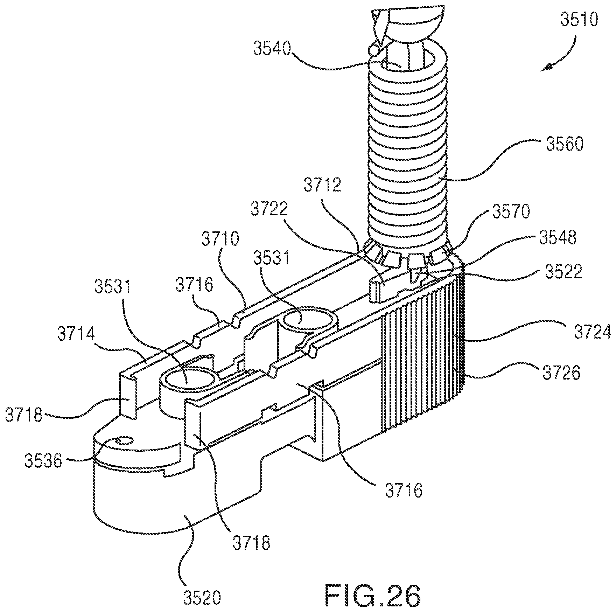

The base 3520 defines an opening 3522 (shown in FIG. 26) configured to receive a portion of the projections 3548 when the base is moved towards the proximal end 3112 of the housing 3110, as indicated by arrow E. When the projections 3548 are received within the opening 3522, they are moved together causing the distal end portion 3544 of the rod 3540 to be released from the spring retainer 3570. In some embodiments, the opening 3522 extends though at least a portion of the base 3520. In some embodiments, the opening 3522 extends completely through the base 3520, for example, such that a locking portion of a safety guard (discussed in detail below) can be inserted through the opening.

As shown in FIGS. 18 and 19, the medicament injector 3210 defines a longitudinal axis Lm that is non-coaxial with the longitudinal axis Le defined by the compressed gas container 3412. Accordingly, the medicament injector 3210, the compressed gas container 3412 and the system actuator 3510 are arranged within the housing 3110 such that the housing has a substantially rectangular shape. Moreover, the non-coaxial relationship between the medicament injector 3210 and the compressed gas container 3412 allows the auto-injector 3002 to be actuated by manipulating the base 3520, which is located at the distal end portion 3114 of the housing 3110.

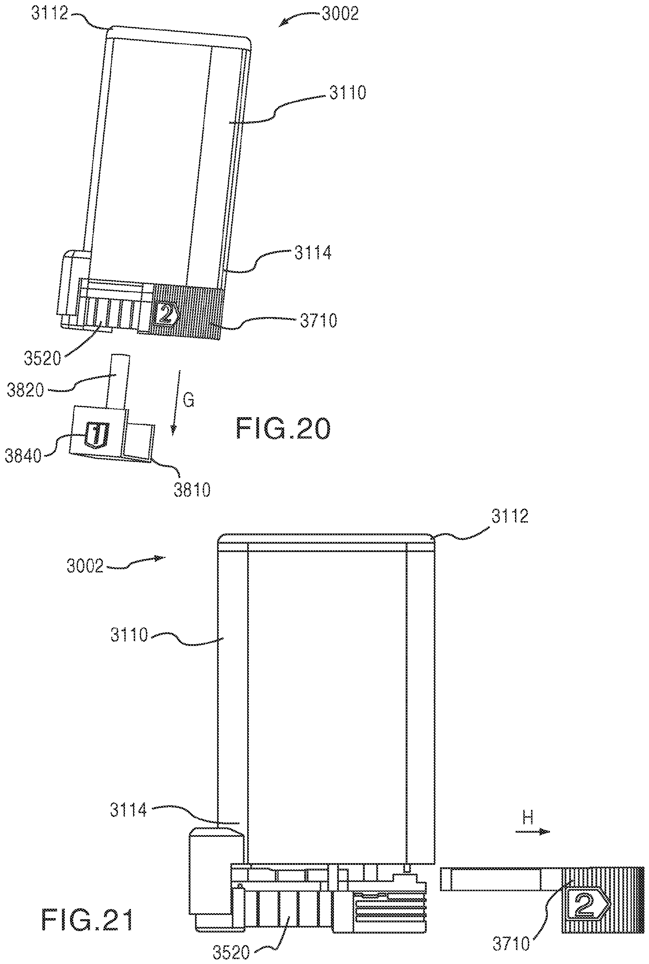

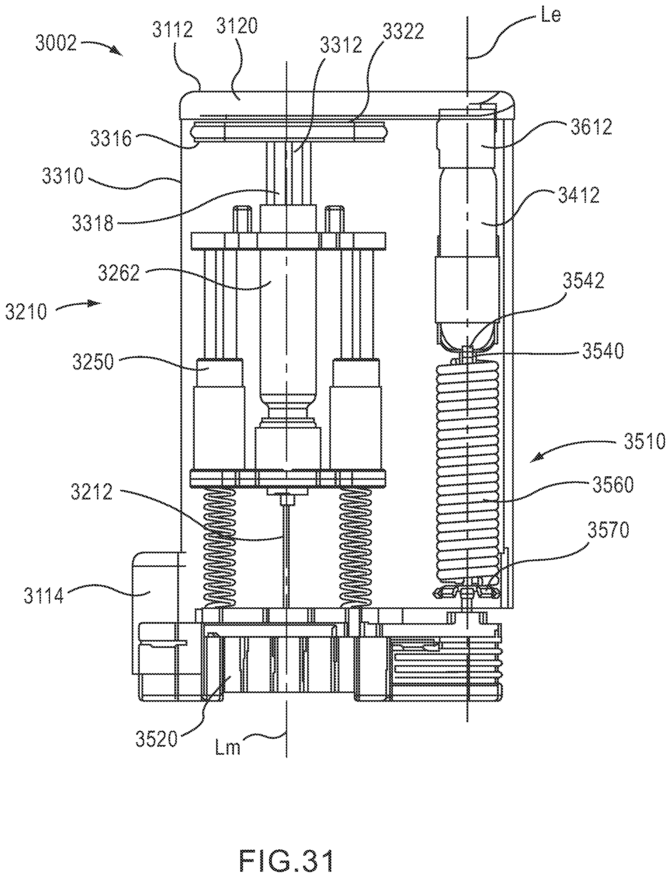

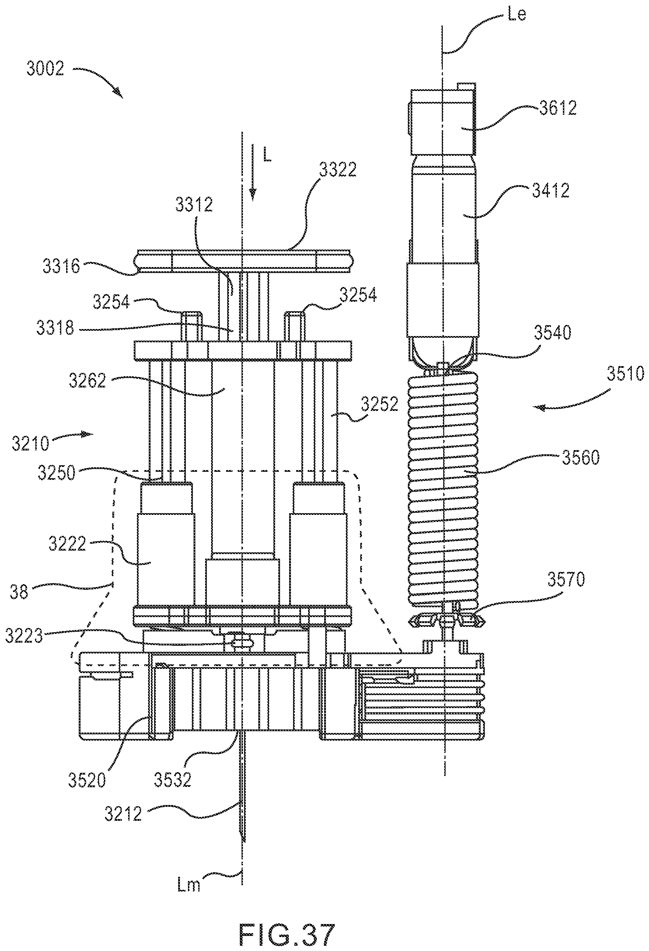

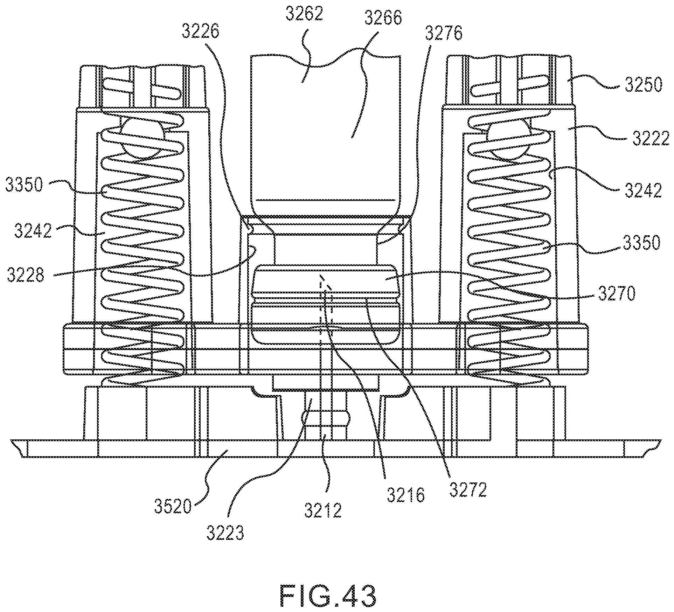

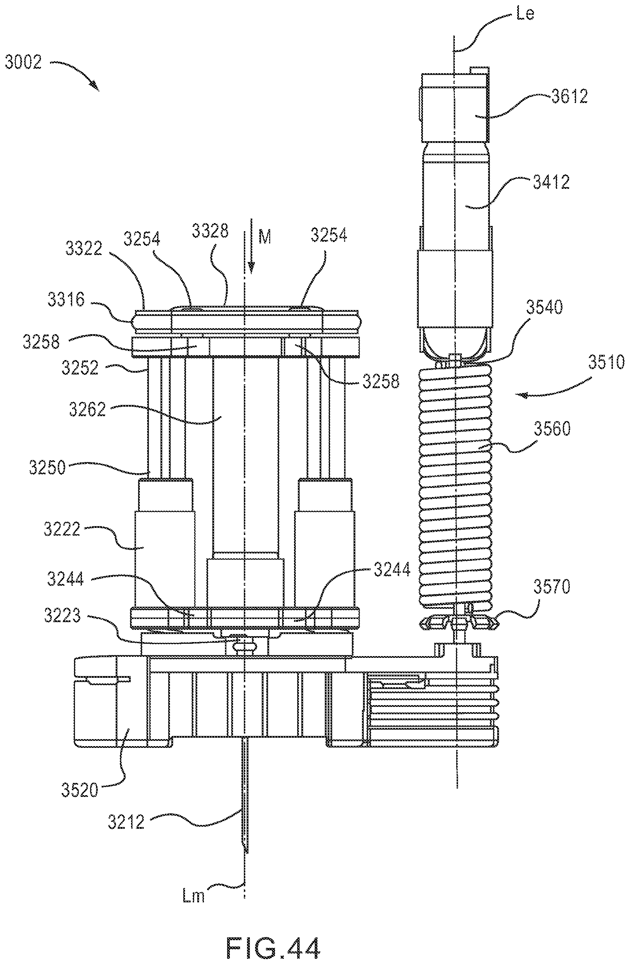

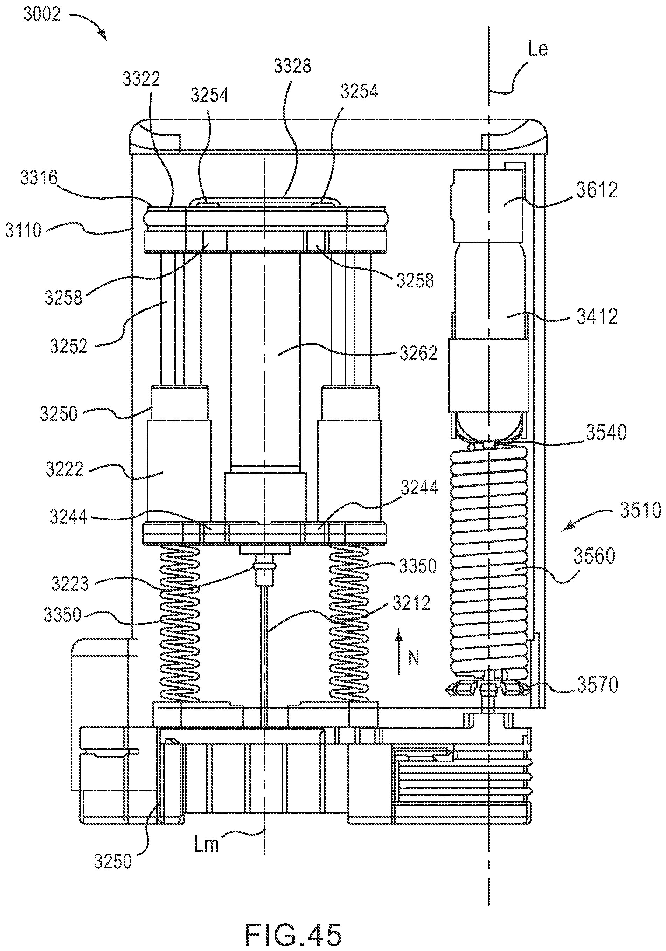

As discussed above, the use and actuation of the auto-injector 3002 includes several discrete operations. First, the auto-injector 3002 is enabled by removing the needle guard 3810 and the safety lock 3710 (see FIGS. 20 and 21). Second, the auto-injector 3002 is actuated by moving the base 3520 proximally towards the housing 3110. Third, when actuated, the compressed gas container 3412 engages the gas release mechanism 3612, which causes the pressurized gas to be released into the gas chamber 3120 (see FIG. 31). Fourth, the pressurized gas produces a force that causes the movable member 3312 and the medicament injector 3210 to move distally within the housing 3110 (see FIG. 37). The movement of the medicament injector 3210 causes the needle 3212 to extend from distal end portion 3114 of the housing 3110 and the base 3520. This operation can be referred to as the "needle insertion" operation. Fifth, when the medicament injector 3210 has completed its movement (i.e., the needle insertion operation is complete), the movable member 3312 continues to move the medicament container 3262 distally within the carrier 3250. The continued movement of the medicament container 3262 places the needle 3212 in fluid communication with the medicament container 3262, thereby allowing the medicament to be injected (see FIG. 43). Sixth, the force from the pressurized gas causes the movable member 3312 to move within the medicament container 3262, thereby expelling the medicament through the needle 3212 (see FIG. 44). This operation can be referred to as the "injection operation." Seventh, upon completion of the injection, the pressurized gas is released from the gas chamber 3120, thereby allowing the medicament injector 3210 and the movable member 3312 to be moved proximally within the housing. This operation can be referred to as the "retraction operation" (see FIG. 45). A detailed description of the components contained in the auto-injector 3002 and how they cooperate to perform each of these operations is discussed below.

Prior to use, the auto-injector 3002 must first be enabled by first removing the needle guard 3810 and then removing the safety lock, or locking member, 3710. As illustrated by arrow G in FIG. 20, the needle guard 3810 is removed by pulling it distally. Similarly, as illustrated by arrow H in FIG. 21, the safety lock 3710 is removed by pulling it substantially normal to the longitudinal axis Le of the compressed gas container 3412. Said another way, the safety lock 3710 is removed by moving it in a direction substantially normal to the direction that the needle guard 3810 moved or to the longitudinal axis Lm of the needle (as shown in FIG. 20) is moved. As described in more detail herein, in some embodiments, the needle guard 3810 and the safety lock 3710 are cooperatively arranged to prevent the safety lock 3710 from being removed before the needle guard 3810 has been removed. Such an arrangement prevents the auto-injector 3002 from being actuated while the needle guard 3810 is in place.

As illustrated in FIG. 22, the needle guard 3810 includes a sheath 3820 and a sheath retainer 3840. The sheath 3820 has a proximal end portion 3822 and a distal end portion 3824 and defines an opening 3826 configured to receive a portion of the needle 3212 when the needle guard 3810 is in a first (or installed) position. Said another way, the sheath 3820 is an inner member of the needle guard 3810 configured to substantially cover at least a portion of the needle 3212 when the needle guard is in a first position, and the sheath retainer 3840 is an outer member of the needle guard.

As illustrated in FIG. 23, the sheath 3820 further defines a recessed portion 3828 within the opening 3826 that engages a corresponding protrusion 3238 defined by an outer surface 3236 of the needle hub 3223. In this manner, when the needle guard 3810 is in its first position, the sheath 3820 is removably coupled to the needle hub 3223. In some embodiments, the recessed portion 3828 and the protrusion 3238 form a seal that is resistant to microbial penetration.

The sheath 3820 can be constructed from any suitable material. For example the sheath can be constructed from polyethylene, including high density polyethylene, polypropylene, polytetrafluoroethylene, thermoplastic polyurethane, rubber or any other elastomer or polymer. In some embodiments, the sheath 3820 is constructed from a rigid material. A rigid needle sheath can reduce the likelihood of needle sticks during the manufacturing process and can inhibit crumpling of the sheath around the needle during insertion of the needle into bodily tissue. In other embodiments, the sheath can be constructed from a flexible material. In some embodiments, the sheath 3820 is constructed from a material configured to resist or substantially prevent microbial penetration therethrough, and thus can maintain sterility of a needle received therein.

The sheath 3820 can be configured for use with one or more sterilization methods. In other words, the sheath can be configured to allow sterilization of the needle when the sheath is disposed over the needle and coupled to the needle hub. In some embodiments, the sheath 3820 is configured to allow a sterilant gas or other sterilizing agent to pass therethrough. For example, the sheath can include a valve configured to allow passage of the sterilant gas. In another example, the sheath is constructed of a porous material, such as a porous material configured to allow passage of the sterilant gas through the material while preventing microbes from passing therethrough.

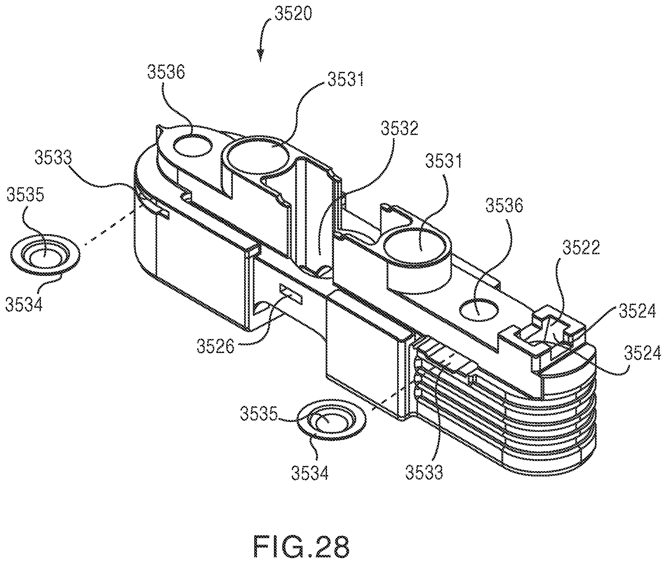

The sheath retainer 3840 has a proximal portion 3842 and a distal portion 3844. The proximal portion 3842 of the sheath retainer 3840 includes a protrusion 3856 that engages a corresponding recess 3526 in the base 3520 (see FIG. 28) to removably couple the sheath retainer 3840 to the base 3520. The distal portion 3844 of the sheath retainer 3840 defines an opening 3846 through which the distal end portion 3824 of the sheath 3820 is disposed. The distal portion 3844 of the sheath retainer 3840 includes a series of retaining tabs 3852 that engage the distal end portion 3824 of the sheath 3820 to couple the sheath 3820 to the sheath retainer 3840. In this manner, when the sheath retainer 3840 is moved distally away from the base 3520 into a second (or removed) position, as shown in FIG. 20, the sheath 3820 is removed from the needle 3212. Moreover, this arrangement allows the sheath 3820 to be disposed about the needle 3212 independently from when the sheath retainer 3840 is coupled to the sheath 3820. As such, the two-piece construction of the needle guard provides flexibility during manufacturing, for example, because the sheath retainer can be installed after the sheath has been disposed about the needle and the needle sterilized. The distal portion 3844 of the sheath retainer 3840 also includes a protrusion 3848 to aid the user when grasping the needle guard 3810.

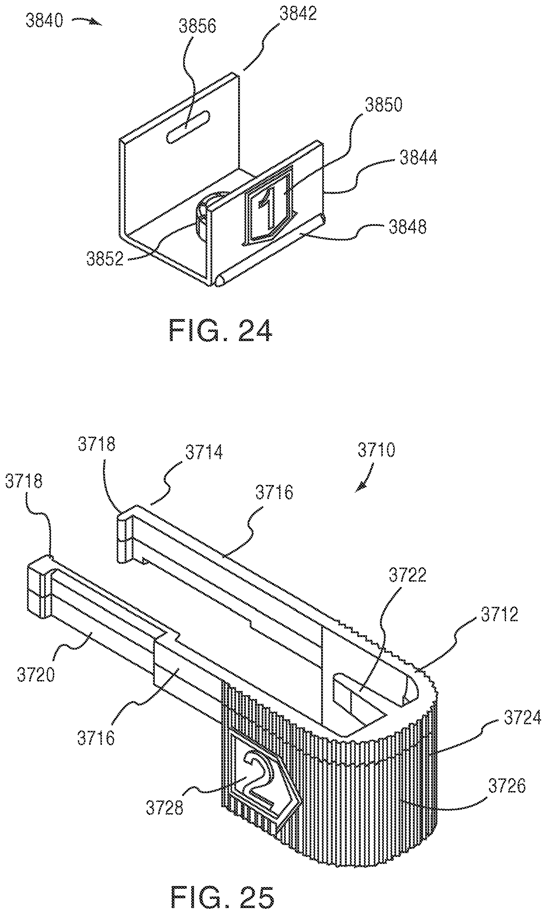

When the needle guard 3810 is in its first (or installed) position, the sheath retainer 3840 is disposed within a recess 3720 defined by one of the extended portions 3716 of the safety lock 3710 (see FIG. 25). This arrangement prevents the safety lock 3710 from being removed when the needle guard 3810 is in its first position, which in turn, prevents the auto-injector 3002 from being actuated when the needle guard 3810 is in its first position.

The outer surface of the needle guard 3810 (or sheath retainer 3840 specifically) includes an indicia 3850 to instruct the user in operating the auto-injector 3002. As shown in FIG. 24, the indicia 3850 includes a numeral to indicate the order of operation and an arrow to indicate the direction in which the needle guard 3810 should be moved. In some embodiments, the indicia 3850 can include different colors, detailed instructions or any other suitable indicia to instruct the user. In other embodiments, the indicia 3850 can protrude from the sheath retainer 3840 to aid the user when grasping the needle guard 3810.

After the needle guard 3810 is removed, the user must then remove the safety lock 3710, as indicated in FIG. 21. As shown in FIG. 25, the safety lock 3710 is a U-shaped member having a first end 3712 and a second end 3714. The second end 3714 of the safety lock 3710 includes two extended portions 3716, each of which includes an inwardly facing protrusion 3718. When the safety lock 3710 is in its first (or locked) position, the extended portions 3716 extend around a portion of the base 3520 to space the base 3520 apart from the distal end portion 3114 of the housing 3110. As shown in FIG. 26, the protrusions 3718 are configured engage a portion of the base 3520 to removably couple the safety lock 3710 in its first position.

One of the extended portions 3716 defines a recess 3720 that receives the sheath retainer 3840 when the needle guard 3810 is in its first position, as discussed above. Although only one extended portion 3716 is shown as including a recess 3720, in some embodiments both extended portions 3716 can include a recess 3720 to receive the sheath retainer 3840. The safety lock 3710 can be engaged with the needle guard 3810 to prevent movement of the safety lock 3710 when the needle guard 3810 is in place in any suitable manner. For example, in some embodiments, the sheath retainer includes protrusions that are received within corresponding openings defined by the safety lock. In some embodiments, the safety lock includes protrusions that are received within corresponding openings defined by the sheath retainer.

The first end 3712 of the safety lock 3710 includes a locking protrusion 3722 that extends inwardly. As shown in FIG. 26, when the safety lock 3710 is in its first position, the locking protrusion 3722 extends between the projections 3548 of the rod 3540 and obstructs an opening 3522 of the base 3520. In this manner, when the safety lock 3710 is in its first position, the base 3520 cannot be moved proximally to allow the projections 3548 to be received within the opening 3522. The arrangement of the locking protrusion 3722 also prevents the projections 3548 from being moved inwardly towards each other. Accordingly, when the safety lock 3710 is in its first position, the auto-injector 3002 cannot be actuated.

The outer surface 3724 of the first end 3712 of the safety lock 3710 includes a series of ridges 3726 to allow the user to more easily grip the safety lock 3710. The outer surface 3724 of the first end 3712 of the safety lock 3710 also includes an indicia 3728 to instruct the user in operating the auto-injector 3002. As shown in FIG. 25, the indicia 3728 includes a numeral to indicate the order of operation and an arrow to indicate the direction in which the safety lock 3710 should be moved. In some embodiments, the indicia 3728 can include different colors, detailed instructions or any other suitable indicia to instruct the user. In other embodiments, the indicia 3728 can protrude from the safety lock 3710 to aid the user when grasping the safety lock 3710.

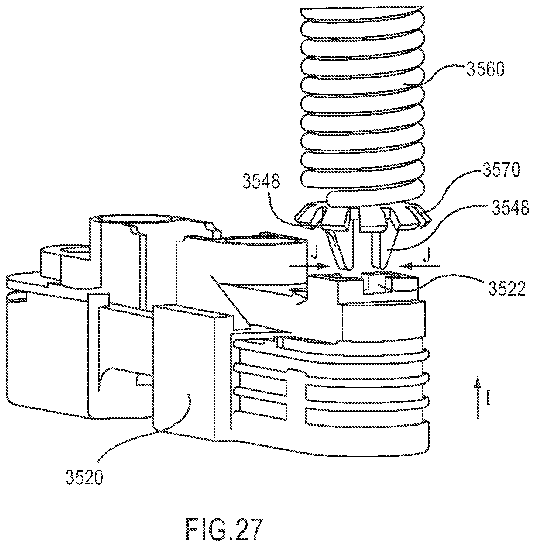

After being enabled, the auto-injector 3002 can then be actuated by moving the base 3520 proximally towards the housing 3110, as indicated by arrow I in FIG. 27. As shown in FIGS. 28 and 36, the base 3520 defines two openings 3536 that receive corresponding attachment protrusions 3150 disposed on the distal end portion 3114 of the housing 3110. In this manner, the movement and/or alignment of the base 3520 relative to the housing 3110 is guided by the attachment protrusions 3150 and the openings 3536 (see FIG. 36).

Each attachment protrusion 3150 is secured within its corresponding opening 3536 by a lock washer 3534. The lock washers 3534 each define an opening 3535 that receives a portion of the attachment protrusion 3150. The lock washers 3534 are disposed within slots 3533 defined by the base 3520 so that the openings 3535 are aligned with the attachment protrusions 3150. The openings 3535 are configured to allow the lock washers 3534 to move proximally relative to the attachment protrusions 3150, but to prevent movement of the lock washers 3534 distally relative to the attachment protrusions 3150. In this manner, when the attachment protrusions 3150 are disposed within the openings 3535 of the lock washers 3534, the base 3520 becomes fixedly coupled to the housing 3110. Moreover, after the base 3520 is moved proximally relative to the housing 3110, the lock washers 3534 prevent the base 3520 from returning to its initial position. Said another way, the arrangement of the lock washers 3534 prevents the base 3520 from being "kicked back" after the auto-injector 3002 has been actuated.

The base 3520 also defines a needle opening 3532, a recess 3526 and two retraction spring pockets 3531. The needle opening 3532 receives a portion of the needle guard 3810 when the needle guard is in its first position. Additionally, when the auto-injector is in its third configuration (see FIG. 37), the needle 3212 extends through the needle opening 3532. As described above, the recess 3526 receives the corresponding protrusion 3856 on the sheath retainer 3840 to removably couple the needle guard 3810 to the base 3520. As will be described in more detail herein, the retraction spring pockets 3531 receive a portion of the retraction springs 3350.

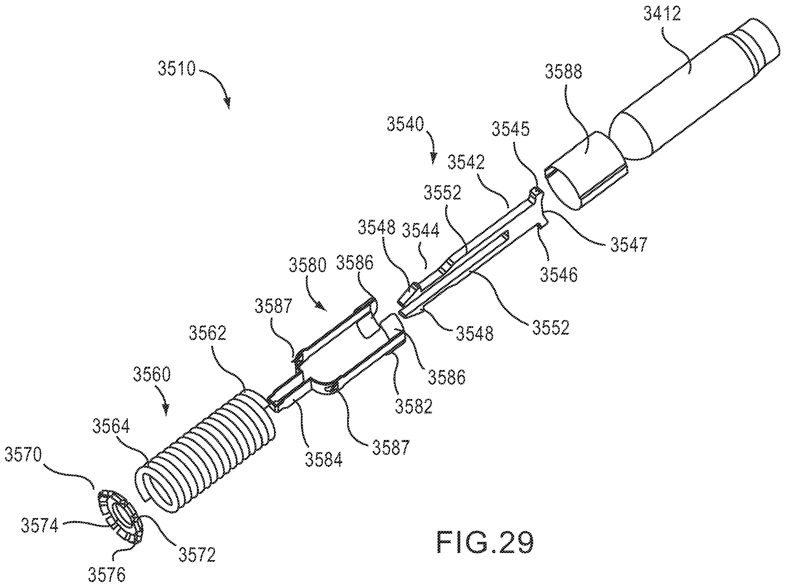

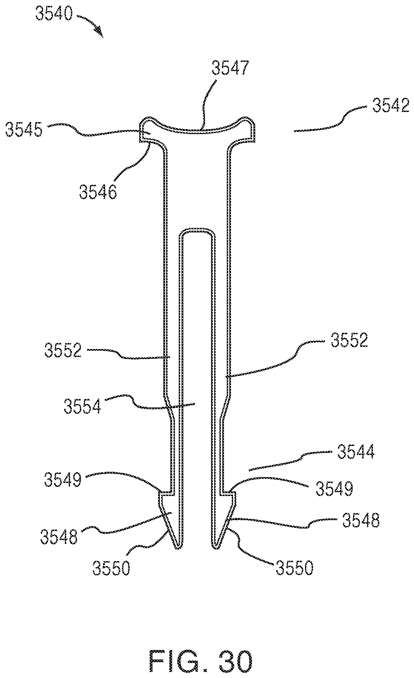

As shown in FIG. 28, the base 3520 includes two opposing tapered surfaces 3524 that define an opening 3522 configured to receive a corresponding tapered surface 3550 of the projections 3548 when the base is moved proximally towards the housing 3110. The opening 3522 can extend through the base 3520 or through at least a portion of the base. When the projections 3548 are received within the tapered opening 3522, they are moved together as indicated by arrows J in FIG. 27. The inward movement of the projections 3548 causes the rod 3540 to become disengaged from the spring retainer 3570, thereby allowing the rod 3540 to be moved proximally along its longitudinal axis as the spring 3560 expands. A more detailed description of the components included in the system actuator 3510 is provided below with reference to FIGS. 29 and 30.

The system actuator 3510 includes a rod 3540, a spring 3560 disposed about the rod 3540 and a spring retainer 3570. As described in more detail herein, the spring retainer 3570 retains both the spring 3560 and the rod 3540. The spring retainer 3570 includes a first surface 3572, a second surface 3574 and a series of outwardly extending engagement tabs 3576. The spring retainer 3570 is disposed within the gas container opening 3124 defined by the housing 3110 (see FIG. 36) such that the engagement tabs 3576 engage the interior surface 3123 of the housing 3110 to produce an interference fit. In this manner, the spring retainer 3570 is fixedly disposed within the housing 3110.

The rod 3540 has a proximal end portion 3542 and a distal end portion 3544. The distal end portion 3544 of the rod 3540 includes two extensions 3552 disposed apart from each other to define an opening 3554 therebetween. Each extension 3552 includes a projection 3548 having a tapered surface 3550 and an engagement surface 3549. When the rod 3540 is in its first (or engaged) position, the engagement surfaces 3549 engage the second surface 3574 of the spring retainer 3570 to prevent the rod 3540 from moving proximally along its longitudinal axis. As described above, when the base 3520 is moved proximally towards the housing 3110, the tapered surfaces 3550 of the projections 3548 cooperate with the corresponding tapered surfaces 3524 of the base 3520 to move the extensions 3552 inwardly towards each other. The inward motion of the extensions 3552 causes the engagement surfaces 3549 to become disengaged from the second surface 3574 of the spring retainer 3570, thereby allowing the rod 3540 to move between its first position to a second (or actuated) position.

The proximal end portion 3542 of the rod 3540 includes a retention portion 3545 having a first surface 3547 and a second surface 3546. The first surface 3547 of the retention portion 3545 engages the distal portion 3416 of the compressed gas container 3412. The second surface 3546 of the retention portion 3545 engages a proximal end 3562 of the spring 3560. Similarly, the first surface 3572 of the spring retainer 3570 engages a distal end 3564 of the spring 3560. In this manner, when the rod 3540 is in its first position, the spring 3560 can be compressed between the spring retainer 3570 and the retention portion 3545 of the rod 3540. Accordingly, when the rod 3540 is disengaged from the spring retainer 3570, the force imparted by the spring 3560 on the retention portion 3545 of the rod 3540 causes the rod 3540 to move proximally into its second position.

The proximal end portion 3542 of the rod 3540 is coupled to the compressed gas container 3412 by a connector 3580, which is secured to the distal end portion 3416 of the compressed gas container 3412 by a securing member 3588. The connector 3580 includes a proximal end portion 3582 and a distal end portion 3584. The distal end portion 3584 of the connector 3580 is disposed within the opening 3554 defined between the extensions 3552. In this manner, the connector 3580 is retained by the proximal end portion 3542 of the rod 3540. As will be described in more detail, the distal end portion 3584 of the connector 3580 includes locking tabs 3587.

The proximal end portion 3582 of the connector 3580 includes engagement portions 3586 that engage the distal end portion 3416 of the compressed gas container 3412. The engagement portions 3586 are coupled to the compressed gas container 3412 by the securing member 3588, which can be, for example, a shrink wrap, an elastic band or the like. In other embodiments, the engagement portions 3586 can produce an interference fit with the compressed gas container 3412, thereby eliminating the need for a securing member 3588.

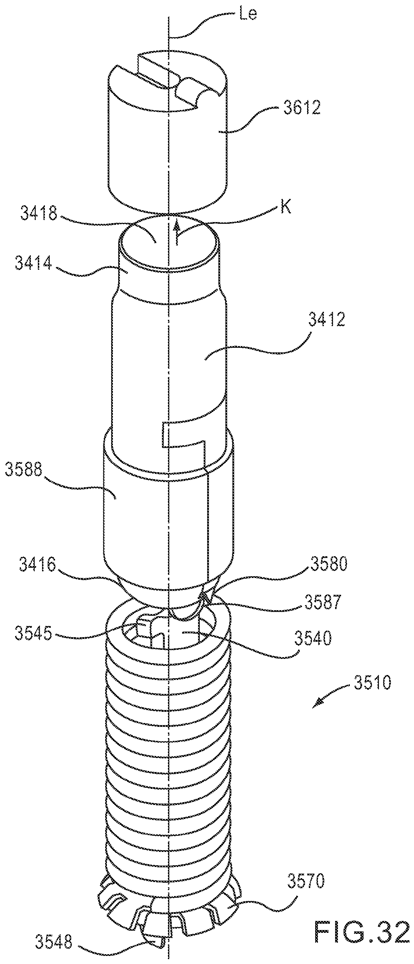

Because the rod 3540 is coupled to the compressed gas container 3412, when the rod 3540 is moved from its first (engaged) position to its second (actuated) position, the compressed gas container 3412 is moved proximally within the housing 3110 into engagement with the gas release mechanism 3612. FIG. 31 shows the auto-injector in a second configuration, in which the compressed gas container 3412 is engaged with the gas release mechanism 3612. When in the second configuration, the compressed gas contained within the compressed gas container 3412 is released to actuate the medicament injector 3210. A more detailed description of the gas release process is provided below with reference to FIGS. 32 through 36.

FIG. 32 shows an exploded view of the system actuator 3510, the compressed gas container 3412 and the gas release mechanism 3612, each of which are disposed within the gas container opening 3124 defined by the housing 3110 (see FIG. 36). As shown, the compressed gas container 3412, the system actuator 3510 and the gas release mechanism 3612 are arranged substantially coaxial with each other. As previously discussed, when the auto-injector 3002 is actuated, the compressed gas container 3412 is moved proximally within the gas container opening 3124 defined by the housing 3110, as indicated by the arrow K in FIG. 32, until the proximal end 3414 of the compressed gas container 3412 engages the gas release mechanism 3612.

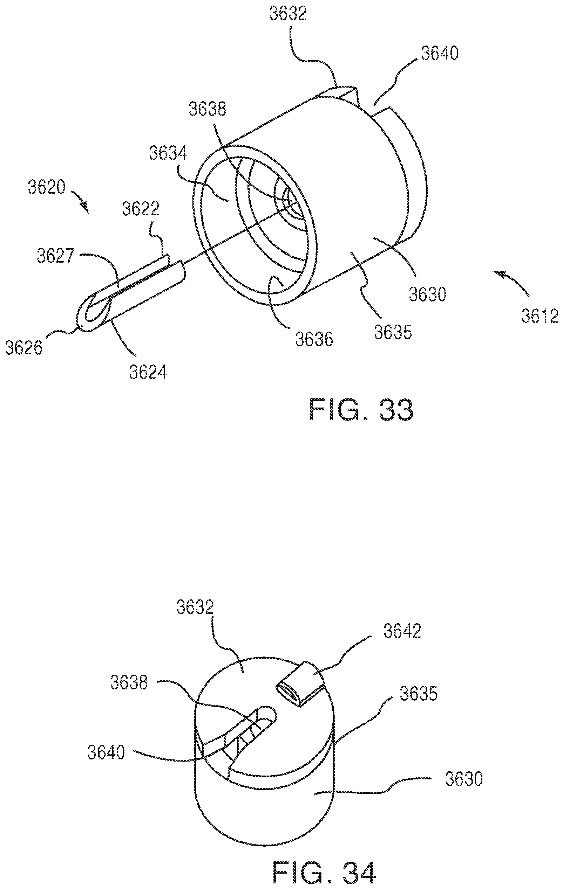

As shown in FIGS. 33 and 34, the gas release mechanism 3612 includes a cap 3630 and a puncturing element 3620 coupled to and disposed within the cap 3630. The puncturing element has a proximal end 3622 and a distal end 3624. The distal end 3624 of the puncturing element 3620 defines a sharp point 3626 configured to puncture the proximal end 3414 of the compressed gas container 3412. The puncturing element 3620 defines an opening 3627 extending from its distal end 3624 to its proximal end 3622.

The cap 3630 has a proximal end 3632, an outer surface 3635 and an inner surface 3636. The inner surface 3636 of the cap 3630 defines an opening 3634 that receives the proximal end 3414 of the compressed gas container 3412 when the auto-injector 3002 is in its second configuration. The proximal end 3632 of the cap 3630 defines an opening 3638 therethrough and a channel 3640 in fluid communication with the opening 3638. The opening 3638 receives the proximal end 3622 of the puncturing element 3620 to couple the puncturing element 3620 to the cap 3630. The puncturing element 3620 is disposed within the cap 3630 such that when the compressed gas container 3412 is moved into the opening 3634, the distal end 3624 of the puncturing element 3620 punctures the proximal end 3414 of the compressed gas container 3412.

The cap 3630 is disposed within the gas container opening 3124 such that the outer surface 3635 of the cap 3630 engages the inner surface 3123 of the housing 3110. In some embodiments, the outer surface 3635 of the cap 3630 can be sized to produce an interference fit with the inner surface 3123 of the housing 3110. In other embodiments, the cap 3630 can be fixedly coupled within the gas container opening 3124 using an adhesive or any other suitable attachment mechanism.

The cap 3630 is oriented within the gas container opening 3124 so that the channel 3640 is aligned with and in fluid communication with the gas passageway 3126 defined by the housing 3110. Moreover, when oriented in this manner, the protrusion 3642 on the proximal end 3632 of the cap 3630 obstructs a portion of the gas passageway 3126, which can be manufactured as a through-hole, to fluidically isolate the gas passageway 3126 from an area outside of the housing 3110. After the proximal end 3414 of the compressed gas container 3412 has been punctured, pressurized gas flows from the compressed gas container 3412 into the gas passageway 3126 through the opening 3627 defined by the puncturing element 3620 and the channel 3640 defined by the proximal end 3632 of the cap 3630.

The inner surface 3636 of the cap 3630 is configured to hermetically seal the proximal end 3414 of the compressed gas container 3412 within the opening 3638. This arrangement prevents pressurized gas from leaking around the compressed gas container 3412 to an area outside of the housing 3110 after the proximal end 3414 of the compressed gas container 3412 has been punctured. In some embodiments, the inner surface 3636 is sized to produce an interference fit with the compressed gas container 3412. In other embodiments, the cap 3630 includes a separate sealing member, such as, for example, an o-ring, to seal the proximal end 3414 of the compressed gas container 3412 within the opening 3638.

After the compressed gas container 3412 is moved into engagement with the gas release mechanism 3612, the position of the compressed gas container 3412 within the gas container opening 3124 is maintained by the locking tabs 3587 on the connector 3580. As shown in FIG. 29, each locking tab 3587 includes a pointed portion that is angled outwardly from the connector 3580. This arrangement allows the connector 3580 to move proximally within the gas container opening 3124 of the housing 3110, but prevents the connector 3580 from moving distally within the gas container opening 3124 of the housing 3110. Said another way, the arrangement of the locking tabs 3587 prevents the compressed gas container 3412 from being "kicked back" when exposed to the force produced by the pressurized gas as the pressurized gas is released.

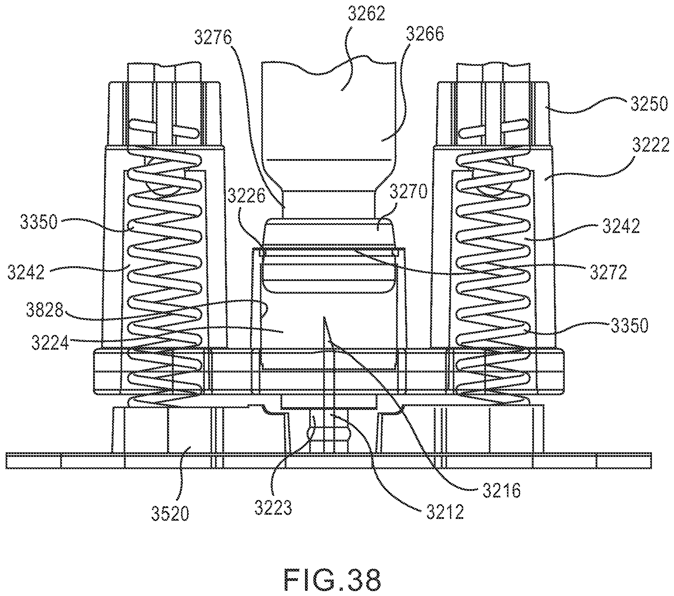

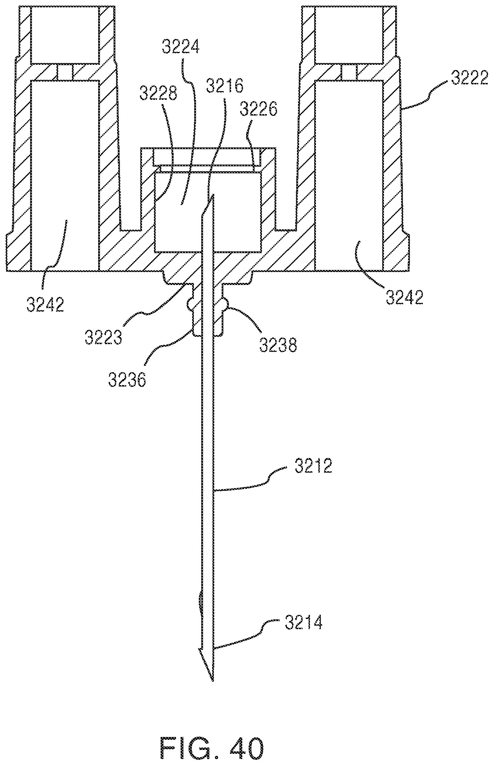

As previously discussed, the pressurized gas released from the compressed gas container 3412 produces a force on the boundary of the gas chamber 3120, including the surface 3322 of the movable member 3312. This force causes the movable member 3312 and the medicament injector 3210 move together distally within the housing 3110, as shown by arrow L, placing the auto-injector 3002 in a third configuration, as shown in FIG. 37. When in the third configuration, the distal end 3214 of the needle 3212 is disposed through the opening 3532 defined by the base 3520 to an area outside of the auto-injector 3002. Moreover, as shown in FIG. 38, when the auto-injector 3002 is in the third configuration, the proximal end 3216 of the needle 3212 remains spaced apart from the distal end 3266 of the medicament container 3210, ensuring that the needle 3212 remains fluidically isolated from the medicament container 3210. In this manner, the needle 3212 can be inserted into a patient as the auto-injector 3002 moves between its second configuration (FIG. 31) and its third configuration (FIG. 37) without injecting the medicament until after insertion is completed. A more detailed description of the medicament injector 3210 and the movable member 3312 is provided below with reference to FIGS. 37 through 42.

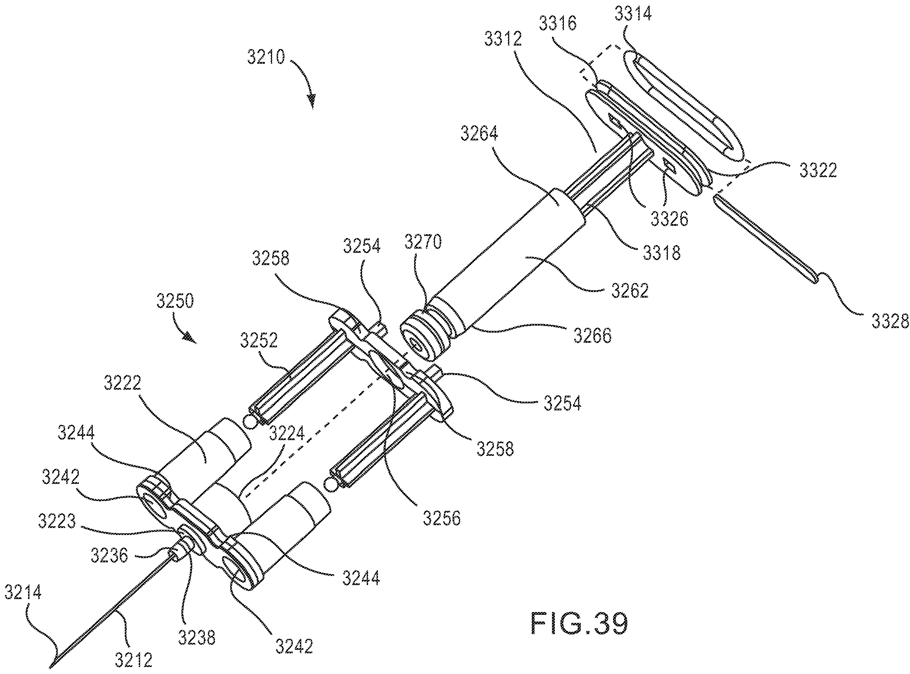

As previously described, the medicament injector 3210 includes a carrier 3250, a medicament container 3262 and a needle 3212. The carrier 3250 has a lower portion 3222 and an upper portion 3252. The lower portion 3222 of the carrier 3250 includes a needle hub 3223, which contains the needle 3212. The lower portion 3222 of the carrier 3250 also defines an opening 3224 configured to receive a distal portion 3266 the medicament container 3262. As shown in FIG. 39, the needle 3212 is coupled to the needle hub 3223 such that the proximal end 3216 of the needle 3212 is disposed within the opening 3224 and the distal end 3214 of the needle 3212 extends distally outside of the needle hub 3223.

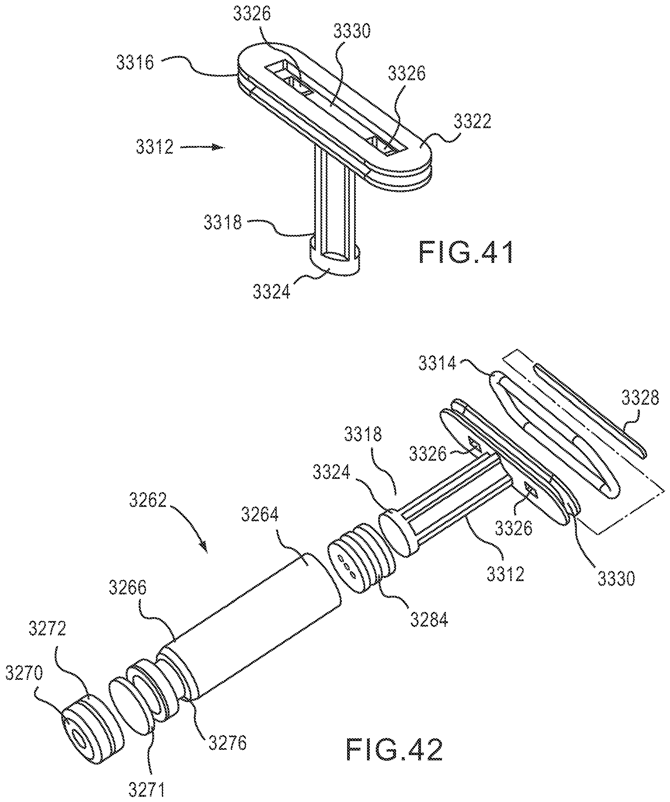

The inner surface 3228 of the lower portion 3222 defining the opening 3224 includes a protrusion 3226. The protrusion 3226 is configured to engage a corresponding recess 3272 defined by a sealing cap 3270 disposed at the distal portion 3266 of the medicament container 3262 (see FIG. 42) to secure the medicament container 3262 within the opening 3224 such that the proximal end 3216 of the needle 3212 is spaced apart from the distal end 3266 of the medicament container 3210. The protrusion 3226 and the recess 3272 are configured such that the protrusion 3226 will become disengaged from the recess 3272 when the force applied exceeds a predetermined value. Said another way, the protrusion 3226 and the recess 3272 collectively form a removable snap-fit that allows the medicament container 3262 to be moved within the opening 3224 when the force applied to the medicament container 3262 exceeds a predetermined value. This arrangement ensures that the needle 3212 remains fluidically isolated from the medicament container 3262 during the insertion operation.

The outer surface 3236 of the lower portion 3222 includes a protrusion 3238. As previously described, the protrusion 3238 is configured to engage a corresponding recess portion 3828 within the opening 3826 of the sheath 3820 (see FIG. 23) to removably couple the sheath 3820 to the needle hub 3223.

The lower portion 3222 of the carrier 3250 also defines two retraction spring pockets 3242 each receiving the proximal end 3352 of a retraction spring 3350. As previously discussed, the distal end 3354 of each retraction spring 3350 is retained within the retraction spring pockets 3531 defined by the base 3520. As shown in FIG. 38, when the carrier 3250 moves distally within the housing 3110, the retraction springs 3350 are compressed and therefore bias the carrier 3250 towards the proximal portion 3112 of the housing 3110.

The upper portion 3252 of the carrier 3250 defines an opening 3256 configured to receive a proximal portion 3264 of the medicament container 3262 and includes two valve actuators 3254. As described in more detail herein, the valve actuators 3254 are configured to engage a gas relief valve 3328 to allow the pressurized gas contained within the gas chamber 3120 to escape when the injection event is complete.

The upper portion 3252 of the carrier 3250 defines four gas relief passageways 3258. Similarly, the lower portion 3222 of the carrier 3250 defines four gas relief passageways 3244. When the pressurized gas is released from the gas chamber 3120, the gas relief passageways 3258, 3244 provide a fluid path to allow the pressurized gas to flow from the gas chamber 3120 to an area outside of the housing 3110.

As described above, the movable member 3312 includes a proximal end portion 3316 and a distal end portion 3318. The distal end portion 3318 includes a piston 3324 disposed within the proximal portion 3264 of the medicament container 3262, such that the piston engages a plunger 3284 contained within the medicament container 3262, as shown in FIG. 42.

The proximal end portion 3316 includes a surface 3322 that defines a portion of a boundary of the gas chamber 3120. As shown in FIG. 41, the proximal end portion 3316 defines two openings 3326 therethrough, each of which are in fluid communication between the gas chamber 3120 and the interior of the housing 3110 outside the gas chamber 3120. The proximal end portion 3316 further defines a slot 3330 that receives a gas relief valve 3328, which can be, for example, a flexible rubber member. The gas relief valve 3328 is positioned within the slot 3330 and adjacent the openings 3326 to selectively allow fluid communication between the gas chamber 3120 and the area outside the gas chamber 3120 through the openings 3326. The operation of the gas relief valve 3328 is discussed in more detail herein.

The proximal end portion 3316 of the movable member 3312 also includes a seal 3314 that engages a portion the inner surface 3122 of the housing 3110 (see FIG. 36) to fluidically isolate the gas chamber 3120. Although the seal 3314 is shown as being an o-ring seal, in some embodiments, the seal need not be a separate component, but can rather be a portion of the proximal end portion 3316 of the movable member 3312.

When the needle insertion operation is completed, the lower portion 3222 of the carrier 3250 engages the base 3520, preventing further distal movement of the carrier 3250 within the housing. Because the distal motion of the carrier 3250 is opposed, the force exerted by the pressurized gas on the surface 3322 of the movable member 3312 increases until the protrusion 3226 of the lower portion 3222 of the carrier 3250 and the recess 3272 defined by sealing cap 3270 of the medicament container 3262 become disengaged. Accordingly, the medicament container 3262 to moves distally relative to the carrier 3250, placing the auto-injector 3002 in a fourth configuration, as shown in FIG. 43. When moving between the third configuration (FIG. 38) and the fourth configuration (FIG. 43), the proximal end 3216 of the needle 3212 pierces the sealing cap 3270 and the liner 3271 disposed at the distal portion 3266 of the medicament container 3262. As such, when in the fourth configuration, the proximal end 3216 of the needle 3212 is in fluid communication with the medicament container 3262, thereby allowing the medicament to be injected.

Once the needle 3212 is in fluid communication with the medicament container 3262, the force from the pressurized gas causes the piston 3324 of the movable member 3312 to move the plunger 3284 within the medicament container 3262, as shown by arrow M, thereby expelling the medicament through the needle 3212. The piston 3324 and the plunger 3284 move a predetermined distance within the medicament container 3262, placing the auto-injector 3002 in a fifth configuration, as shown in FIG. 44. When the auto-injector 3002 is in the fifth configuration, the injection of medicament is complete.

When the auto-injector 3002 is in its fifth configuration, proximal portion 3316 of the movable member 3312 is in contact with the upper portion 3252 of the carrier 3250, thereby preventing further movement of the piston 3324 within the medicament container 3262. In this manner, the distance through which the piston 3324 travels, and therefore the amount of medicament injected, can be controlled.

Additionally, when the auto-injector 3002 is in its fifth configuration, the valve actuators 3254 are disposed within the openings 3326 such that the valve actuators 3254 displace the gas relief valve 3328. Accordingly, the pressurized gas contained within the gas chamber 3120 can flow from the gas chamber 3120 to the area within the housing 3310 outside of the gas chamber 3310. As previously discussed, the gas relief passageways 3258, 3244 provide a fluid path to allow the pressurized gas to flow from the gas chamber 3120, through the opening 3532 defined by the base 3520 and to an area outside of the housing 3110.

When the pressurized gas flows out of the gas chamber 3120, the pressure exerted on the surface 3322 of the movable member 3312 decreases. Accordingly, the force exerted by the retraction springs 3350 is sufficient to move the medicament injector 3210 and the movable member 3312 proximally within the housing 3110, as shown by arrow N, into a sixth (or retracted) configuration as shown in FIG. 45. Because the medicament injector 3210 and the movable member 3312 move together, the valve actuators 3254 remain disposed within the openings 3326 as the auto-injector 3002 moves into the sixth configuration. In this manner, the gas relief valve 3328 remains displaced and the openings 3326 remain in fluid communication with the gas chamber 3120 and the area within the housing 3310 outside of the gas chamber 3310 independent of the position of the movable member 3312. Such an arrangement ensures that all of the pressurized gas flows out of the gas chamber 3120, thereby ensuring that the medicament injector 3210 and the movable member 3312 return to the sixth configuration and do not oscillate between the sixth configuration and the fifth configuration, which could lead to the needle 3212 not being fully retracted into the housing 3110.

Although the auto-injector 3002 has been shown and described having a housing 3110 having a substantially rectangular shape, in some embodiments, an auto-injector can have a housing having any shape. In some embodiments, for example, an auto-injector can have a substantially cylindrical shape. In other embodiments, for example, the auto-injector can have an irregular and/or asymmetrical shape.

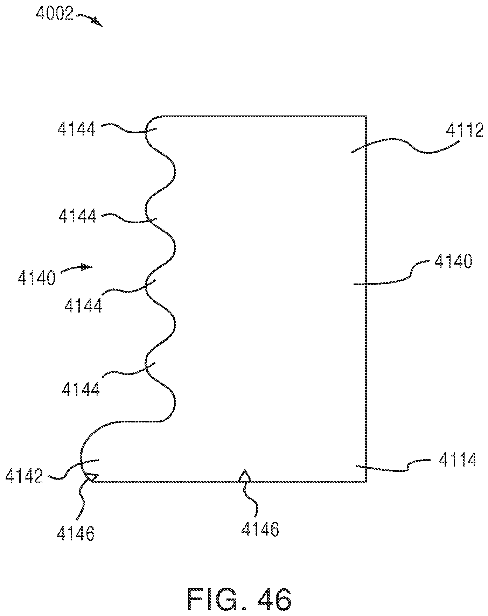

Although the auto-injector 3002 has been shown and described as including a protrusion 3142 disposed at the distal end portion 3114 of the housing 3110 to help a user grasp and retain the housing 3110, in some embodiments, a protrusion can be disposed anywhere along the housing. In other embodiments, a protrusion can symmetrically surround the distal portion of the housing. In yet other embodiments, the housing of an auto-injector can include a gripping portion configured to help a user grasp and retain the housing. The gripping portion can include, for example, a textured surface, a contoured surface, a surface having an adhesive that forms a tacky surface to adhere to the user's hand or the like. For example, FIG. 46 shows an auto-injector 4002 according to an embodiment of the invention having a housing 4110. The housing 4110 includes a proximal end portion 4112, a distal end portion 4114 and a gripping portion 4140. The distal end portion 4114 of the housing 4110 includes a protrusion 4142 to prevent the user's hand from slipping off of the distal end portion 4114 of the housing 4110 when using the auto-injector 4002. Similarly, the gripping portion 4140 includes a series of contours 4144 that engage the user's fingers to help the user grasp and retain the housing 4110 when the auto-injector 4002 is in use.

The distal end portion 4114 of the housing 4110 also includes two alignment marks 4146 to guide the user when placing the auto-injector 4002 against the body. Although the alignment marks 4146 are shown as markings on the housing 4110, in other embodiments, the alignment marks can include protrusions, openings or the like.

Certain components of the auto-injector 3002 are shown and described as being coupled together via protrusions and mating recesses. The protrusions and/or recesses can be disposed on any of the components to be coupled together and need not be limited to only a certain component. For example, the base 3520 is shown as defining two openings 3536 that receive corresponding attachment protrusions 3150 on the distal end portion 3114 of the housing 3110. In some embodiments, however, the protrusions can be disposed on the base and the mating recesses can be defined by the distal end portion of the housing. In other embodiments, two or more components can be coupled together in any suitable way, which need not include protrusions and mating recesses. For example, in some embodiments, two or more components can be coupled together via mating shoulders, clips, adhesive and the like.

Similarly, although certain components of the auto-injector 3002 are shown and described as being constructed from multiple separate components, in some embodiments, such components can be monolithically constructed. For example, the carrier 3250 is shown and described as including an upper portion 3252 and a lower portion 3222 that are constructed separately and then coupled together. In other embodiments, a carrier can be constructed monolithically.

Although the sheath retainer 3840 of the auto-injector 3002 has been shown and described as including a protrusion 3856 that engages a corresponding recess 3526 in the base 3520 to removably couple the sheath retainer 3840 to the base 3520, in some embodiments, the sheath retainer can include a protrusion configured to engage a different corresponding recess on the auto-injector 3002. For example, the sheath retainer can include a protrusion configured to engage a corresponding recess in the distal end portion 3114 of the housing 3110.

Although the safety lock (or locking member) 3710 of the auto-injector 3002 has been shown and described as including a protrusion 3718 configured to engage a base 3520 movably coupled to the housing 3110, in some embodiments, the safety lock can include a protrusion configured to engage a different portion of the auto-injector 3002. For example, the safety lock can include a protrusion configured to engage a portion of the housing 3110, such as the distal end portion 3114 of the housing 3110, to removably couple the safety lock in its first position.

Although the base 3520 of the auto-injector 3002 has been shown and described covering almost the entire distal end portion 3114 of the housing 3110, in some embodiments, a base configured to actuate the auto-injector can be disposed about only a portion of the distal end of the housing. For example, in some embodiments, an auto-injector can include a button extending from the distal end portion of the housing configured to engage and release the system actuator.

Although the rod 3540 is shown and described as being an elongated member that is released by being elastically deformed, in some embodiments, a rod can be of any suitable shape and in any suitable orientation within the housing. Moreover, in some embodiments, a rod can be released by being plastically deformed. For example, in some embodiments, a rod can be disposed along an axis that is offset from the longitudinal axis of the energy storage member. In some embodiments, the rod can be configured to break upon actuation.

Although the gas release mechanism 3612 is shown and described as including a puncturing element 3620 to puncture a portion of the compressed gas container 3262, the gas release mechanism 3612 need not include a puncturing element 3620. For example, in some embodiments, the gas release mechanism can include an actuator configured to actuate a valve that controls the flow of gas out of the compressed gas container. For example, in some embodiments, a compressed gas container can include a spring loaded check ball and the gas release mechanism can include an actuator configured to engage and depress the check ball to release pressurized gas from the compressed gas container.

Although the distance through which the piston 3324 travels, and therefore the amount of medicament injected, is shown and described as being controlled by configuring the movable member 3312 such that it is in contact with the upper portion 3252 of the carrier 3250 when the auto-injector 3002 is in its fifth configuration, in other embodiments, any suitable method of controlling the piston travel can be employed. For example, in some embodiments, piston travel can be limited by including a protrusion within the medicament container, such as a necked portion, that limits the motion of the piston within the medicament container. In other embodiments, the housing can include a protrusion to limit the motion of the movable member. In yet other embodiments, the valve actuator can be configured to actuate the gas relief valve when the piston has moved a predetermined distance within the medicament container. In yet other embodiments, a combination of each of the above methods for controlling the piston travel can be employed.

Although the auto-injector 3002 is shown and described as having six different configurations that are different from each other, in some embodiments, a certain configuration of an auto-injector can be the same as another configuration. For example, in some embodiments, a "pre-actuation" configuration can be the same as a "retracted" configuration. In other embodiments, any of the functions described above can be accomplished when an auto-injector is moved between any number of different configurations.

Although the auto-injector 3002 is shown and described as including a compressed gas cylinder 3412, in other embodiments an auto-injector can include any suitable energy storage member. For example, in some embodiments, an auto-injector can include a mechanical energy storage member, such as a spring, an electrical energy storage member, such as a battery or a capacitor, a chemical energy storage member, such as a container containing two substances that can react to produce energy, a magnetic energy storage member or the like. Similarly, although the auto-injector 3002 is shown and described as including a gas release mechanism 3612, in other embodiments an auto-injector can include any suitable energy release mechanism. Such energy release mechanism can include, for example, an electrical circuit, a mechanical spring retainer, a fluid control valve or the like.

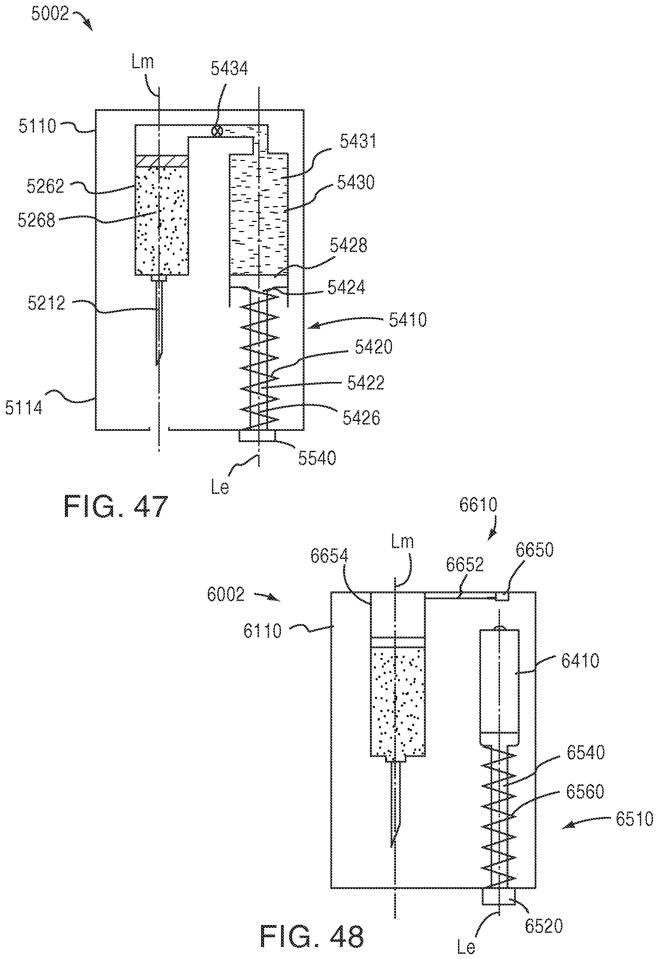

For example, FIG. 47 shows a schematic illustration of an auto-injector 5002 that includes a mechanical energy storage member 5410. The auto-injector 5002 includes a housing 5110 that contains a medicament container 5262, an energy storage member 5410, a release member 5540. The medicament container 5262 is movably disposed within the housing 5110 and includes a needle 5212 through which a medicament 5268 can be injected. As illustrated, the medicament container 5262 can be moved along its longitudinal axis Lm between a first position (FIG. 47) and a second position (not shown), in which the needle 5212 extends from the housing 5110.

The energy storage member 5410 includes a spring 5420 that is disposed about a rod 5422. The rod 5422 has a proximal end 5424 and a distal end 5426. The proximal end 5424 of the rod 5422 includes a plunger 5428 that retains the spring 5420 such that the spring 5420 can be compressed when the auto-injector 5002 is in a first configuration. The plunger is also disposed within a working fluid chamber 5430. The working fluid chamber 5430 can be, for example, a hydraulic cylinder filled with a hydraulic fluid. The distal end 5426 of the rod 5422 engages the release member 5540, as discussed below.

In use, the spring 5420 can be moved within the housing 5110 along its longitudinal axis Le between a first position and a second position. When the spring 5420 moves between its first position to its second position, the plunger 5428 moves proximally within the working fluid chamber 5430, causing the working fluid 5431 to be forced through a valve 5434 and into contact with the medicament container 5262. Through the kinetic energy produced by the spring 5420, the working fluid 5431 produces a force that acts upon the medicament container 5262 to move the medicament container 5262 between its first position and its second position.

The arrangement of a mechanical energy storage member, such as a spring, and a fluidic circuit allows the direction and/or magnitude of the force produced by the energy storage member to be changed. In this manner, as shown in FIG. 47, the longitudinal axis Le of the energy storage member can be offset from the longitudinal axis Lm of the medicament container 5262, thereby allowing the medicament container 5262 and the energy storage member 5410 to be arranged within the housing 5110 in any number of different configurations.

The release member 5540 is disposed adjacent a distal end portion 5114 of the housing 5110 and is configured to selectively deploy the spring 5420 from its first position to its second position. The release member 5540 can be any suitable mechanism of the types described above for moving the spring 5420. In this manner, a user can actuate the auto-inj ector by manipulating the distal end portion 5114 of the housing 5110.

FIG. 48 shows a schematic illustration of an auto-injector 6002 that includes an electrical energy storage member 6410, such as, for example a battery. The auto-injector 6002 includes a housing 6110 that contains a medicament container 6262, an energy storage member 6410, a system actuator 6510 and an energy release mechanism 6610. The medicament container 6262 is movably disposed within the housing 6110 and includes a needle 6212 through which a medicament 6268 can be injected. As illustrated, the medicament container 6262 can be moved along its longitudinal axis Lm between a first position (FIG. 48) and a second position (not shown), in which the needle 6212 extends from the housing 6110.

The energy storage member 6410 is also movably disposed within the housing 6110 along its longitudinal axis Le, which is offset from the longitudinal axis Lm of the medicament container 6262. When the energy storage member 6410 is in its first position (FIG. 48), it is spaced apart from the electrical contact 6650 of the energy release mechanism 6610. When the energy storage member 6410 is in its second position, it is in contact with the electrical contact 6650, thereby allowing current to flow from the energy storage member 6410 to an actuator 6654 via a circuit 6652. The actuator 6654 converts the electrical energy into a force that acts upon the medicament container 6262 to move the medicament container 6262 between its first position and its second position.

The system actuator 6510 includes a release member 6540 coupled to the energy storage member 6410, a spring 6560 and an actuator button 6520. The spring 6560 is disposed about the release member 6540 in a compressed configuration. The release member 6540 is removably coupled to the actuator button 6520, which is disposed at the distal end of the housing 6110. When the actuator button 6520 is manipulated, the release member 6540 is de-coupled from the actuator button 6520, thereby allowing the force from the spring 6560 to move the release member 6540. In this manner, the energy storage member 6410 is moved proximally within the housing 6110 into its second configuration. In some embodiments, the components included in the system actuator 6510 can be electrically coupled to the energy storage member 6410.

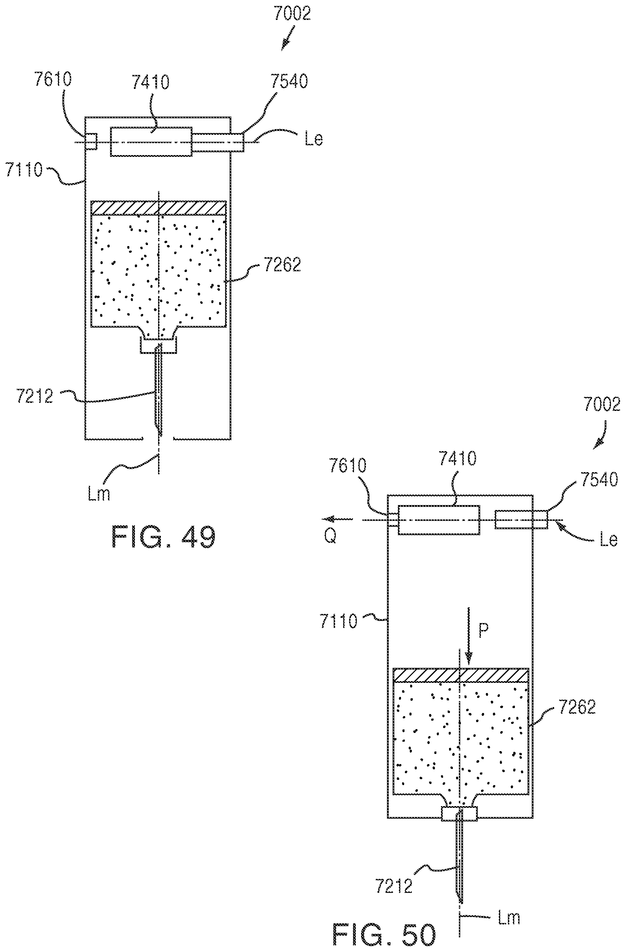

Although the auto-injectors shown and described include a medicament container and an energy storage member that are substantially parallel, in some embodiments, the medicament container and the energy storage member can be angularly offset from each other. For example, FIGS. 49 and 50 are schematic illustrations of an auto-injector 7002 in a first configuration and a second configuration, respectively. Similar to the auto-injectors described above, the auto-injector 7002 includes a housing 7110 that contains a medicament container 7262, an energy storage member 7410, a release member 7540 and an energy release mechanism 7610. The medicament container 7262, which includes a needle 7212, is disposed within the housing such that it can be moved along its longitudinal axis Lm as indicated by arrow P between a first position (FIG. 49) and a second position (FIG. 50).

The energy storage member 7410 is also movably disposed within the housing 7110 along its longitudinal axis Le, as shown by arrow Q. As shown, the longitudinal axis Le is substantially perpendicular to the longitudinal axis Lm of the medicament container 7262. When the energy storage member 7410 is in its first position (FIG. 49), it is spaced apart from the energy release mechanism 7610. When the energy storage member 7410 is in its second position (FIG. 50), it is in contact with the energy release mechanism 7610, thereby releasing energy to produce a force on the medicament container 7262 in a manner as described above.

As described above, the release member 7540 can be any suitable mechanism configured to selectively deploy the energy storage member 7410 from its first position to its second position.

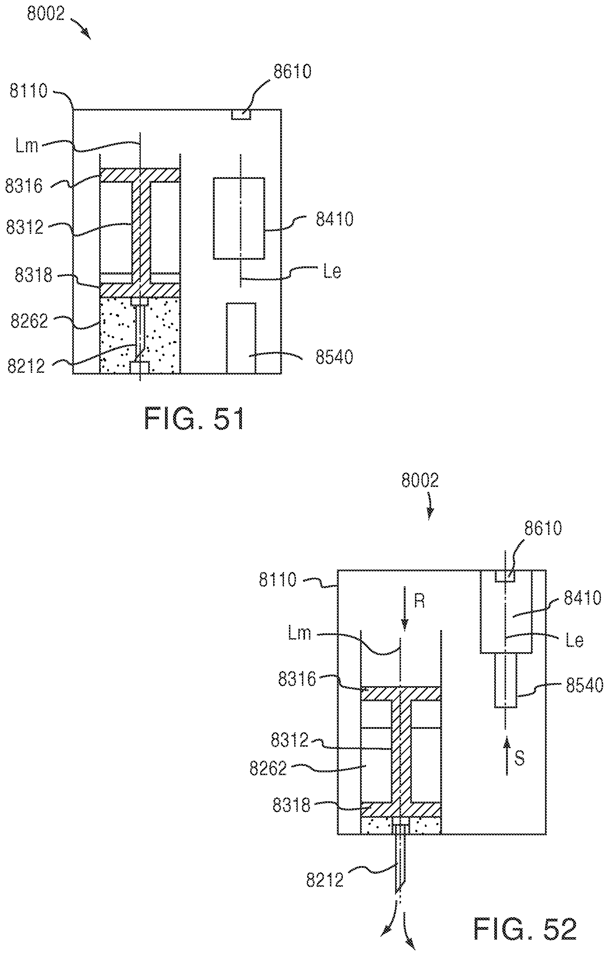

Although the auto-injectors shown and described above include a medicament container configured to move within the housing, in some embodiments, an auto-injector can be configured to move a needle within a stationary medicament container. For example, FIGS. 51 and 52 are schematic illustrations of an auto-injector 8002 in a first configuration and a second configuration, respectively. The auto-injector 8002 includes a housing 8110 that contains a medicament container 8262, a movable member 8312, an energy storage member 8410, an energy release mechanism 8610 and a release member 8540. The medicament container 8262 is fixedly disposed within the housing and defines a longitudinal axis Lm.

The movable member 8312 includes a proximal end 8316 and a distal end 8318. The distal end 8318 of the movable member 8312 is disposed within and movable within the medicament container 8262 along the longitudinal axis Lm, as shown by the arrow R. A needle 8212 is coupled to the distal end 8318 of the movable member 8312.

The energy storage member 8410 is also movably disposed within the housing 8110 along its longitudinal axis Le, as shown by arrow S. As shown, the longitudinal axis Le is offset from the longitudinal axis Lm of the medicament container 8262. When the energy storage member 8410 is in its first position (FIG. 51), it is spaced apart from the energy release mechanism 8610. When the energy storage member 8410 is in its second position (FIG. 52), it is in contact with the energy release mechanism 8610, thereby producing a force on the proximal end 8316 of the movable member 8312. The force causes the movable member 8312 to be moved within the medicament container 8262. In this manner, the needle 8212 is extended through the housing 8110 as the medicament is being injected.

As described above, the release member 8540 can be any suitable mechanism configured to selectively deploy the energy storage member 8410 from its first position to its second position.

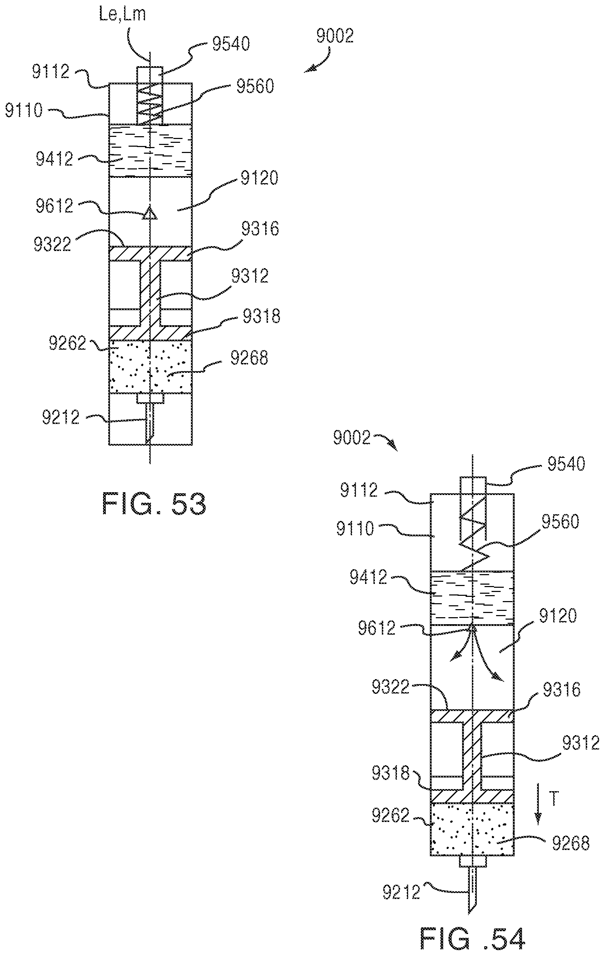

Although the auto-injector 3002 is shown and described as including a compressed gas container 3412 disposed non-coaxially with a medicament container 3262, in some embodiments, an auto-injector can include a compressed gas container that is coaxial with a medicament container. For example, FIGS. 53-55 are schematic illustrations of an auto-injector 9002 in a first configuration, a second configuration, and a third configuration, respectively. The auto-injector 9002 includes a housing 9110 that contains a medicament container 9262, a movable member 9312, a compressed gas container 9412 and a puncturer 9612. The medicament container 9262 is movably disposed within the housing 9110 and includes a needle 9212 through which a medicament 9268 can be injected. As illustrated, the medicament container 9262 can be moved along its longitudinal axis Lm between the first configuration (FIG. 53) and the second configuration (FIG. 54).

The compressed gas container 9412 is also movably disposed within the housing 9110 along its longitudinal axis Le, which is coaxial with the longitudinal axis Lm of the medicament container 9262. A biasing member 9560, such as, for example, a spring, is engaged with the compressed gas container 9412 to bias the compressed gas container 9412 distally towards the puncturer 9612. As shown in FIG. 53, when the auto-injector 9002 is in the first configuration, a retainer 9540 retains the compressed gas container 9412 in the proximal portion 9112 of the housing spaced apart from the puncturer 9612.

The movable member 9312 includes a proximal end portion 9316 and a distal end portion 9318. The proximal end portion 9316 includes a surface 9322 that, together with the housing 9110, defines a gas chamber 9120. The distal end portion 9318 is disposed within the medicament container 9262. The movable member 9312 is configured to move the medicament container 9262 within the housing 9110 and inject the medicament 9268.

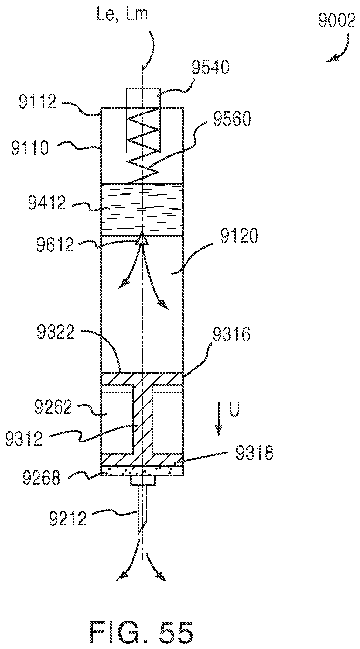

In use, the auto-injector 9002 is actuated by manipulating the proximal portion 9112 of the housing 9110 to move the retainer 9540, thereby allowing the compressed gas container 9412 to be moved distally until it engages the puncturer 9612, as shown in FIG. 54. As described above, the puncturer 9612 punctures a portion of the compressed gas container 9412 thereby releasing the pressurized gas contained therein into the gas chamber 9120. The pressurized gas produces a force on the movable member 9312, which causes the movable member 9312 and the medicament container 9262 to move distally into the second configuration, as shown by the arrow T in FIG. 54. When in the second configuration, the needle 9212 is extended outside of the housing 9110. The movable member 9312 then continues to move distally within the medicament container 9262, as shown by the arrow U in FIG. 55. In this manner, the medicament is injected through the needle 9212.

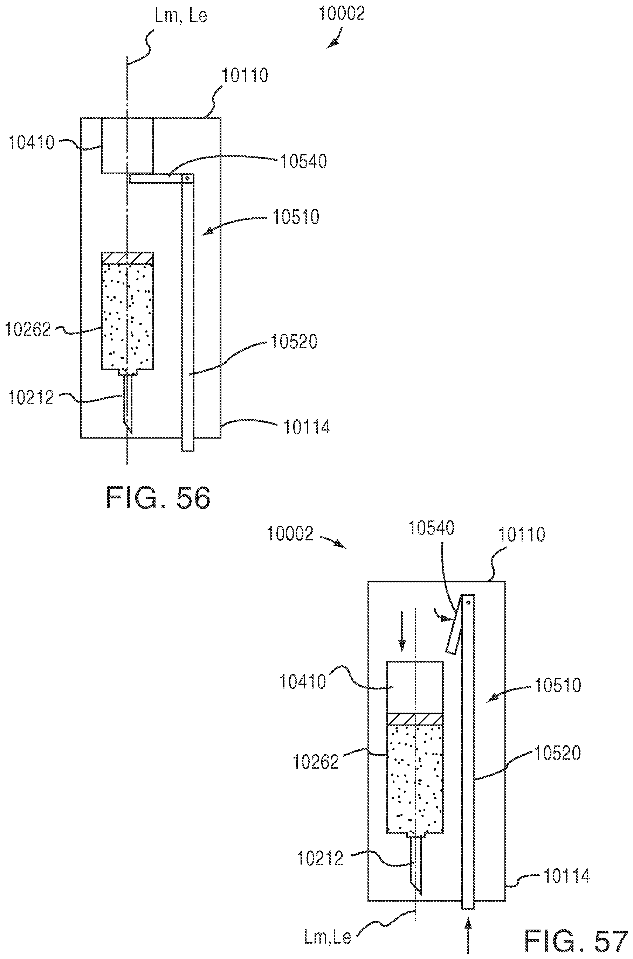

Although the auto-injectors are shown and described as being actuated from the distal end and including an energy storage member 3412 disposed non-coaxially with a medicament container 3262, in some embodiments, an auto-injector can be actuated from its distal end and include an energy storage member that is coaxial with a medicament container. For example, FIGS. 56 and 57 are schematic illustrations of an auto-injector 10002 in a first and a second configuration, respectively. The auto-injector 10002 includes a housing 10110 that contains a medicament container 10262, an energy storage member 10410 and a system actuator 10510.

The medicament container 10262 defines a longitudinal axis Lm that is coaxial with a longitudinal axis of the energy storage member 10410. The medicament container 10262 includes a needle 10212 through which a medicament can be injected. The medicament container 10262 is movable within the housing along its longitudinal axis Lm between a first position (FIGS. 56 and 57) and a second position (not shown), in which the needle 10212 extends outside of the housing 10110. As described above, the medicament container 10262 is moved by a force produced by the energy storage member 10410.

The energy storage member 10410 is also movably disposed within the housing 10110 along its longitudinal axis Le, as shown by arrow V in FIG. 57. When the energy storage member 10410 moves between a first position (FIG. 56) and a second position (FIG. 57), it produces a force on the medicament container 10262.

The system actuator 10510 includes a release member 10540 and an actuator button 10520. The release member 10540 is configured to selectively deploy the energy storage member 10410 from its first position to its second position. The release member 10540 can be, for example, a spring-loaded rod, a retainer or the like. The actuator button 10520 is coupled to the release member 10540 such that when the actuator button 10520 is manipulated, the release member 10540 can deploy the energy storage member 10410 from its first position to its second position. A portion of the actuator button 10520 extends outside of the distal end portion 10114 of the housing 10110 such that the user can actuate the auto-injector 10002 by manipulating the distal end portion 10114 of the housing 10110.

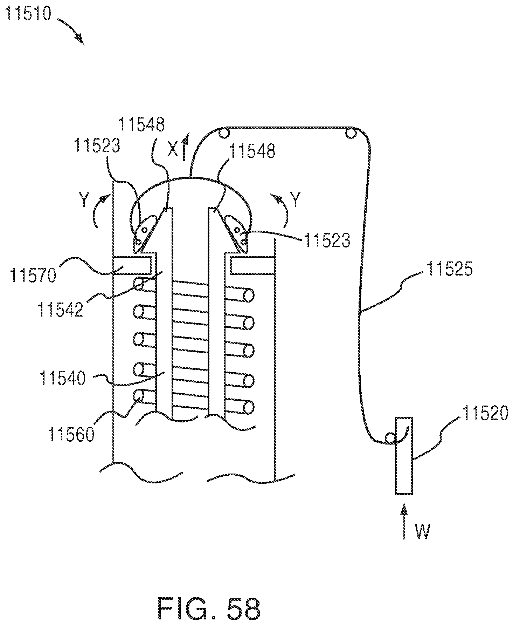

FIG. 58 shows a portion of a distally actuated system actuator 11510 according to an embodiment of the invention. Similar to the system actuators shown and described above, the system actuator 11510 is configured to selectively move an energy storage member (not shown) into contact with an energy release mechanism (not shown). The system actuator 11510 includes a rod 11540, a spring 11560 and a spring retainer 11570. A proximal portion 11542 of the rod 11540 is coupled to the spring retainer 11570 by two projections 11548, which can be moved inwardly towards each other to decouple the rod 11540 from the spring retainer 11570, as previously discussed.

The spring 11560 is disposed about the rod 11540 in a compressed state such that the spring 11560 is retained by a distal end portion (not shown) of the rod 11540 and the spring retainer 11570. In this manner, the rod 11540 is spring-loaded, similar to the rod 3540 discussed above.

The system actuator 11510 also includes an actuator button 11520 that is coupled via a flexible member 11525 to a pair of pivoting members 11523. A portion of the actuator button 11520 extends outside of the distal end portion of the housing (not shown). In use, the user can actuate the auto-injector by manipulating the distal end portion of the housing, for example, by pressing the actuator button 11520 inwardly as indicated by the arrow W. The inward movement of the actuator button 11520 causes the flexible member 11525, which can be, for example, a thin cable, to move as indicated by the arrow X. The movement of the flexible member 11525 causes the pivoting members 11523 to pivot as indicated by the arrows Y, which then causes the projections 11548 to move together, thereby releasing the rod 11540 from the spring retainer 11570.

Although the compressed gas container 3412 is shown and described above as a single-use compressed gas container disposed within the housing 3110, in some embodiments, a compressed gas container can be a multi-use container. Moreover, the compressed gas container need not be contained within the housing. For example, in some embodiments, the compressed gas container can be a container disposed outside of the housing. Additionally, the compressed gas container can be any source of pressurized gas. For example, in some embodiments, the compressed gas source can be a container having two or more chemicals formulated to produce a pressurized gas when mixed. In other embodiments, the compressed gas source can be any reservoir that can supply a gas at pressures greater than atmospheric pressure.

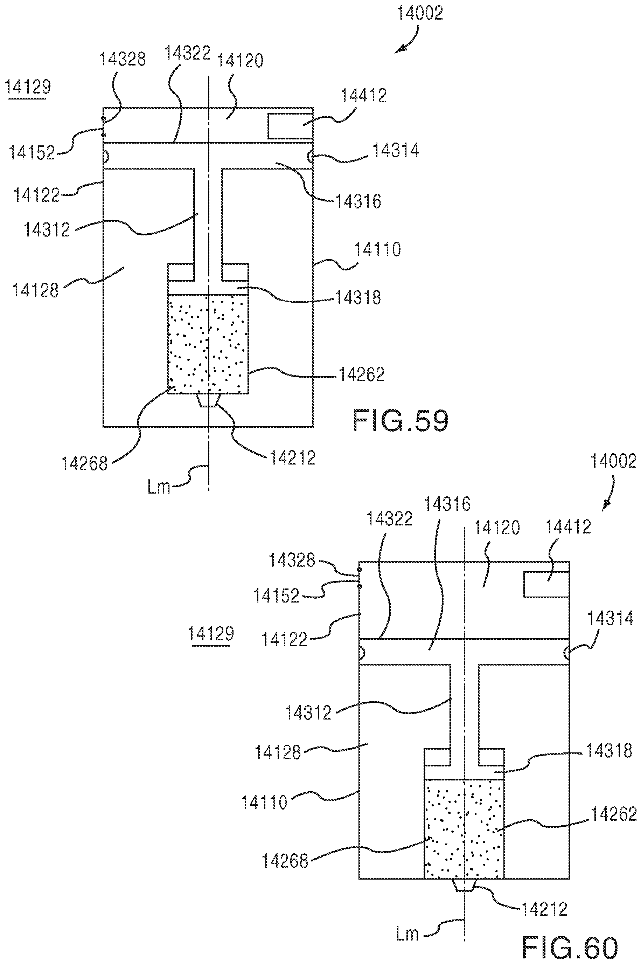

Although the auto-injectors shown and described above include a gas relief valve coupled to a movable member and configured to selectively allow fluid flow through an opening defined by the movable member, in some embodiments, the gas relief valve and/or the opening can be disposed apart from the movable member. For example, FIGS. 59 - 61 are schematic illustrations of an auto-injector 14002 in a first configuration, a second configuration and a third configuration, respectively. The auto-injector 14002 includes a housing 14110, a medicament container 14262, a movable member 14312, a gas relief valve 14328 and a compressed gas source 14412.

The medicament container 14262 is movably disposed within the housing 14110 and defines a longitudinal axis Lm. An injection member 14212 is coupled to and can be placed in fluid communication with the medicament container 14262. The injection member 14212 can be, for example, a needle, a nozzle or the like. As illustrated, the medicament container 14262 can be moved along its longitudinal axis Lm between a first position (FIG. 59) and a second position (FIG. 60). When the medicament container 14262 is in its first (or retracted) position, the injection member 14212 is disposed within the housing 14110. When the medicament container 14262 is in the second (or advanced) position (FIG. 60), a portion of the injection member 14212 is disposed outside of the housing 14110 and is placed in fluid communication with the medicament container 14262. In this manner, when the medicament container 14262 is in the second (or advanced) position, a medicament 14268 can be conveyed via the injection member 14212 from the medicament container 14262 into a body of a patient. In some embodiments, the injection member 14212 is disposed adjacent an outer surface of the housing, but can be able to deliver a medicament into a body.

The movable member 14312 includes a proximal end portion 14316 and a distal end portion 14318. As described above, the proximal end portion 14316 includes a surface 14322 that, together with the housing 14110, defines a gas chamber 14120. The proximal end portion 14316 also includes a seal 14314 that engages a portion of the housing to fluidically isolate the gas chamber 14120 from an area 14128 within the housing 14110. The distal end portion 14318 is disposed within and movable within the medicament container 14262 along the longitudinal axis Lm.

The housing 14110 includes a side wall 14122 that defines a portion of the gas chamber 14120. The side wall 14122 defines an opening 14152, which can be in fluid communication between the gas chamber 14120 and an area outside of the housing 14129. The gas relief valve 14328 is coupled to the housing 14110 such that it can selectively allow fluid communication between the gas chamber 14120 and the area outside of the housing 14129 through the opening 14152.

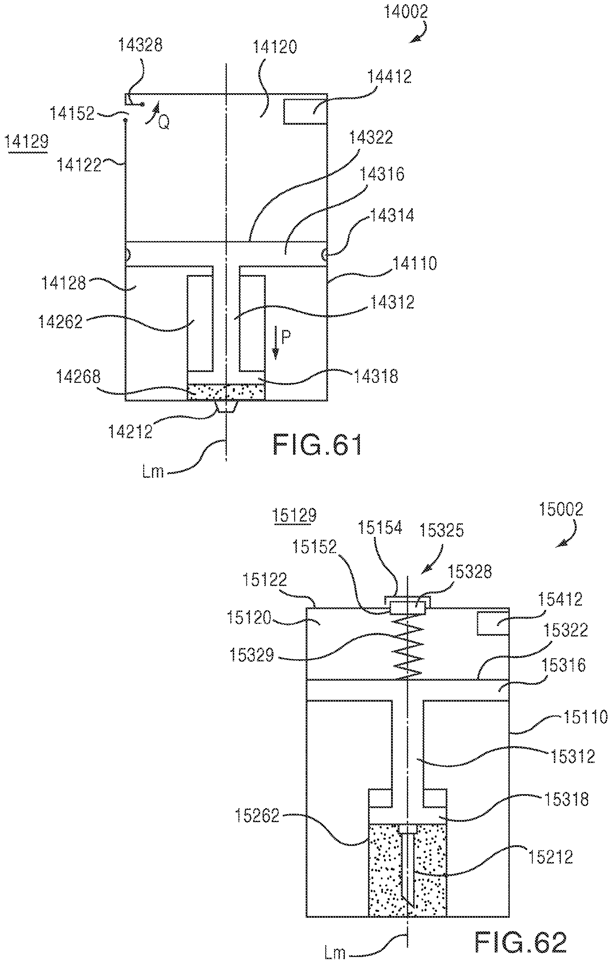

Similar to the operation described above, when the auto-injector 14002 is actuated, a pressurized gas flows from the compressed gas source 14412 into the gas chamber 14120. In response to a force produced by the pressurized gas, the movable member 14312 moves within the housing 14110 thereby placing the medicament container 14262 in its second position (FIG. 60). The movable member 14312 continues to move within the medicament container 14262, as indicated by arrow P in FIG. 61, to expel a medicament 14268 through the injection member 14212. When the medicament container 14262 is in is second position, the gas relief valve 14328 is actuated as indicated by the arrow Q in FIG. 61, thereby allowing pressurized gas to flow from the gas chamber 14120 to the area outside of the housing 14129 through the opening 14152. The gas relief valve 14328 can be actuated by any suitable valve actuator. For example, in some embodiments the auto-injector 14002 can include a mechanical valve actuator (not shown) that the user manually depresses to actuate the valve 14328.

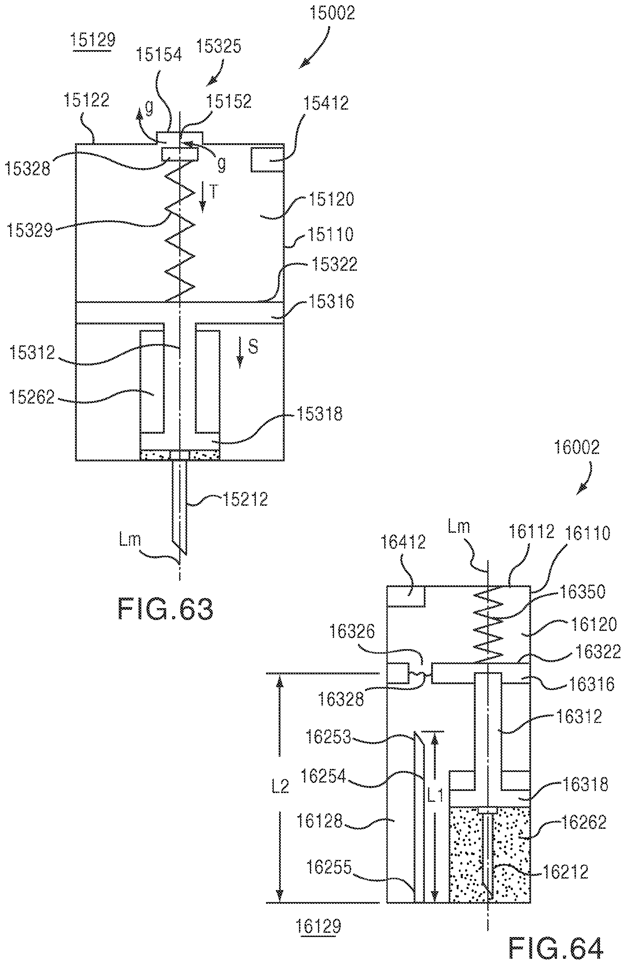

FIGS. 62 and 63 are schematic illustrations of an auto-injector 15002 in a first configuration and a second configuration, respectively. The auto-injector 15002 includes a housing 15110, a medicament container 15262, a movable member 15312, a compressed gas source 15412 and a gas release assembly 15325. As described above, the medicament container 15262 is fixedly disposed within the housing 15110 and defines a longitudinal axis Lm.

The movable member 15312 includes a proximal end portion 15316 and a distal end portion 15318. The proximal end portion 15316 includes a surface 15322 that defines a portion of a boundary of a gas chamber 15120. The distal end portion 15318 is movably disposed within the medicament container 15262 along the longitudinal axis Lm, as shown by the arrow S. A needle 15212 defining a lumen and a side opening (not shown) is coupled to the distal end 15318 of the movable member 15312.

The gas release assembly 15325 includes a gas relief valve 15328, a flexible member 15329 and an opening 15152. The opening 15152 is defined by a side wall 15122 of the housing 15110 that defines a portion of the gas chamber 15120. In this manner, the opening 15152 can provide fluid communication between the gas chamber 15120 and an area outside of the housing 15129. The housing 15110 includes a covering portion 15154 disposed adjacent the opening 15152 to prevent the opening 15152 from becoming obstructed, to prevent the gas relief valve 15328 from being inadvertently actuated or the like.

The gas relief valve 15328 is removably disposed within the opening 15152 and has a first configuration (FIG. 62) and a second configuration (FIG. 63). When the gas relief valve 15328 is in its first configuration, it is disposed within the opening 15152 such that it fluidically isolates the gas chamber 15120 from the area outside of the housing 15129. When the gas relief valve 15328 is in its second configuration, it is removed from the opening 15152, thereby placing the gas chamber 15120 in fluid communication with the area outside of the housing 15129. The gas relief valve 15328 can be, for example, a rigid member that is press fit within the opening 15152, a flexible member that is secured about the opening 15152 by an adhesive, a frangible sealing member or any other suitable device that can be removably disposed within and/or about the opening 15152.

The gas relief valve 15328 is coupled to the movable member 15312 by a flexible member 15329. By coupling the gas relief valve 15328 to the movable member 15312, the gas relief valve 15328 can be moved from its first configuration to its second configuration when the movable member 15312 reaches a predetermined position within the housing 15110. Moreover, after the gas relief valve 15328 has been actuated, this arrangement allows the gas relief valve 15328 to remain in its second configuration independent of the position of the movable member 15312. The flexible member 15329 can be any suitable structure for coupling the gas relief valve 15328 to the movable member 15312. For example, the flexible member can be a string, an elastic member, a biasing member or the like.

In use, when the auto-injector 15002 is actuated, a pressurized gas flows from the compressed gas source 15412 into the gas chamber 15120. In response to a force produced by the pressurized gas, the movable member 15312 moves within the housing 15110 and the medicament container 15262. As a result, the needle 15212 is extended through the housing 15110 and the medicament is injected via the needle 15212. When the movable member 15312 reaches a predetermined position within the housing 15110, the flexible member 15329 moves the gas relief valve 15328 into its second configuration, as shown by the arrow T in FIG. 63. In this manner, pressurized gas flows from the gas chamber 15120 to the area outside of the housing 15129 through the opening 15152, as shown by the arrows g. As the pressure in the gas chamber 15120 is reduced, the movable member 15312 and the needle 15212 can be retracted into the housing 15110, as described above.

Although the auto-injector 3002 is shown and described as including a gas relief valve 3328 that is automatically actuated by a valve actuator 3254 disposed on the carrier 3250, in some embodiments, an auto-injector can include a gas relief valve that is automatically actuated by any type of valve actuator. For example, in some embodiments, an auto-injector can include a gas relief valve that is actuated electronically, magnetically, hydraulically, pneumatically or by any other suitable mechanism. In other embodiments, an auto-injector can include a gas relief valve that is manually actuated by the user, for example, by a push button that extends within the housing.

Although the auto-injector 3002 shown and described above includes an valve actuator 3254 coupled to the carrier 3250, in some embodiments, an auto-injector can include a valve actuator disposed anywhere within the auto-injector. For example, FIGS. 64 - 66 are schematic illustrations of an auto-injector 16002 in a first configuration, a second configuration and a third configuration, respectively, in which a valve actuator 16254 is coupled to a housing 16110. The auto-injector 16002 includes the housing 16110, a medicament container 16262, a movable member 16312, a gas relief valve 16328, the valve actuator 16254 and a compressed gas source 16412. As described above, the medicament container 16262 is fixedly disposed within the housing 16110 and defines a longitudinal axis Lm.

The movable member 16312 includes a proximal end portion 16316 and a distal end portion 16318. The proximal end portion 16316 includes a surface 16322 that defines a portion of a boundary of a gas chamber 16120. The proximal end portion 16316 defines an opening 16326 therethrough, which can be selectively placed in fluid communication between the gas chamber 16120 and an area outside of the gas chamber 16128. The distal end portion 16318 is movably disposed within the medicament container 16262 along the longitudinal axis Lm, as shown by the arrow U. A needle 16212 defining a lumen and a side opening (not shown) is coupled to the distal end 16318 of the movable member 16312.

A biasing member 16350 extends between the proximal end portion 16316 of the movable member 16312 and the housing 16110. The biasing member, which can be, for example, a spring, an elastic member or the like, is configured to bias the movable member 16312 towards the proximal portion 16112 of the housing 16110.

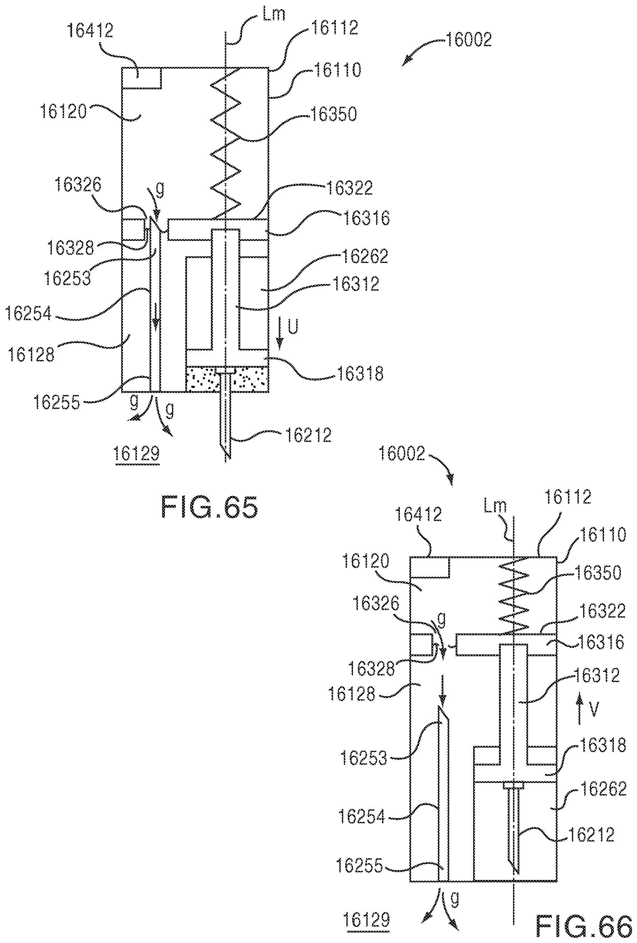

The gas relief valve 16328 is coupled to the movable member 16312 adjacent the opening 16326 and has a first configuration (FIG. 64) and a second configuration (FIGS. 65-66). When the gas relief valve 16328 is in its first configuration, it is disposed within the opening 16326 such that it fluidically isolates the gas chamber 16120 from the area outside of the gas chamber 16128. When the gas relief valve 16328 is in its second configuration, it is moved or punctured, thereby placing the gas chamber 16120 in fluid communication with the area outside of the gas chamber 16128.

The valve actuator 16254 has a proximal end 16253 and a distal end 16255 and defines a lumen therethrough (not shown). The proximal end 16253 of the valve actuator 16254 is configured to move or puncture the gas relief valve 16328 to move the gas relief valve 16328 between its first configuration and its second configuration. The distal end 16255 of the valve actuator 16254 is coupled to the housing 16110. In use, when the auto-injector 16002 is actuated, the gas chamber 16120 is placed in fluid communication with the compressed gas source 16412, thereby allowing a pressurized gas to flow into the gas chamber 16120. The force produced by the pressurized gas on the surface 16322 of the movable member 16312 causes the movable member 16312 to move within the housing 16110 and the medicament container 16262, as shown in FIG. 65. As a result, the needle 16212 is extended through the housing 16110 and the medicament is injected via the needle 16212.

When the movable member 16312 reaches a predetermined position within the housing 16110, the proximal end 16253 of the valve actuator 16254 punctures the gas relief valve 16328, thereby causing the gas relief valve 16328 to move irreversibly into its second configuration. In this manner, pressurized gas flows from the gas chamber 16120 to the area outside of the gas chamber 16128 through the opening 16326, as shown by the arrows g. The pressurized gas also flows from the area outside of the gas chamber 16128 to an area outside of the housing 16129 through the lumen defined by the valve actuator 16254. In this manner, the valve actuator 16254 defines a portion of the gas release path.

As shown in FIG. 66, when the pressurized gas flows out of the gas chamber 16120, the pressure exerted on the surface 16322 of the movable member 16312 decreases. Accordingly, the force exerted by the biasing member 16350 is sufficient to move the movable member 16312 proximally within the housing 16110, as indicated by arrow V, such that the needle 16212 is retracted into the housing 16110. Because the gas relief valve 16328 remains in its second configuration during retraction, the opening 16326 remains in fluid communication with the gas chamber 16120 and the area outside of the gas chamber 16128 independent of the position of the movable member 16312.

Additionally, the arrangement of the valve actuator 16254 can control the distance through which the movable member 16312 moves within the medicament container 16262 (i.e., the stroke of the movable member), and therefore the amount of medicament injected. As shown in FIG. 64, the stroke of the movable member 16312 is a function of the distance between the length L1 of the valve actuator 16254 and the length L2 of the movable member 16312 in its initial position. Accordingly, the stroke of the movable member 16312 can be controlled by varying the length L1 of the valve actuator 16254 and/or the length L2 of the movable member 16312 in its initial position.

The proximal end portion 16316 and the distal end portion 16318 are shown in FIGS. 64 - 66 as being separate components that are coupled together to form the movable member 16312. Such construction allows flexibility during manufacturing. For example, in some embodiments, the medicament container 16262 and the distal end portion 16318 are assembled in a sterile environment and later coupled to the proximal end portion 16316 in a non-sterile environment. In other embodiments, the two-piece arrangement of the movable member 16312 provides flexibility in setting the length L2. For example, when a greater dosage of medicament is required, a shim or spacer (not shown) can be placed in the assembly joint between the proximal end portion 16316 and the distal end portion 16318 to increase the length L2.

Although the stroke of the movable member 16312, and therefore the amount of medicament injected, is shown and described as being controlled by configuring the valve actuator 16254 to actuate the gas relief valve 16328 when the movable member 16312 has moved a predetermined distance within the medicament container 16262, in other embodiments, any suitable mechanism for controlling the stroke of the movable member can be used. For example, the auto-injector 3002 shown and described above is configured so that the movable member 3312 contacts the carrier 3250 to limit the stroke of the movable member 3312. In other embodiments, the stroke of the movable member can be limited by including a protrusion within the medicament container, such as a necked portion, that limits the motion of the piston within the medicament container. In other embodiments, the housing can include a protrusion to limit the stroke of the movable member. In yet other embodiments, a combination of each of the above methods for controlling the stroke of the movable member can be employed.

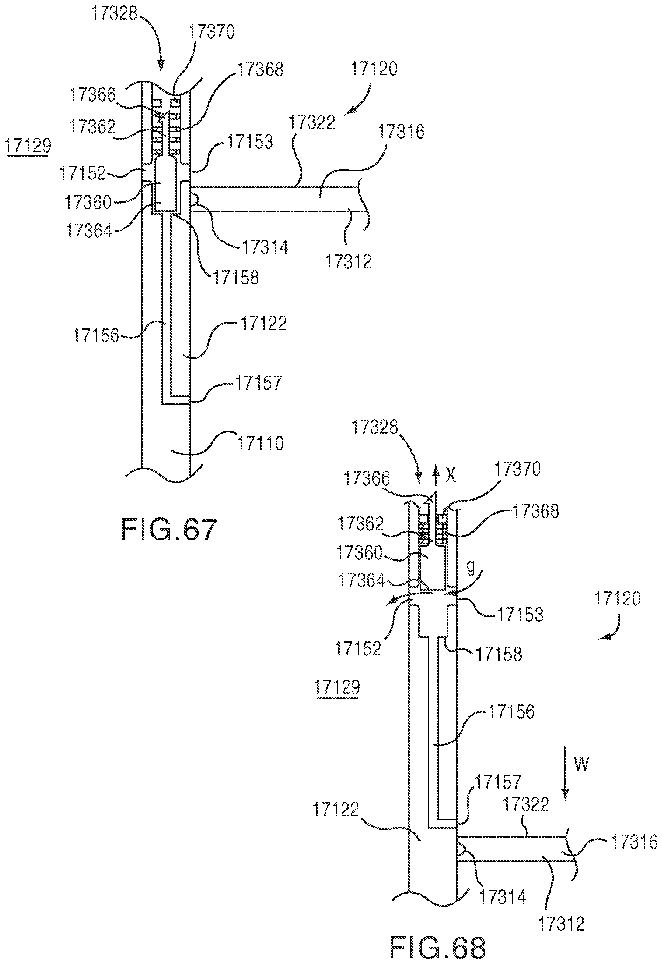

As discussed above, the valve actuator need not mechanically actuate the gas relief valve. For example, FIGS. 67 and 68 are schematic illustrations of a portion of an auto-injector 17002 having a pneumatically actuated gas relief valve 17328. Because the auto-injector 17002 is similar to the auto-injectors described above, only the gas relief mechanism is discussed in detail. The auto-injector 17002 includes a housing 17110, a movable member 17312 and a gas relief valve 17328. As described above, the movable member 17312 includes a proximal end portion 17316 that includes a surface 17322 that defines a portion of a boundary of a gas chamber 17120. The proximal end portion 17316 also includes a seal 17314 that engages a portion the housing 17110 to fluidically isolate the gas chamber 17120.

The housing 17110 includes a side wall 17122 that defines a portion of the gas chamber 17120. The side wall 17122 defines a first passageway 17152, which can be selectively placed in fluid communication between the gas chamber 17120 and an area outside of the housing 17129. The first passageway 17152 includes an opening 17153 into the gas chamber 17120 that is defined proximal to the movable member 17312. The side wall 17122 defines a second passageway 17156 that is substantially parallel to the side wall 17122 and intersects the first passageway 17152. The second passageway 17156 includes an opening 17157 selectively disposable within the gas chamber 17120 depending on the position of the movable member 17312. The opening 17157 is defined distally from the opening 17153.

The gas relief valve 17328 includes a valve body 17360, a spring 17368 and a spring retainer 17370. The valve body 17360 is movably disposed within the second passageway 17156 and has a first position (FIG. 67) and a second position (FIG. 68). The spring retainer 17370 is disposed within the second passageway 17156 and engages one end of the spring 17368. The second end of the spring 17368 engages a proximal end portion 17362 of the valve body 17360. In this manner, the valve body 17360 is biased in its first position, such that a distal end portion 17364 of the valve body 17360 engages a shoulder 17158 defined by the second passageway 17156.

When the valve body 17360 is in its first position, the valve body 17360 obstructs the first passageway 17152, thereby fluidically isolating the gas chamber 17120 from the area outside of the housing 17129. As the movable member 17312 moves distally within the housing 17110, as shown by arrow W, the seal 17314 uncovers the opening 17157 of the second passageway 17156. This allows pressurized gas from the gas chamber 17120 to flow into the second passageway 17156 and exert a force on the distal end portion 17364 of the valve body 17360. When force produced by the pressurized gas exceeds the force produced by the spring 17368, the valve body 17360 moves proximally within the second passageway 17156, as shown by arrow X. In this manner, the opening 17153 of the first passageway 17152 is uncovered, thereby allowing fluid communication between the gas chamber 17120 and the area outside of the housing 17129.

The proximal end portion 17362 of the valve body 17360 includes a projection 17366 designed to engage the spring retainer 17370 thereby maintaining the valve body 17360 in its second position. Accordingly, when the movable member 17312 moves proximally within the housing 17110 (i.e., the retraction operation) and the opening 17157 is covered by the seal 17314, the valve body 17360 will not return to its first configuration. In this manner, the gas chamber 17120 remains in fluid communication with the area outside of the housing 17129 regardless of the position of the movable member 17312, thereby ensuring that the gas chamber 17120 is fully exhausted.

Although the auto-injectors shown and described above include a gas relief valve having a first configuration in which the gas chamber is fluidically isolated and a second configuration in which the gas chamber is in fluid communication with an area outside the gas chamber, in some embodiments, an auto-injector can include a gas relief valve having more than two configurations. For example, in some configurations, an auto-injector can include a gas relief valve having a fully closed configuration, a fully opened configuration and a partially opened configuration. In this manner, the gas relief valve can be used to regulate the pressure within the gas chamber and/or the flow of the pressurized gas from the gas chamber. Such regulation can be tailored to optimize the needle insertion and/or the medicament injection operations (i.e., to ensure that the needle insertion is as painless as possible, that the medicament absorption profile is optimal, etc.).

Although the auto-injectors shown and described above include a gas relief valve that irreversibly changes from a first configuration in which the gas chamber is fluidically isolated to a second configuration in which the gas chamber is in fluid communication with an area outside the gas chamber, in some embodiments an auto-injector can include a gas relief valve configured to irreversibly change between the first configuration and the second configuration throughout the insertion and/or injection cycle. For example, in some embodiments, an auto-injector can include a gas relief valve that repeatedly cycles between its fully opened and its fully closed configurations during a single injection event. Such an arrangement also allows the gas relief valve to be used to regulate the pressure within the gas chamber and/or the flow of the pressurized gas from the gas chamber.

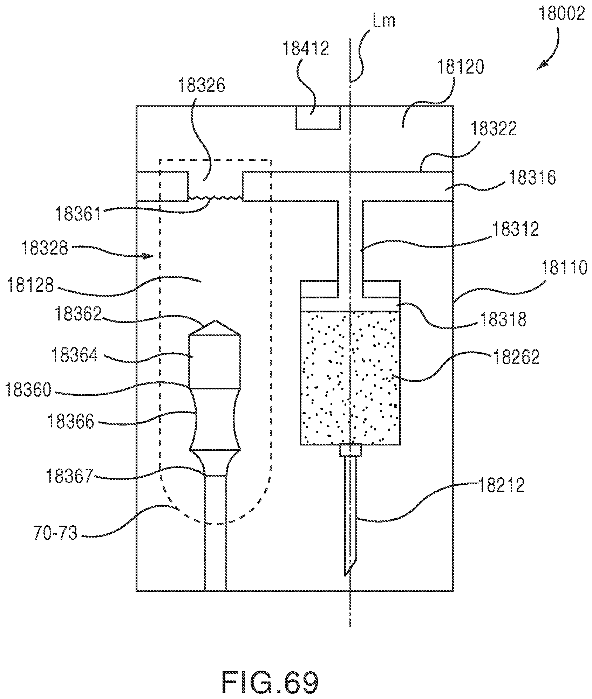

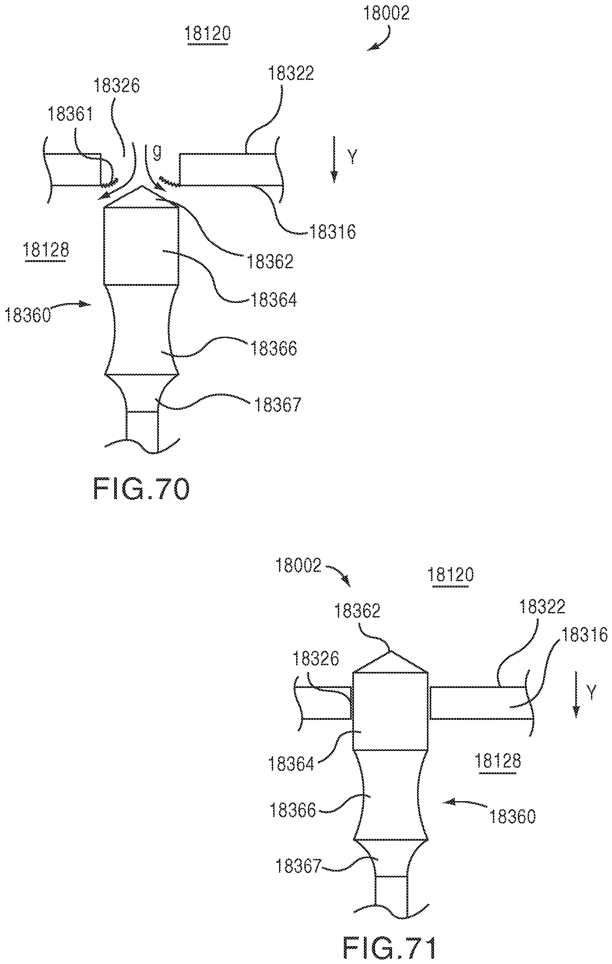

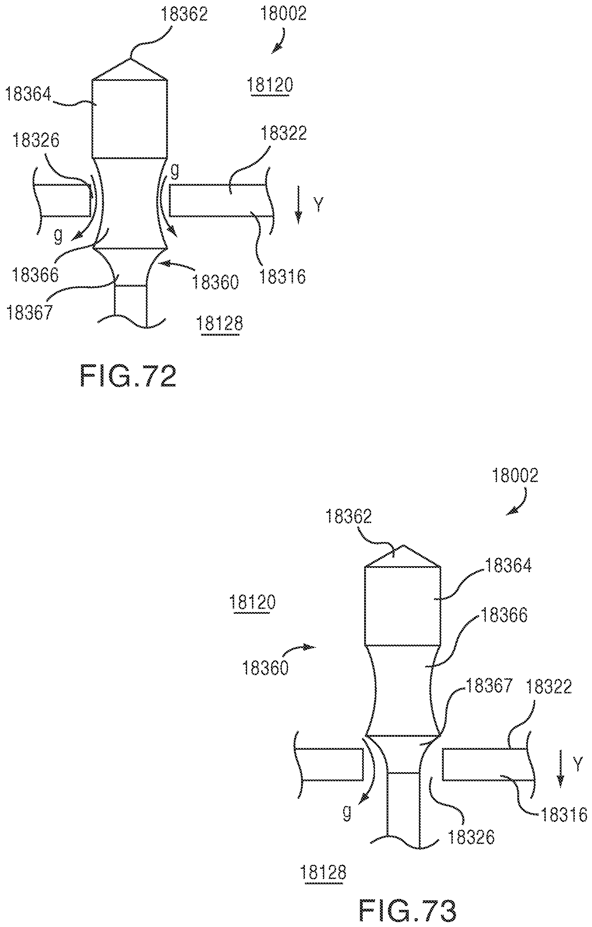

FIG. 69 is a schematic illustration of an auto-injector 18002 in which the gas relief valve 18328 has multiple different configurations, the gas relief valve 18328 being shown in a first configuration. FIGS. 70 - 73 are schematic illustrations of a portion of the auto-injector 18002 in which the gas relief valve 18328 is in a second through a fifth configuration, respectively. Because the auto-injector 18002 is similar to the auto-injectors described above, only the gas relief mechanism is discussed in detail.

The auto-injector 18002 includes a housing 18110, a movable member 18312, a medicament container 18262 and a gas relief valve 18328. The medicament container 18262 is movably disposed within the housing 18110 and defines a longitudinal axis Lm. A needle 18212 is coupled to and can be placed in fluid communication with the medicament container 18262. As described above, the medicament container 18262 can be moved along its longitudinal axis Lm between a first position (FIG. 69) and a second position. When the medicament container 18262 is in its first (or retracted) position, the needle 18212 is disposed within the housing 18110. When the medicament container 18262 is in the second position, at least a portion of the needle 18212 extends outside of the housing 18110.

The movable member 18312 includes a proximal end portion 18316 and a distal end portion 18318. As described above, the proximal end portion 18316 includes a surface 18322 that, together with the housing 18110, defines a gas chamber 18120. The proximal end portion 18316 also defines an opening 18326 therethrough, which can be selectively placed in fluid communication with the gas chamber 18120 and an area outside of the gas chamber 18128. The distal end portion 18318 is movably disposed within the medicament container 18262.

The gas relief valve 18328 includes a frangible seal 18361 and a valve body 18360. The frangible seal 18361 is coupled to the movable member 18312 adjacent the opening 18326. When the gas relief valve 18328 is in its first configuration (FIG. 69) the frangible seal 18361 fluidically isolates the gas chamber 18120 from the area outside of the gas chamber 18128. When gas relief valve 18328 is in its second through fifth configurations (FIGS. 70 - 73), the frangible seal 18361 is moved or punctured, which as described below, can allow fluid communication between the gas chamber 18120 and the area outside the gas chamber 18128 via the opening 18326. The valve body 18360 is coupled to the housing 18110 and is configured to be disposed within the opening 18326 when the movable member 18312 moves distally within the housing 18110. The valve body includes a first portion 18362, a second portion 18364, a third portion 18366 and a fourth portion 18367.

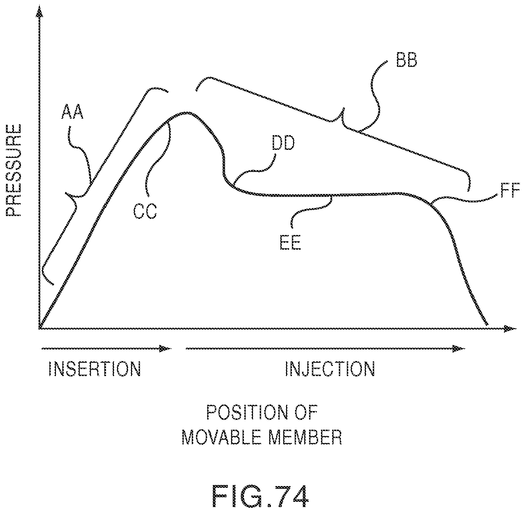

The operation of the auto-injector 18002 and the various configurations of the gas relief valve 18128 are discussed with reference to FIG. 74, which shows a plot of the pressure within the gas chamber 18120 as a function of the position of the movable member 18312. In FIG. 74, the position of the movable member 18312, which also corresponds to the configuration of the gas relief valve, is represented on the x-axis. The pressure within the gas chamber 18120 is represented on the y-axis.

In use, when the auto-injector 18002 is actuated, a pressurized gas flows from a compressed gas source 18412 (see FIG. 69) into the gas chamber 18120, causing the movable member 18312 to move distally within the housing. The movable member 18312 moves the medicament container 18262 between its first and its second position (the "needle insertion" operation). The needle insertion operation is shown in FIG. 74 as region AA. As shown in FIG. 70, towards the end of the needle insertion operation, the movable member 18312 is positioned such that the first portion 18362 of the valve body 18360 moves or punctures the frangible seal 18361, thereby placing the gas relief valve 18128 in its second configuration (point CC on the plot in FIG. 74). When the gas relief valve is in its second configuration, the gas chamber 18120 is in fluid communication with the area outside the gas chamber 18128 via the opening 18326. Accordingly, the pressure within the gas chamber 18120 is reduced, as indicated in FIG. 74. Reducing the pressure during the needle insertion operation can, for example, reduce patient discomfort during the needle insertion operation.

When the medicament container 18262 reaches its second position, the movable member 18312 continues to move distally within the medicament container 18262, as shown by arrow Y, to inject the medicament through the needle 18212. The medicament injection operation is shown in FIG. 74 as region BB. As shown in FIG. 71, during the beginning of the injection operation, the movable member 18312 is positioned such that the second portion 18364 of the valve body 18360 is disposed within the opening 18326, placing the gas relief valve 18128 in its third configuration (point DD on the plot in FIG. 74). The second portion 18364 of the valve body 18360 is configured to fit within the opening 18326 such that the gas chamber 18120 is substantially fluidically isolated from the area outside of the gas chamber 18128. Because pressurized gas continues to flow from the compressed gas source (not shown) into the gas chamber 18120, by fluidically isolating the gas chamber 18120, the pressure within the gas chamber 18120 will no longer decrease, but will instead remain constant or increase slightly.

During the middle portion of the injection operation, the movable member 18312 is positioned such that the third portion 18366 of the valve body 18360 is disposed within the opening 18326, placing the gas relief valve 18128 in its fourth configuration (point EE on the plot in FIG. 74). The third portion 18366 of the valve body 18360 is shaped to allow a controlled amount of pressurized gas to flow from the gas chamber 18120 to the area outside the gas chamber 18128 via the opening 18326. Said another way, the third portion 18366 of the valve body 18360 and the opening 18326 define a flow passageway between the gas chamber 18120 and the an area outside the gas chamber 18128. The flow passageway varies based on the shape of the third portion 18366 of the valve body 18360. For example, a narrow shaped third portion 18364 results in a larger flow area, whereas a larger shaped third portion 18366 results in a smaller flow area. In this manner, the flow area can be varied as a function of a longitudinal position of the movable member 18312. The third portion 18366 can be shaped such that the pressurized gas entering the gas chamber 18120 from the compressed gas source (not shown) is equal to the pressurized gas exiting the gas chamber 18120. Accordingly, as shown in FIG. 74, the pressure within the gas chamber 18120 can be substantially constant throughout the injection operation.

At the end of the injection operation, the movable member 18312 is positioned such that the fourth portion 18367 of the valve body 18360 is disposed within the opening 18326, placing the gas relief valve 18128 in its fifth configuration (point FF on the plot in FIG. 74). The fourth portion 18367 of the valve body 18360 is considerably smaller than the third portion 18366, thereby allowing a significant amount of pressurized gas to flow from the gas chamber 18120 to the area outside the gas chamber 18128 via the opening 18326. Said another way, when the fourth portion 18367 of the valve body 18360 is within the opening 18326, the valve 18128 is "fully opened." Accordingly, as shown in FIG. 74, the pressure within the gas chamber 18120 decreases rapidly. In some embodiments, the rapid drop in pressure allows the movable member 18312 to be retracted by a biasing member. In this manner, the needle 18212 is also retracted into the housing 18110, thereby minimizing post-injection hazards.

Although the gas relief valve 18128 is described as being a mechanical component that varies a flow area as a function of the movable member, in other embodiments, the gas relief valve can be any suitable type of variable area valve. For example, in some embodiments, a gas relief valve can be an electrically operated spool valve.

While the valve body 18360 is shown as having four distinct regions corresponding to four variably functional positions, in other embodiments, the valve body can have fewer or greater distinct regions corresponding to a different number of functional positions. Additionally, the shapes and sizes of the illustrated valve body portions 18362, 18364, 18366 and 18367 are shown by way of example only. In some embodiments, the valve body can be shaped according to a desired pressure and/or injection profile.

Although the auto-injectors are shown and described above as having a single gas chamber and a single gas relief valve, in some embodiments, an auto-injector can include any number of gas chambers and/or gas relief valves. For example, in some embodiments, an auto-injector can include a compressed gas source, an auxiliary gas chamber and a primary gas chamber. In a similar manner as described above, the compressed gas source can be selectively placed in fluid communication with the auxiliary gas chamber, thereby allowing the auxiliary gas chamber to be filled with a pressurized gas. The auto-injector can include a first gas relief valve configured to selectively place the auxiliary gas chamber in fluid communication with the primary gas chamber. When pressurized gas is conveyed from the auxiliary gas chamber into the primary gas chamber via the first gas relief valve, the gas pressure within the primary gas chamber causes an injection event, as described above. The auto-injector can also include a second gas relief valve configured to selectively place the primary gas chamber in fluid communication with an area outside of the auto-injector housing. By including an auxiliary gas chamber, which can be vented independently from the primary gas chamber, the auto-injector can be configured as a multiple-use injector.

Similarly, while the auto-injectors are shown and described above as having an area outside of the gas chamber that is in fluid communication with an area outside of the housing, in some embodiments, the area outside of the gas chamber need not be vented to the atmosphere. For example, in some embodiments, an auto-injector can include an area outside of the gas chamber that is in fluid communication with a secondary gas chamber.

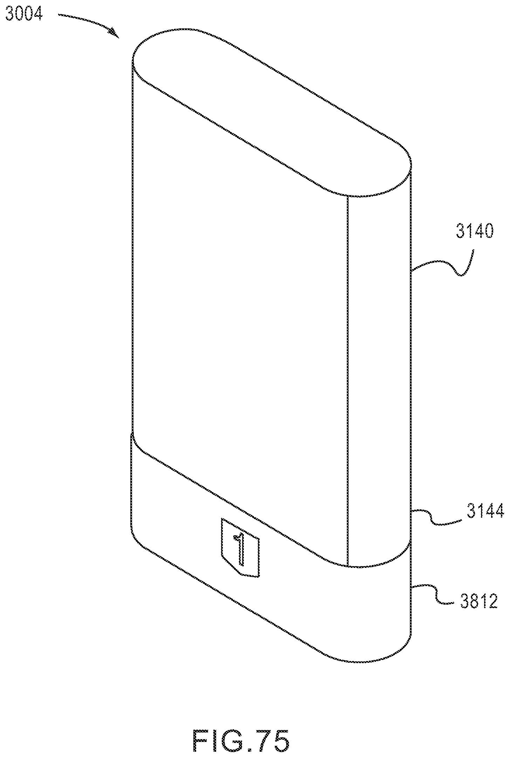

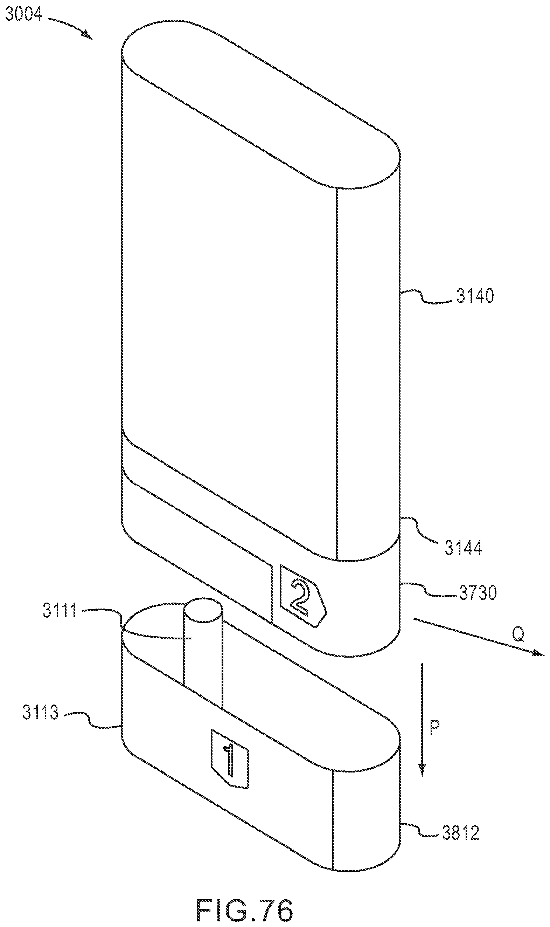

Although the needle guard auto-injector 3002 is illustrated and described above as including a needle guard that covers or is disposed on a portion of the distal end 3114 of the housing 3110, in some embodiments, an auto-injector 3004 includes a housing 3140 and a needle guard 3812 removably coupled to the distal end portion 3144 of the housing 3140, as illustrated in FIGS. 75 - 76. The needle guard 3812 includes a sheath 3111 and a sheath retaining portion 3113. The needle guard 3812 has a first position and a second position. In its first position, the needle guard 3812 is coupled to the housing 3140. For example, the sheath retaining portion 3113 of the needle guard 3812 is configured to substantially cover or encase the distal end portion 3144 of the housing 3140 when the needle guard is in its first position. In its second position, the needle guard 3812 is removed from the housing 3140. The sheath 3111 is coupled to the sheath retaining portion 3113 similar to the coupling of the sheath 3820 and sheath retainer 3840 as described in detail above with reference to FIG. 22. As such, as the sheath retaining portion 3113 is moved distally in the direction of arrow P, the sheath 3111 is also moved distally and removed from the housing 3140. Once the needle guard 3812 is moved to its second (or removed) position, the safety lock 3730 is accessible. The safety lock 3730 is removed from the housing 3140 by pulling the safety lock in a direction that is substantially normal to the direction in which the needle guard 3812 is removed, such as in the direction of arrow Q as illustrated in FIG. 76.

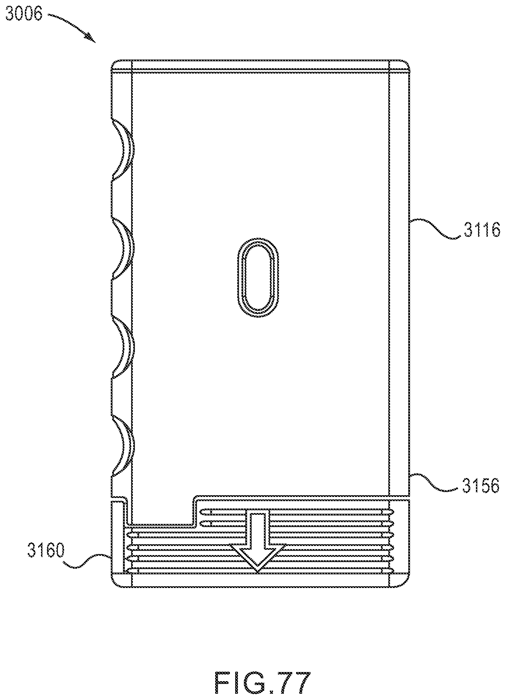

In some embodiments, as illustrated in FIGS. 77 - 79, an auto-injector 3006 includes a housing 3116, a safety guard 3130, and a distal end cap 3160. The distal end cap 3160 is configured to selectively engage or be coupled to the housing 3116. The distal end cap 3160 prevents inadvertent actuation of the auto-injector 3006 by substantially covering at least a portion of the safety guard 3130 when the distal end cap 3160 is engaged with or coupled to the housing 3116.

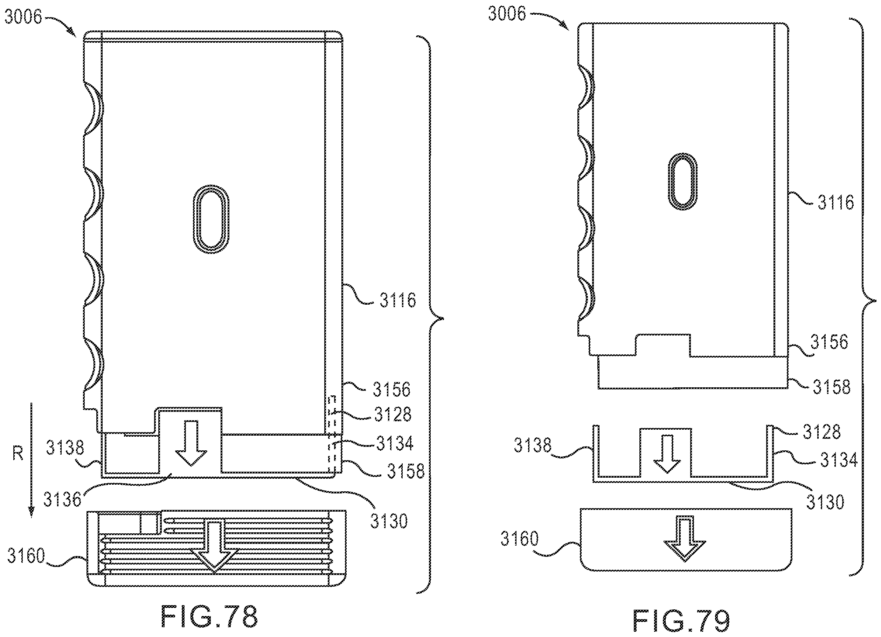

The distal end cap 3160 has a first position and a second position. In its first position, illustrated in FIG. 77, the distal end cap 3160 is removably coupled to or engaged with the distal end portion 3156 of the housing 3116. In its second position, illustrated in FIG. 78, the distal end cap 3160 is removed from the housing 3116. The distal end cap 3160 must be removed from the auto-injector 3006 before the auto-injector can be enabled for use, thus preventing inadvertent actuation of the device. Furthermore, the distal end cap 3160 provides an additional barrier to contamination of the needle and the medicament disposed therein. The distal end cap 3160 can have a series of ridges or other tactile mechanism for assisting a user in gripping and/or removing the distal end cap. The distal end cap 3160 is replaceable. As such, if the distal end cap 3160 is removed before a user intends to use the auto-injector 3006, the user can put the distal end cap back in its first position without actuating or jeopardizing the sterility of the device.

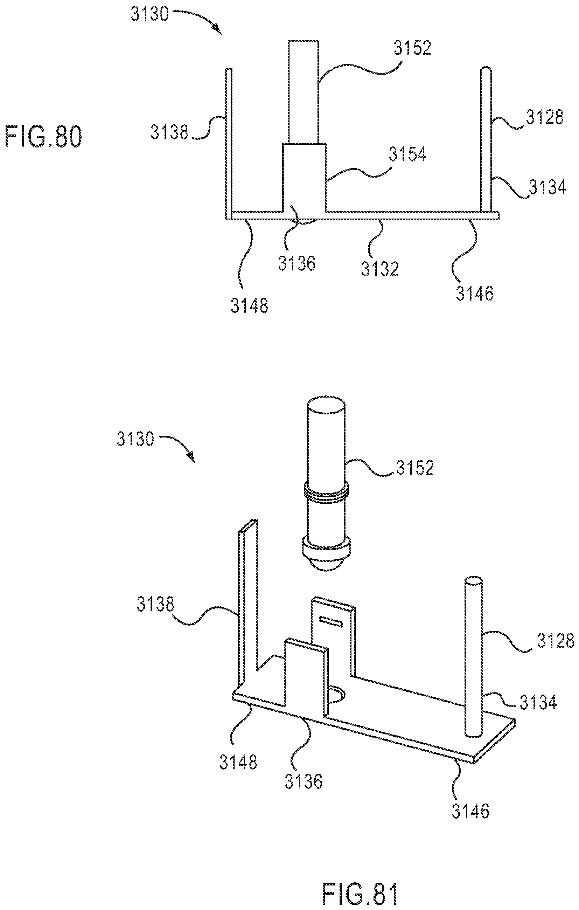

Once the distal end cap 3160 is removed, the safety guard 3130 is exposed and can be removed. With the safety guard 3130 in place, the auto-injector 3006 can not be actuated. The safety guard 3130, illustrated in FIGS. 80 - 81, includes a base portion 3132, a locking portion 3134, and a needle guard portion 3136. The locking portion 3134 and needle guard portion 3136 extend proximally from the base portion 3132. The base includes a first end 3146 and a second end 3148. The locking portion 3134 is disposed adjacent the first end 3146 and includes a first engagement portion 3128. A second engagement portion 3138 is disposed adjacent the second end 3148 of the base portion 3132.

The needle guard portion 3136 of the safety guard 3130 includes a sheath 3152 and a sheath retaining portion 3154. The sheath 3152, which is similar to sheath 3820 discussed in detail above, defines an opening configured to receive at least a portion of a needle of the auto-injector and is removably coupled to the sheath retaining portion 3154. The sheath retaining portion 3154 is couplable to the housing 3116 or to the base 3158 which is coupled to the housing.

The safety guard 3130 has a first position and a second position. In its first position, illustrated in FIG. 78, the safety guard 3130 is coupled to the distal end 3156 of the housing 3116. For example, the safety guard 3130 can be coupled to a base 3158 movably coupled to the distal end 3156 of the housing 3116. In its second position, shown in FIG. 79, the safety guard 3130 is removed from the housing 3116. The safety guard 3130 is removed from the housing 3116 by pulling the safety guard distally in the direction of arrow R.

When the safety guard 3130 is in its first position, the locking portion 3134 inhibits or prevents actuation of the auto-injector 3006. Referring to FIG. 78, the locking portion 3134 includes a first engagement portion 3128, or protrusion, that extends at least partially into the housing 3116 of the auto-injector 3006 (shown in dashed lines). In some embodiments, the locking portion 3134 extends through an opening (not shown in FIG. 78) of the base 3158 movably coupled to the distal end portion 3156 of the housing 3116, similar to the opening 3522 defined by base 3520 as illustrated in FIG. 28. The locking portion 3134 is configured to keep separate the projections of the actuator, similar to projections 3548 of actuator 3510 illustrated in FIG. 27, when the safety guard 3130 is in its first position. As the safety guard 3130 is moved from its first position to its second position, the locking portion 3134 is removed from between the projections 3548. Thus, the projections can be moved to actuate the auto-injector as previously described.

When the safety guard 3130 is in its first position, the needle guard portion 3136 substantially covers the needle (not shown) of the auto-injector 3006. As the safety guard 3130 is moved to its second position, the sheath retaining portion 3154 remains coupled to the sheath 3152, and thus sheath is removed from its position covering the needle.

The second engagement portion 3138 of the safety guard 3130 is configured to be selectively coupled to at least a portion of the housing 3116 when the safety guard 3130 is in its first position. The second engagement portion 3138, for example, can assist in guiding and removing the safety guard 3130 by balancing the safety guard relative to the housing 3116. In other words, as the safety guard 3130 is moved to its second (or removed) position, the second engagement portion 3138 inhibits the safety guard 3130 from becoming skewed, and restricting movement of the first engagement portion 3128. In some embodiments, the second engagement portion 3138 can be coupled to the housing 3116 to prevent unwanted movement of the safety guard 3130 away from the housing, such as via a resistance fit with the housing.

In some embodiments, the safety guard 3130 is constructed monolithically. In other embodiments, the safety guard can be constructed from separate components. For example, one or more of the base portion, locking portion and/or needle guard portion can be constructed separately and then coupled to the other portions. Although the illustrated embodiment shows the second engagement portion 3138 as being disposed at or proximate to an edge of the base portion 3132, in some embodiments, the second engagement portion 3138 can be disposed elsewhere on the base portion. Although the first engagement portion 3128, or locking member, is illustrated as being at or proximate to an edge of the base portion 3132, in some embodiments, the first engagement portion 3128 can extend from another portion of the base portion.

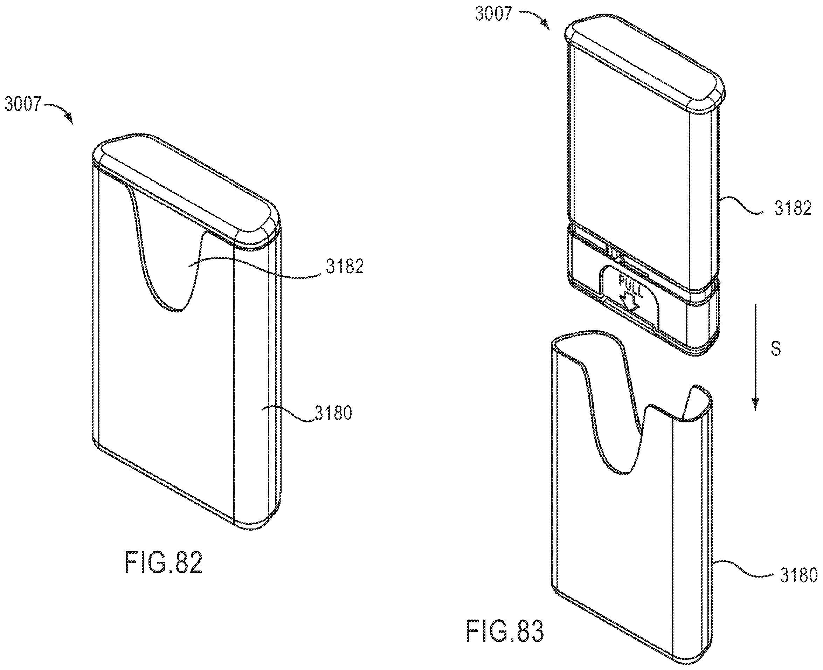

In some embodiments, a sleeve covers all or at least a substantial portion of the auto-injector. For example, as illustrated in FIG. 82, the sleeve 3180 covers substantially all of the safety guard (not shown) and the housing 3182 of the auto-injector 3007. The sleeve 3180 can be configured for use in an embodiment having only a safety lock or a separate needle guard and safety lock. The sleeve 3180 has a first position in which the sleeve is configured to substantially cover the housing 3182, as illustrated in FIG. 82, and a second position in which the sleeve is configured to be removed from the housing 3182 by pulling the sleeve distally in the direction of arrow S, as illustrated in FIG. 83.

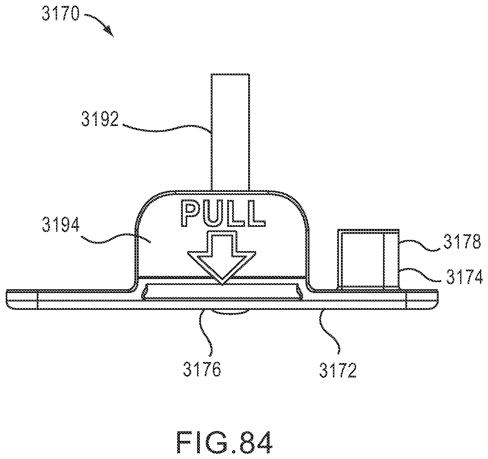

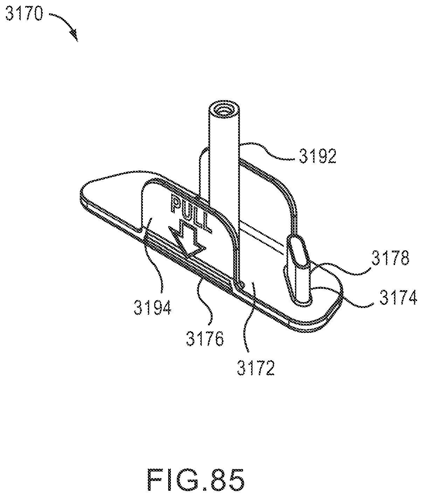

Although the safety lock is described as having a first engagement portion and a second engagement portion, in some embodiments, the safety lock has only a first engagement portion. For example, as illustrated in FIGS. 84 and 85, a safety lock 3170 includes a locking portion 3174 and a needle guard portion 3176. The locking portion 3174 has a first engagement portion 3178 disposed on the base portion 3172 of the safety lock. The first engagement portion 3178 extends proximally from the base portion 3172. The needle guard portion 3176 includes a sheath 3192 and a sheath retaining portion 3194. The sheath retaining portion 3194 extends proximally from the base portion 3172 and is coupled to the sheath 3192. The sheath retaining portion 3194 is coupled to the sheath 3192 similar to the coupling of the sheath 3820 and sheath retainer 3840 as described in detail above with reference to FIG. 22. The safety lock 3170 is removed by pulling the safety lock distally in the direction of arrow S as shown in FIG. 83, away from housing 3182. When the safety lock 3170 is in its second (or removed) position, the first engagement portion 3178 is removed from between the projections of the system actuator rod (not shown in FIG. 83), and thus the auto-injector 3007 can be actuated.

In some embodiments, the locking member, distal end cap, safety guard, or sleeve are configured to mate or otherwise interface with the housing to prevent actuation of the auto-injector. The connection between the housing and the sleeve, for example, can be a snug fit and can be an interlocking connection. For example, in some embodiments, some force must be applied to remove the distal end cap, safety guard, or sleeve from the housing.

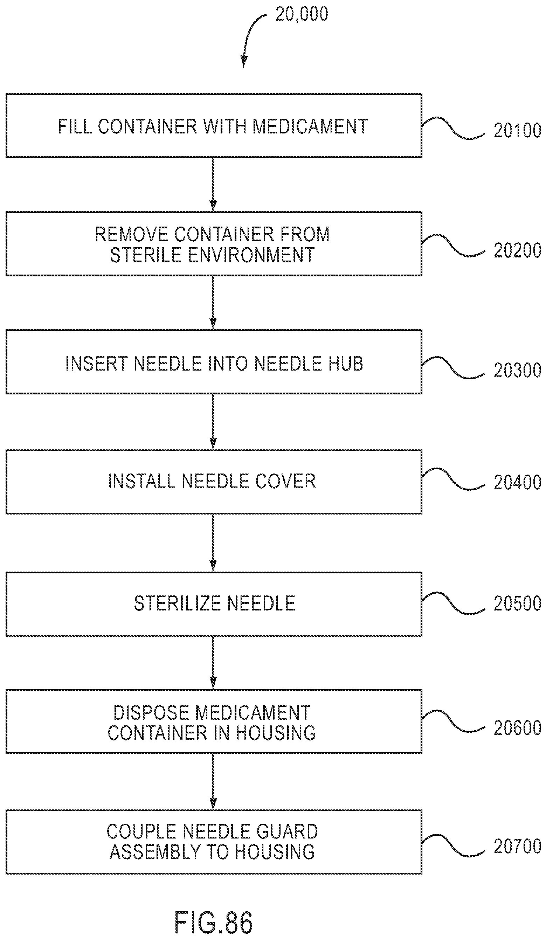

FIG. 86 is a flowchart of an embodiment of a method 20000 for manufacturing a medicament delivery apparatus. At 20100, a medicament container is filled with a predetermined amount of medicament. For example, the medicament container can be filled with a predetermined amount of epinephrine. As used herein, filling the medicament container includes putting medicament into the container, not necessarily filling the container to capacity. The filling of the medicament container occurs in a sterile environment. In some embodiments, the container can be filled with a second medicament. In such an embodiment, the second medicament can be any constituent of a medicament, including water. Once the medicament container is filled, a seal can be placed on the container to prevent leakage and/or contamination of the medicament. At activity 20200, the medicament container is removed from the sterile environment. For example, the medicament container can be filled in a first sterile manufacturing facility, and then the filled containers can be transported to a second facility, which is not necessarily a sterile facility, to continue assembly of the apparatus.

At 20300, at least a portion of a needle is inserted into a needle hub disposed in or on a housing. At 20400, a needle cover, or sheath, is installed over at least a portion of the needle so that the needle cover substantially covers the portion of the needle extending from the needle hub. For example, a needle cover constructed of at least one of polyethylene, high density polyethylene, polypropylene, polytetrafluoroethylene, thermoplastic polyurethane, rubber, a polymer, or an elastomer can be installed to cover at least a portion of the needle extending from the needle hub. When the needle cover is installed, the needle cover can also be coupled to the needle hub. For example, in some embodiments the needle cover includes a recessed portion configured to be coupled to a corresponding protrusion on the needle hub. In some embodiments, the recessed portion and the protrusion forms a seal that is resistant to microbial penetration. One or both of the inserting the needle into the needle hub 20300 and installing the needle cover 20400 can occur in a non-sterile environment.

At 20500, the needle is sterilized. Various sterilization techniques may be utilized. In some embodiments, a suitable sterilization technique includes the use of one or more of ethylene oxide, gamma radiation, e-beam radiation, ultraviolet radiation, steam, plasma, or hydrogen peroxide. In some embodiments, the needle is sterilized prior to installing the needle cover. In some embodiments, the needle is sterilized after the needle cover is installed. For example, in some embodiments, the needle cover is installed and then a gas sterilant is conveyed through at least a portion of the needle cover. The needle is sterilized using a gas sterilization technique that can penetrate one or more pores of a porous needle cover. In some embodiments, the needle can be sterilized using a gas sterilization technique that can penetrate one or more pores of a porous needle cover, but that will not react with a medicament in a medicament container disposed in the housing.

In some embodiments, the gas sterilant is conveyed through a valve disposed on the needle cover. For example, the valve may be a one-way check valve, a springloaded valve, a self-sealing membrane, or the like.

At 20600, the medicament container is disposed in the housing. At 20700, a needle guard assembly is coupled to at least one of a distal end portion of the housing or an actuator (or base portion) coupled to the housing. In some embodiments, the coupling includes coupling a one piece safety guard that is configured to prevent actuation of the apparatus and to receive at least a portion of the needle cover. In some embodiments, the coupling includes first coupling an actuation guard, or locking member, configured to prevent actuation of the apparatus, and then coupling a needle guard configured to receive at least a portion of the needle cover and to prevent movement of the locking member when the needle guard is coupled to the housing or the base portion.

Although disposing the medicament container in the housing is illustrated and described as occurring after the needle cover is installed over at least a portion of the needle, in some embodiments, the medicament container is attached to the needle hub when the needle cover is installed over at least a portion of the needle.

Although only the needle is illustrated and described as being sterilized, in some embodiments, one or more of the needle hub, needle cover, and medicament container are sterilized in addition to the needle being sterilized. The sterilization of the needle hub, needle cover, medicament container and needle can occur substantially simultaneously or at different times.

Although the flowchart in FIG. 86 presents each activity for manufacturing an auto-injector in a particular order, the various activities can occur in a different order. For example, the medicament container can be filled with medicament after the needle has been sterilized. In another example, the medicament container can be disposed in the housing prior to inserting the portion of the needle into the needle hub.

While various embodiments of the invention have been described above, it should be understood that they have been presented by way of example only, and not limitation. Where methods described above indicate certain events occurring in certain order, the ordering of certain events may be modified. Additionally, certain of the events may be performed concurrently in a parallel process when possible, as well as performed sequentially as described above.

Although various embodiments have been described as having particular features and/or combinations of components, other embodiments are possible having a combination of any features and/or components from any of embodiments where appropriate. For example, in some embodiments, an auto-injector can include a fluidic circuit to change the direction and/or magnitude of the force produced by the energy storage member and a fluid relief valve to relieve the pressure within the fluidic circuit to assist in the retraction of the needle. In another example, in some embodiments, a gas relief mechanism can include an first opening defined by the movable member that can be selectively placed in fluid communication with the gas chamber and an area outside of the gas chamber and a second opening defined by the housing that can be selectively placed in fluid communication with the gas chamber and an area outside of the housing. In still another example, in some embodiments, the sleeve 3180 illustrated in FIG. 82 can be used in connection with the auto-injector 3002, 3004, 3006. Additionally, any of the components of the needle guard and safety lock can be interchanged with similar components in similar embodiments.

## Claims

1. An apparatus (1000, 3002, 3006, 3007), comprising:
a housing (1100, 3110, 3116, 3182) having a distal end portion and a proximal end portion, the distal end portion including a distal end surface;
a medicament injector including a medicament container (3262) and a needle (3212, 6100), the needle configured to move within the housing between a first needle position and a second needle position, in the first needle position the needle is contained within the housing, in the second needle position at least a portion of the needle extends from the distal end portion of the housing;
an energy storage member (2400, 2412, 3412) having a first configuration and a second configuration, the energy storage member configured to produce a force when transitioned from the first configuration to the second configuration to move the needle between the first needle position and the second needle position; and
an actuation member (3540, 7540, 9540, 10540) configured to transistion the energy storage member from its first configuration to its second configuration when the actuation member is moved from a first position to a second position; and
a locking member (3130, 3170) configured to be removeably coupled to the distal end portion of the housing, a protrusion (3128, 3178) of the locking member disposed through an opening defined by the distal end surface of the housing such that the protrusion engages the actuation member to prevent movement of the actuation member from the first position to the second position when the locking member is coupled to the distal end portion of the housing.

2. The apparatus (1000, 3002, 3006, 3007) of claim 1, wherein a longitudinal axis of the medicament container (3262) is offset from a longitudinal axis of the energy storage member (2400, 2412, 3412).

3. The apparatus (1000, 3002, 3006, 3007) of claim 1, wherein a longitudinal axis of the medicament container (3262) is substantially parallel to a longitudinal axis of the energy storage member (2400, 2412, 3412).

4. The apparatus (1000, 3002, 3006, 3007) of claim 1, wherein the energy storage member (2400, 3412) is configured to move between a first energy storage member position and a second energy storage member position, the energy storage member being disposed in its first energy storage member position when the energy storage member is in its first configuration, the energy storage member being disposed in its second energy storage member position when the energy storage member is in its second configuration.

5. The apparatus (1000, 3002, 3006, 3007) of claim 1, wherein the energy storage member (2400, 2412, 3412) is a gas container configured to contain a gas having a first pressure when the gas container is in its first configuration and a second pressure when the gas container is in its second configuration, the second pressure being lower than the first pressure, the apparatus further comprising:
a puncturer configured to penetrate a portion of the gas container when the gas container is in its second configuration.

6. The apparatus (1000, 3002, 3006, 3007) of claim 1, further comprising:
a biasing member (3560) configured to bias the actuation member towards its second position.

7. The apparatus (1000, 3002, 3006, 3007) of claim 1, further comprising:
a spring (1600, 3560) configured to bias the actuation member towards its second position, the actuation member including a distal end (3544) configured to engage the distal end portion of the housing and a proximal end (3542) configured to engage the energy storage member, the actuation member configured to be moved from its first position to its second position by disengaging the distal end of the actuation member from the distal end portion of the housing.

8. The apparatus (1000, 3002, 3006, 3007) of claim 1, wherein:
the needle (3212, 6100) moves in a first direction when moved from the first needle position to the second needle position;
and the actuation member moves in a second direction when moved from its first position to its second position, the second direction substantially opposite the first direction.

9. The apparatus (1000, 3002, 3006, 3007) of claim 1, wherein the actuation member is a first actuation member, the apparatus further comprising:
a second actuation member (3520, 3158), the second actuation member defining an opening (3532) within which a portion of the needle (3212, 6100) is disposed when the needle is in the second needle position.

10. The apparatus (1000, 3002, 3006, 3007) of claim 1, further comprising:
a retraction spring (3350) configured to bias the needle in the first needle position.

11. The apparatus (1000, 3002, 3006, 3007) of claim 1, further comprising:
a retraction spring (3350) configured to bias the needle in the first needle position, a longitudinal axis of the retraction spring being offset from a longitudinal axis of the medicament container.

12. The apparatus (1000, 3002, 3006, 3007) of claim 1, wherein the actuation member (3540) is a first actuation member, the apparatus further comprising:
a second actuation member (3520, 3158) coupled to the distal end portion of the housing (1100, 3110, 3116, 3182), the second actuation member configured to cause the first actuation member to move from its first position to its second position when the second actuation member is manipulated.

13. The apparatus (1000, 3002, 3006, 3007) of claim 1, wherein the locking member includes a needle sheath (3820, 3111, 3152, 3192)configured to be disposed about at least a portion of the needle when the locking member is coupled to the distal end portion of the housing (1100, 3110, 3116, 3182).

14. The apparatus (1000, 3002, 3006, 3007) of claim 1, wherein:
the actuation member (3540, 7540, 9540, 10540) includes a rod (3540) having a plurality of projections (3548); and
the protrusion is configured to extend between the plurality of projections of the rod, the protrusion configured to prevent the plurality of projections from being moved inwardly towards each other when the locking member is coupled to the distal end portion of the housing (1100, 3110, 3116, 3182).

15. The apparatus (1000, 3002, 3006, 3007) of claim 1, wherein the medicament container (3262) includes epinephrine.

## Patentansprüche

1. Einrichtung (1000, 3002, 3006, 3007), Folgendes umfassend:
ein Gehäuse (1100, 3110, 3116, 3182) mit einem distalen Endabschnitt und einem proximalen Endabschnitt, wobei der distale Endabschnitt eine distale Endoberfläche einschließt;
einen Medikamenteninjektor, der einen Medikamentenbehälter (3262) und eine Nadel (3212, 6100) einschließt, wobei die Nadel konfiguriert ist, um sich innerhalb des Gehäuses zwischen einer ersten Nadelposition und einer zweiten Nadelposition zu bewegen, wobei die Nadel in der ersten Nadelposition innerhalb des Gehäuses enthalten ist, wobei sich in der zweiten Nadelposition wenigstens ein Abschnitt der Nadel von dem distalen Endabschnitt des Gehäuses erstreckt;
ein Energiespeicherelement (2400, 2412, 3412) mit einer ersten Konfiguration und einer zweiten Konfiguration, wobei das Energiespeicherelement konfiguriert ist, um eine Kraft zu erzeugen, wenn es von der ersten Konfiguration in die zweite Konfiguration gewechselt wird, um die Nadel zwischen der ersten Nadelposition und der zweiten Nadelposition zu bewegen; und
ein Betätigungselement (3540, 7540, 9540, 10540), das konfiguriert ist, um das Energiespeicherelement von seiner ersten Konfiguration in seine zweite Konfiguration zu wechseln, wenn das Betätigungselement von einer ersten Position in eine zweite Position bewegt wird; und
ein Verriegelungselement (3130, 3170), das konfiguriert ist, um abnehmbar an den distalen Endabschnitt des Gehäuses gekoppelt zu sein, einen Vorsprung (3128, 3178) des Verriegelungselements, der durch eine Öffnung angeordnet ist, die durch die distale Endoberfläche des Gehäuses definiert ist, sodass der Vorsprung das Betätigungselement in Eingriff nimmt, um ein Bewegen des Betätigungselements von der ersten Position in die zweite Position zu verhindern, wenn das Verriegelungselement an den distalen Endabschnitt des Gehäuses gekoppelt ist.

2. Einrichtung (1000, 3002, 3006, 3007) nach Anspruch 1, wobei eine Längsachse des Medikamentenbehälters (3262) von einer Längsachse des Energiespeicherelements (2400, 2412, 3412) versetzt ist.

3. Einrichtung (1000, 3002, 3006, 3007) nach Anspruch 1, wobei eine Längsachse des Medikamentenbehälters (3262) im Wesentlichen parallel zu einer Längsachse des Energiespeicherelements (2400, 2412, 3412) ist.

4. Einrichtung (1000, 3002, 3006, 3007) nach Anspruch 1, wobei das Energiespeicherelement (2400, 3412) konfiguriert ist, um sich zwischen einer ersten Energiespeicherelementposition und einer zweiten Energiespeicherelementposition zu bewegen, wobei das Energiespeicherelement in seiner ersten Energiespeicherelementposition angeordnet ist, wenn das Energiespeicherelement in seiner ersten Konfiguration ist, wobei das Energiespeicherelement in seiner zweiten Energiespeicherelementposition angeordnet ist, wenn das Energiespeicherelement in seiner zweiten Konfiguration ist.

5. Einrichtung (1000, 3002, 3006, 3007) nach Anspruch 1, wobei das Energiespeicherelement (2400, 2412, 3412) ein Gasbehälter ist, der konfiguriert ist, um ein Gas zu enthalten, das einen ersten Druck, wenn der Gasbehälter in seiner ersten Konfiguration ist, und einen zweiten Druck aufweist, wenn der Gasbehälter in seiner zweiten Konfiguration ist, wobei der zweite Druck niedriger als der erste Druck ist, wobei die Einrichtung ferner Folgendes umfasst:
einen Punktierer, der konfiguriert ist, um einen Abschnitt des Gasbehälters zu durchdringen, wenn der Gasbehälter in seiner zweiten Konfiguration ist.

6. Einrichtung (1000, 3002, 3006, 3007) nach Anspruch 1, ferner Folgendes umfassend:
ein Vorspannelement (3560), das konfiguriert ist, um das Betätigungselement zu seiner zweiten Position hin vorzuspannen.

7. Einrichtung (1000, 3002, 3006, 3007) nach Anspruch 1, ferner Folgendes umfassend:
eine Feder (1600, 3560), die konfiguriert ist, um das Betätigungselement zu seiner zweiten Position hin vorzuspannen, wobei das Betätigungselement ein distales Ende (3544), das konfiguriert ist, um den distalen Endabschnitt des Gehäuses in Eingriff zu nehmen, und ein proximales Ende (3542) einschließt, das konfiguriert ist, um das Energiespeicherelement in Eingriff zu nehmen, wobei das Betätigungselement konfiguriert ist, um von seiner ersten Position in seine zweite Position bewegt zu werden, indem das distale Ende des Betätigungselements von dem distalen Endabschnitt des Gehäuses gelöst wird.

8. Einrichtung (1000, 3002, 3006, 3007) nach Anspruch 1, wobei:
sich die Nadel (3212, 6100) in einer ersten Richtung bewegt, wenn sie von der ersten Nadelposition in die zweite Nadelposition bewegt wird;
und sich das Betätigungselement in einer zweiten Richtung bewegt, wenn es von seiner ersten Position in seine zweite Position bewegt wird, wobei die zweite Richtung im Wesentlichen der ersten Richtung entgegengesetzt ist.

9. Einrichtung (1000, 3002, 3006, 3007) nach Anspruch 1, wobei das Betätigungselement ein erstes Betätigungselement ist, wobei die Einrichtung ferner Folgendes umfasst:
ein zweites Betätigungselement (3520, 3158), wobei das zweite Betätigungselement eine Öffnung (3532) definiert, innerhalb derer ein Abschnitt der Nadel (3212, 6100) angeordnet ist, wenn die Nadel in der zweiten Nadelposition ist.

10. Einrichtung (1000, 3002, 3006, 3007) nach Anspruch 1, ferner Folgendes umfassend:
eine Rückzugsfeder (3350), die konfiguriert ist, um die Nadel in die erste Nadelposition vorzuspannen.

11. Einrichtung (1000, 3002, 3006, 3007) nach Anspruch 1, ferner Folgendes umfassend:
eine Rückzugsfeder (3350), die konfiguriert ist, um die Nadel in die erste Nadelposition vorzuspannen, wobei eine Längsachse der Rückzugsfeder von einer Längsachse des Medikamentenbehälters versetzt ist.

12. Einrichtung (1000, 3002, 3006, 3007) nach Anspruch 1, wobei das Betätigungselement (3540) ein erstes Betätigungselement ist, wobei die Einrichtung ferner Folgendes umfasst:
ein zweites Betätigungselement (3520, 3158), das an den distalen Endabschnitt des Gehäuses (1100, 3110, 3116, 3182) gekoppelt ist, wobei das zweite Betätigungselement konfiguriert ist, um zu bewirken, dass sich das erste Betätigungselement von seiner ersten Position in seine zweite Position bewegt, wenn das zweite Betätigungselement beeinflusst wird.

13. Einrichtung (1000, 3002, 3006, 3007) nach Anspruch 1, wobei das Verriegelungselement eine Nadelhülle (3820, 3111, 3152, 3192) einschließt, die konfiguriert ist, um wenigstens um einen Abschnitt der Nadel angeordnet zu sein, wenn das Verriegelungselement an den distalen Endabschnitt des Gehäuses (1100, 3110, 3116, 3182) gekoppelt ist.

14. Einrichtung (1000, 3002, 3006, 3007) nach Anspruch 1, wobei:
das Betätigungselement (3540, 7540, 9540, 10540) einen Stab (3540) mit mehreren Vorsprüngen (3548) einschließt; und
der Vorsprung konfiguriert ist, um sich zwischen den mehreren Vorsprüngen des Stabes zu erstrecken, wobei der Vorsprung konfiguriert ist, um zu verhindern, dass die mehreren Vorsprünge nach innen zu einander hin bewegt werden, wenn das Verriegelungselement an den distalen Endabschnitt des Gehäuses (1100, 3110, 3116, 3182) gekoppelt ist.

15. Einrichtung (1000, 3002, 3006, 3007) nach Anspruch 1, wobei der Medikamentenbehälter (3262) Epinephrin einschließt.

## Revendications

1. Appareil (1000, 3002, 3006, 3007) comprenant :
un logement (1100, 3110, 3116, 3182) ayant une partie d'extrémité distale et une partie d'extrémité proximale, la partie d'extrémité distale comprenant une surface d'extrémité distale ;
un injecteur de médicament comprenant un récipient à médicament (3262) et une aiguille (3212, 6100), l'aiguille étant configurée pour se déplacer dans le logement entre une première position d'aiguille et une seconde position d'aiguille, dans la première position d'aiguille, l'aiguille est contenue dans le logement, dans la deuxième position d'aiguille, au moins une partie de l'aiguille s'étend depuis la partie d'extrémité distale du logement ;
un élément de stockage d'énergie (2400, 2412, 3412) ayant une première configuration et une seconde configuration, l'élément de stockage d'énergie étant configuré pour produire une force lors du passage de la première configuration à la seconde configuration pour déplacer l'aiguille entre la première position d'aiguille et la deuxième position de l'aiguille ; et
un élément d'actionnement (3540, 7540, 9540, 10540) configuré pour faire passer l'élément de stockage d'énergie de sa première configuration à sa seconde configuration lorsque l'élément d'actionnement est déplacé d'une première position à une seconde position ; et
un élément de verrouillage (3130, 3170) configuré pour être couplé de manière amovible à la partie d'extrémité distale du logement, une saillie (3128, 3178) de l'élément de verrouillage disposé à travers une ouverture définie par la surface d'extrémité distale du logement de sorte que la saillie engage l'élément d'actionnement pour empêcher le mouvement de l'élément d'actionnement de la première position à la seconde position lorsque l'élément de verrouillage est couplé à la partie d'extrémité distale du boîtier.

2. Appareil (1000, 3002, 3006, 3007) selon la revendication 1, dans lequel un axe longitudinal du récipient à médicament (3262) est décalé par rapport à un axe longitudinal de l'élément de stockage d'énergie (2400, 2412, 3412).

3. Appareil (1000, 3002, 3006, 3007) selon la revendication 1, dans lequel un axe longitudinal du récipient à médicament (3262) est sensiblement parallèle à un axe longitudinal de l'élément de stockage d'énergie (2400, 2412, 3412).

4. Appareil (1000, 3002, 3006, 3007) selon la revendication 1, dans lequel l'élément de stockage d'énergie (2400, 3412) est configuré pour se déplacer entre une première position d'élément de stockage d'énergie et une seconde position d'élément de stockage d'énergie, l'élément de stockage d'énergie étant disposé dans sa première position d'élément de stockage d'énergie lorsque l'élément de stockage d'énergie est dans sa première configuration, l'élément de stockage d'énergie étant disposé dans sa seconde position d'élément de stockage d'énergie lorsque l'élément de stockage d'énergie est dans sa seconde configuration.

5. Appareil (1000, 3002, 3006, 3007) selon la revendication 1, dans lequel l'élément de stockage d'énergie (2400, 2412, 3412) est un récipient à gaz configuré pour contenir un gaz ayant une première pression lorsque le récipient à gaz est dans sa première configuration et une seconde pression lorsque le réservoir à gaz est dans sa seconde configuration, la seconde pression étant inférieure à la première pression, l'appareil comprenant en outre :
un dispositif de perforation configuré pour pénétrer dans une partie du récipient à gaz lorsque le récipient à gaz est dans sa seconde configuration.

6. Appareil (1000, 3002, 3006, 3007) selon la revendication 1, comprenant en outre :
un élément de sollicitation (3560) configuré pour solliciter l'élément d'actionnement vers sa seconde position.

7. Appareil (1000, 3002, 3006, 3007) selon la revendication 1, comprenant en outre :
un ressort (1600, 3560) configuré pour solliciter l'élément d'actionnement vers sa seconde position, l'élément d'actionnement comprenant une extrémité distale (3544) configurée pour engager la partie d'extrémité distale du logement et une extrémité proximale (3542) configurée pour engager l'élément de stockage d'énergie, l'élément d'actionnement étant configuré pour être déplacé de sa première position à sa seconde position en désengageant l'extrémité distale de l'élément d'actionnement de la partie d'extrémité distale du logement.

8. Appareil (1000, 3002, 3006, 3007) selon la revendication 1, dans lequel :
l'aiguille (3212, 6100) se déplace dans une première direction lorsqu'elle est déplacée de la première position d'aiguille à la seconde position d'aiguille ;
et l'élément d'actionnement se déplace dans une seconde direction lorsqu'il est déplacé de sa première position à sa seconde position, la seconde direction étant sensiblement opposée à la première direction.

9. Appareil (1000, 3002, 3006, 3007) selon la revendication 1, dans lequel l'élément d'actionnement est un premier élément d'actionnement, l'appareil comprenant en outre :
un second élément d'actionnement (3520, 3158), le second élément d'actionnement définissant une ouverture (3532) à l'intérieur de laquelle une partie de l'aiguille (3212, 6100) est disposée lorsque l'aiguille est dans la seconde position de l'aiguille.

10. Appareil (1000, 3002, 3006, 3007) selon la revendication 1, comprenant en outre :
un ressort de rappel (3350) configuré pour solliciter l'aiguille dans la première position de l'aiguille.

11. Appareil (1000, 3002, 3006, 3007) selon la revendication 1, comprenant en outre :
un ressort de rappel (3350) configuré pour solliciter l'aiguille dans la première position d'aiguille, un axe longitudinal du ressort de rappel étant décalé par rapport à un axe longitudinal du récipient à médicament.

12. Appareil (1000, 3002, 3006, 3007) selon la revendication 1, dans lequel l'élément d'actionnement (3540) est un premier élément d'actionnement, l'appareil comprenant en outre :
un second élément d'actionnement (3520, 3158) couplé à la partie d'extrémité distale du logement (1100, 3110, 3116, 3182), le second élément d'actionnement étant configuré pour amener le premier élément d'actionnement à se déplacer de sa première position à sa seconde position lorsque le second élément d'actionnement est manipulé.

13. Appareil (1000, 3002, 3006, 3007) selon la revendication 1, dans lequel l'élément de verrouillage comprend une gaine d'aiguille (3820, 3111, 3152, 3192) configurée pour être disposée autour d'au moins une partie de l'aiguille lorsque l'élément de verrouillage est couplé à la partie d'extrémité distale du logement (1100, 3110, 3116, 3182).

14. Appareil (1000, 3002, 3006, 3007) selon la revendication 1, dans lequel :
l'élément d'actionnement (3540, 7540, 9540, 10540) comprend une tige (3540) ayant une pluralité de saillies (3548) ; et
la saillie est configurée pour s'étendre entre la pluralité de saillies de la tige, la saillie étant configurée pour empêcher la pluralité de saillies de se déplacer vers l'intérieur l'une vers l'autre lorsque l'élément de verrouillage est couplé à la partie d'extrémité distale du logement (1100, 3110, 3116, 3182).

15. Appareil (1000, 3002, 3006, 3007) selon la revendication 1, dans lequel le récipient à médicament (3262) comprend de l'épinéphrine.
